(19) Europäisches Patentamt
European Patent Office
Office européen des brevets

(11) EP 4 389 736 A1

# (12) EUROPEAN PATENT APPLICATION
published in accordance with Art. 153(4) EPC

(43) Date of publication:
**26.06.2024 Bulletin 2024/26**

(21) Application number: **22857522.1**

(22) Date of filing: **26.07.2022**

(51) International Patent Classification (IPC):
***C07D 213/75*** *(2006.01)* ***A61P 35/00*** *(2006.01)*
***A61K 31/506*** *(2006.01)*

(52) Cooperative Patent Classification (CPC):
**A61K 31/44; A61K 31/443; A61K 31/4439; A61K 31/4545; A61K 31/4985; A61K 31/505; A61K 31/506; A61K 31/5377; A61K 31/5383; A61K 31/55; A61P 9/00; A61P 31/12; A61P 35/00; A61P 35/02; C07D 213/75;** (Cont.)

(86) International application number:
**PCT/CN2022/107742**

(87) International publication number:
**WO 2023/020209 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.08.2021 CN 202110936628**

(71) Applicant: **Shanghai Institute of Materia Medica, Chinese Academy of Sciences**
**Pudong, Shanghai 201203 (CN)**

(72) Inventors:
- **HU, Youhong**
  **Shanghai 201203 (CN)**
- **CHEN, Yi**
  **Shanghai 201203 (CN)**
- **XIE, Zhicheng**
  **Shanghai 201203 (CN)**
- **DING, Jian**
  **Shanghai 201203 (CN)**
- **LI, Xin**
  **Shanghai 201203 (CN)**
- **FANG, Yanfen**
  **Shanghai 201203 (CN)**
- **SHEN, Qianqian**
  **Shanghai 201203 (CN)**

(74) Representative: **Grünecker Patent- und Rechtsanwälte**
**PartG mbB**
**Leopoldstraße 4**
**80802 München (DE)**

## (54) UREA COMPOUND CONTAINING 2-HETEROAROMATIC RING SUBSTITUTION, PREPARATION METHOD THEREFOR AND USE THEREOF

(57) The present invention relates to a urea compound containing 2-heteroaromatic ring substitution represented by formula (I), its enantiomers, diastereomers, racemates or mixtures thereof, or pharmaceutically acceptable salts thereof, solvate, metabolite or prodrug. The compound of formula (I) of the present invention has inhibitory activity against CDK9, and representative compounds have significant anti-tumor activity against CDK9 high-expressing tumor cells. Representative compounds have plasma stability and low clearance.

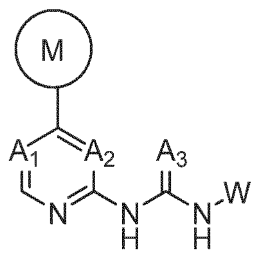

(I)

EP 4 389 736 A1

(52) Cooperative Patent Classification (CPC): (Cont.)
**C07D 213/85; C07D 239/42; C07D 401/04;**
**C07D 401/12; C07D 405/04; C07D 405/12;**
**C07D 487/04; C07D 487/10; C07D 498/04;**
Y02P 20/55

## Description

### Technical field

The present invention relates to the field of medicine, and in particular to urea compounds containing 2-heteroaromatic ring substitutions, the preparation method and use thereof. More specifically, the present invention relates to inhibitor compounds that can inhibit cyclin-dependent kinases (especially CDK9), preparation method for the compounds, and use of the compounds or compositions for the preventing and/or treating CDK9-related diseases.

### Background Art

Cyclin Dependent Kinase (CDK) is a type of serine/threonine protein kinase whose activity depends on the binding and activation of cyclin partners, and is a key regulator of cell cycle progression and gene transcription. So far, 21 subtypes of the CDK family have been discovered and identified, and these CDK protein kinase functional domains are highly evolutionary conserved. According to the differences in CDK functions, they can be divided into cell cycle regulatory CDKs (such as CDK1, 2, 4, 6) and transcriptional function CDKs (such as CDK7, 8, 9, 12, 13) (Lim S, Kaldis P. Development 2013, 140: 3079-3093). Cell cycle regulatory CDKs directly regulate cell cycle progression by phosphorylating cell cycle-related protein substrates. Transcriptional function CDKs regulate gene transcription processes by phosphorylating the RNA polymerase II complex. Clinical studies have shown that CDKs are frequently mutated, amplified, and overexpressed in different level in many malignant tumor cells (such as acute myeloid leukemia, skin cancer, melanoma, lung cancer, gastric cancer, breast cancer, pancreatic cancer, etc.), these mutations are closely related to the occurrence and development of tumors, as well as patient survival and drug resistance. In addition, a large number of basic studies have also found that high activation of CDKs protein kinases leads to abnormalities in cell cycle regulation and gene transcription, thereby driving tumor occurrence, and inhibiting CDKs can effectively inhibit tumor growth in vivo and in vitro (Chou J, et al., Cancer Discov. 2020, 10(3): 351-370.). In recent years, the studies have found that CDK4/5/6 has tumor immune regulation function, and the selective inhibition of CDK4/5/6 can enhance the effect of tumor immunotherapy. Therefore, kinase inhibitors targeting CDKs have become an important cancer treatment strategy. In particular, the three commercially available CDK4/6 inhibitors (Palbociclib, Ribociclib, Abemaciclib) have fully proved the scientific rationality and commercial viability of developing medicines targeting CDKs protein kinases. Although many different types of CDKs inhibitors have been subjected to extensive preclinical and clinical studies, most CDKs inhibitors have problems such as poor subtype selectivity, high toxic and side effects, and narrow therapeutic windows. So far, only CDK4/6 selective inhibitors (Palbociclib, Ribociclib, Abemaciclib) have been approved for marketing, and they have only been successfully used in the clinical treatment of estrogen receptor-negative, HER2-negative advanced or recurrent breast cancer. Therefore, it is of great significance to develop new, low-toxic, and highly efficient CDK subtype-selective kinase inhibitors. More and more studies have shown that the anti-tumor benefits of non-selective CDKs inhibitors are produced by inhibiting the CDK9-mediated transcriptional regulatory pathway, so research on selective inhibitors of CDK9 has attracted people’s attention (Chen R, et al., Blood, 2009, 113: 4637-4645.; MacCallum DE, et al., Cancer Res., 2005, 65: 5399-5407.; Gregory GP, et al., Leukemia, 2015, 29: 1437- 1441.; Krystof V, et al., Cell Cycle, 2016, 15: 519-527.).

CDK9 is different from cycle-regulating CDKs in that it only plays a role in the transcription elongation phase and does not participate in the regulation of the cell cycle. CDK9 forms a heterodimeric complex with cell cycle proteins (T and K) as a positive transcription elongation factor b (P-TEFb), which promote transcription elongation by phosphorylating the Ser-2 of carbon-terminal domain of RNA polymerase II. Abnormal activation of CDK9 kinase activity in the P-TEFb complex is associated with the pathological processes of a variety of human diseases, such as hyperproliferative diseases (such as cancer), virus-induced infectious diseases, and cardiovascular diseases (Krystof V, et al., Cell Cycle, 2016, 15: 519-527.; Shudong Wang and Peter M. Fischer, Trends Pharmacol. Sci., 2008, 29: 302-313.). Studies have shown that abnormal activation of CDK9 signaling pathway is present in a variety of human hematomas (such as multiple myeloma, acute myeloid leukemia, lymphoma, chronic lymphocytic leukemia, etc.) and solid tumors (such as lung cancer, liver cancer, breast cancer, prostate cancer, etc.), which affects the expression of anti-apoptotic proteins and promotes occurrence of tumor cell proliferation. Selective inhibition of CDK9 kinase can inhibit the transcriptional activity of RNA polymerase II, downregulates the RNA transcription of a variety of short-term anti-apoptotic proteins related to tumor survival, such as c-Myc, NF-xB response gene transcripts, mitosis Kinase, and affects the expression of anti-apoptotic Bcl-2 family proteins, especially the decrease in the expression of Mcl-1 and XIAP, thereby induces apoptosis in tumor cells to exert anti-tumor effects (Shudong Wang and Peter M. Fischer, Trends Pharmacol. Sci., 2008, 29: 302-313.). In conclusion, multiple evidences suggest that selective inhibition of CDK9 is an important strategy for anti-tumor treatment, and the tumor includes, but not limited to hematological malignancies (such as acute myeloid leukemia, multiple myeloma, chronic lymphocytic leukemia, diffuse large B-cell lymphoma, Burkitt’s lymphoma, follicular lymphoma) and solid tumors (such as prostate cancer, breast cancer, lung cancer, neuroblastoma, and colon cancer). In addition, CDK9 inhibitors

may also have therapeutic effects in other diseases, including cardiac disease, viral diseases, inflammation and pain.

So far, no highly selective CDK9 inhibitor has been approved for marketing in the world. Currently, only CDK9 selective inhibitors AZD4573, BAY1251152, BAY1143572, KB0742 and GFH009 have entered clinical research. Although these compounds have demonstrated good CDK9 kinase selectivity and anti-tumor effects in vitro and in vivo, the urgent clinical need for highly efficient CDK9 inhibitors with low toxicity has not yet been met. At the same time, in view of the shortcomings of CDK9 selective inhibitors currently under development, the kinase selectivity, activity and druggability still need to be further improved. The compounds disclosed in this patent have high CDK9 activity and selectivity, and have broad application prospects in preventing and/or treating CDK9-mediated diseases.

## Summary of the Invention

The oject of the present invention is to provide a new small molecule compound with good specificity and high activity, which can be used as a cyclin-dependent kinase 9 (CDK9) inhibitor to prevent and/or treat diseases mediated by CDK9 kinase, especially in medicines for hyperproliferative diseases and/or virus-induced infectious diseases and/or cardiovascular diseases.

The invention relates to a new type of urea compound containing 2-heteroaromatic ring substitutions, which can effectively inhibit the in vitro growth of CDK9-positive lymphoma cell WSU-DLCL2 and a variety of different tumor cells, and its $IC_{50}$ value can reach low nanomolar or even picomolar concentrations. Specifically, the present invention provides urea compounds containing 2-heteroaromatic ring substitution of formula (I), their enantiomers, diastereomers, racemates or mixtures thereof, or pharmaceutically acceptable salts thereof, solvate, metabolite or prodrug:

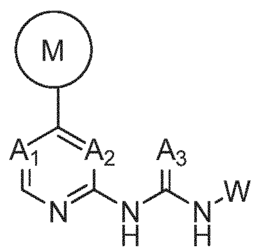

(I)

in which

- $A_1$ and $A_2$ are each independently selected from Nor C-$R_1$;
- $A_3$ is selected from O or S;
- M is selected from unsubstituted or substituted 5-8 membered aryl, unsubstituted or substituted 5-8 membered heteroaryl, unsubstituted or substituted 5-10 membered heterocyclyl; the substitution means that each of the above groups is independently substituted by 1-5 $R_2$ groups; or two $R_2$ and the two atoms connected to each of them form a 5-7 membered cycloalkyl, heterocyclyl or spirocyclyl, and further substituted by 0-5 R' group; wherein one or more $CH_2$ on the above-mentioned ring are optionally replaced by a corresponding number of C(O) groups, and one or more S atom or N atom on the ring are optionally oxidized to form S-oxide or N-oxide;
- W is selected from unsubstituted or substituted C1-C6 alkyl, unsubstituted or substituted 3-7 membered cycloalkyl, unsubstituted or substituted 3-7 membered heterocycloalkyl, unsubstituted or substituted 5-10 membered heterocyclyl, unsubstituted or substituted -($C_{1-6}$ alkyl)-(3-7 membered cycloalkyl), unsubstituted or substituted -($C_{1-6}$ alkyl)-(3-7 membered heterocycloalkyl), unsubstituted or substituted 4-8 membered cycloalkenyl, unsubstituted or substituted 4-8 membered heterocycloalkenyl, unsubstituted or substituted -($C_{1-6}$ alkyl)-(4-8 membered cycloalkenyl), unsubstituted or substituted -($C_{1-6}$ alkyl)-(4-8 membered heterocycloalkenyl), unsubstituted or substituted 5-8 membered aryl, unsubstituted or substituted 5-8 heteroaryl, unsubstituted or substituted -($C_{1-6}$ alkyl)-(5-8 membered heteroaryl), unsubstituted or substituted -(5-8 membered heteroaryl)-($C_{1-6}$ alkyl), unsubstituted or substituted -(5-8 membered heteroaryl)-(3-7 membered cycloalkyl), unsubstituted or substituted -(5-8 membered heteroaryl)-(3-7 membered heterocycloalkyl), unsubstituted or substituted -($C_{1-6}$ alkyl)-(5-10 membered heterocyclyl); the substitution means each independently substituted by 1-5 $R_4$ groups; or two $R_4$ and the two atoms connected to each of them form a cycloalkyl or heterocyclyl and are further substituted by 0-5 R' group; wherein one or more $CH_2$ on the above-mentioned ring are optionally replaced by a corresponding number of C(O) groups, and one or more S atom or N atom on the ring are optionally oxidized to form S-oxide or N-oxide;
- $R_1$ is selected from hydrogen, halogen, cyano or $C_{1-3}$ alkyl, $C_{3-6}$ cycloalkyl;
- $R_2$ at each occurrence is independently selected from halogen, hydroxyl, amino, cyano, $C_{1-4}$ alkyl, $C_{1-4}$ heteroalkyl,

$C_{2-6}$ alkenyl, $C_{2-6}$ alkynyl, $C_{3-6}$ cycloalkyl, 3-6 membered heterocycloalkyl, 4-8 membered cycloalkenyl, 4-8 membered heterocycloalkenyl, -O($C_{1-6}$ alkyl), -O($C_{3-6}$ cycloalkyl), -NH($C_{1-6}$ alkyl), -NH($C_{3-6}$ cycloalkyl), -C(O)($C_{1-4}$ alkyl), -C(O)($C_{3-6}$ cycloalkyl); wherein the above substituent is further substituted by 0-5 substituents selected from the following: halogen, hydroxyl, cyano group, 5-7 membered aryl, 5-7 membered heteroaryl, $C_{3-6}$ cycloalkyl, 3-6 membered heterocycloalkyl, 3-6 membered heterocycloalkyl, -$C_{1-4}$ alkyl-$R_3$, -$OR_3$, -$NHR_3$, -$N(R_3)_2$, -$NHC_{1-4}$ alkyl-$R_3$, -$OC_{1-4}$ alkyl-$R_3$, -$CONHR_3$, -$CON(R_3)_2$, -$SR_3$, -$SOR_3$, -$SO_2R_3$;

$R_3$ is selected from 5-7 membered aryl, 5-7 membered heteroaryl, $C_{3-6}$ cycloalkyl, 3-6 membered heterocycloalkyl, 3-6 membered heterocycloalkyl; the substituent is further substituted by 0-5 groups selected from the following: halogen, hydroxyl, $C_{1-4}$ alkyl, -O($C_{1-4}$ alkyl), amino, -NH($C_{1-4}$ alkyl), -C(O)($C_{1-4}$ alkyl), -NHC(O)($C_{1-4}$ alkyl);

$R_4$ at each occurrence is independently selected from halogen, carbonyl, cyano, nitro, $C_{1-6}$ alkyl, $C_{1-6}$ heteroalkyl, $C_{2-6}$ alkenyl, $C_{2-6}$ alkynyl, $C_{3-6}$ cycloalkyl, 3-6 membered heterocycloalkyl, 4-8 membered cycloalkenyl, 4-8 membered heterocycloalkenyl, 5-7 membered aryl, 5-7 membered heteroaryl, ($C_{1-6}$ alkyl)-O-($C_{1-6}$ alkyl), $OR_5$, $SR_5$, $S(O)R_5$, $SO_2R_5$, $C(O)R_5$, $C(O)N(R_5)_2$, $C(O)NR_5OR_5$, $C(O)NR_5SO_2R_5$, $CO(O)R_5$, $N(R_5)_2$, $NR_5C(O)R_5$, $NR_5SO_2R_5$, $NR_5SO_2NR_5C(O)OR_5$, $NR_5C(O)N(R_5)_2$, $OC(O)R_5$, $OC(O)OR_5$, $OC(O)N(R_5)_2$, $OC(O)NR_5SO_2R_5$, $SO_2N(R_5)_2$, $SO_2NR_5C(O)R_5$, $NR_5S(O)_2R_5$, $NR_5C(O)OR_5$, $SO_2NR_5C(O)OR_5$, $OSO_2N(R_5)_2$; wherein any of the alkyl, cycloalkyl, heterocycloalkyl, alkenyl, alkynyl, cycloalkenyl, heterocycloalkenyl, aryl and heteroaryl are further substituted by 0-5 $R_5$ groups;

$R_5$ at each occurrence is independently selected from hydrogen, halogen, hydroxyl, amino, carboxyl, aldehyde group, carbonyl, cyano, nitro, $C_{1-6}$ alkyl, $C_{2-6}$ alkenyl, $C_{2-6}$ alkynyl, $C_{3-6}$ cycloalkyl, 3-6 membered heterocycloalkyl, -O($C_{1-6}$ alkyl), ($C_{1-6}$ alkyl)-O-($C_{1-6}$ alkyl); the above alkyl, alkenyl, alkynyl, cycloalkyl and heterocycloalkyl are substituted by 0-5 R' groups; or two $R_5$ groups together with the two atoms to which they are connected form 3-6 membered cycloalkyl or heterocycloalkyl and substituted by 0-5 R' groups;

R' at each occurrence is independently selected from H, halogen, cyano, hydroxyl, -O-($C_{1-6}$ alkyl), -C(O)R", -C(O)OR", $C_{1-6}$ alkyl, $C_{3-6}$ cycloalkyl, $C_{1-6}$ haloalkyl, $C_{1-6}$ heteroalkyl, hydroxy $C_{1-6}$ alkyl, -N(R")(R"), NHC(O)-($C_{1-3}$ alkyl), unsubstituted or substituted cycloalkyl; or two R' together with the atoms to which they are connected form a 3-6 membered cycloalkyl or heterocycloalkyl; wherein the alkyl, cycloalkyl and heterocycloalkyl are substituted by 0-5 R" groups;

R" at each occurrence is independently selected from hydrogen, hydroxyl, and $C_{1-6}$ alkyl;

In a preferred embodiment, $A_3$ is O; $A_1$ and $A_2$ are each independently selected from C-$R_1$; that is, a compound represented by formula (II);

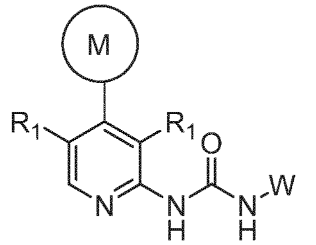

(II)

in which:

M is preferably selected from unsubstituted or substituted phenyl, unsubstituted or substituted pyridyl, unsubstituted or substituted pyrimidinyl, unsubstituted or substituted pyrazolyl, unsubstituted or substituted imidazolyl, unsubstituted or substituted thiazole group; the substitution means that the above groups are each independently substituted by 1-5 $R_2$ groups; or two $R_2$ and the two atoms connected to each of them form a 5-7 membered cycloalkyl or heterocyclyl and further substituted by 0-5 R' groups; wherein one or more $CH_2$ on the above-mentioned ring are optionally replaced by a corresponding number of C(O) groups, and one or more S atom or N atom on the ring are optionally oxidized to form S-oxide or N-oxide;

More preferably , M is selected from the group consisting of :

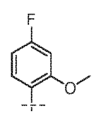, 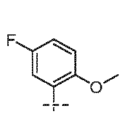,

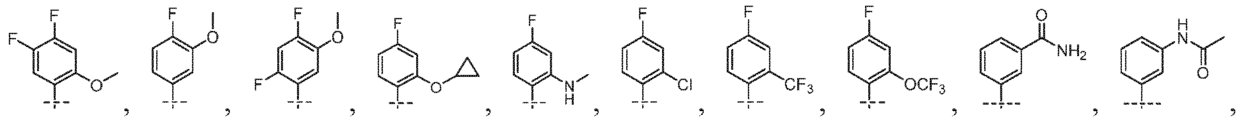

, , , , , , , , , ,

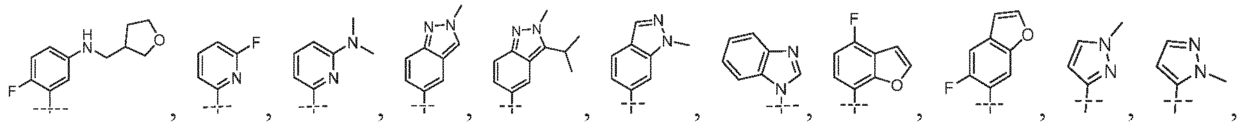

, , , , , , , , , , ,

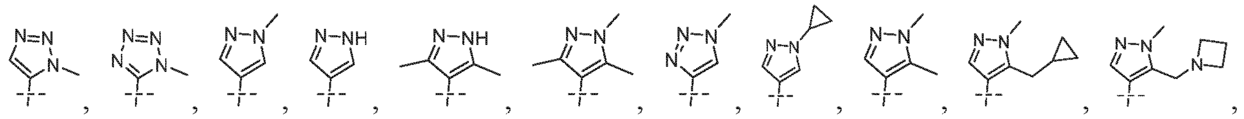

, , , , , , , , , , ,

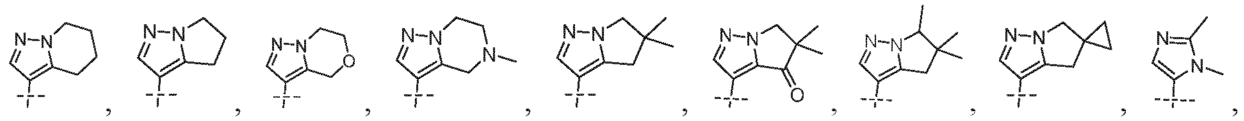

, , , , , , , , ,

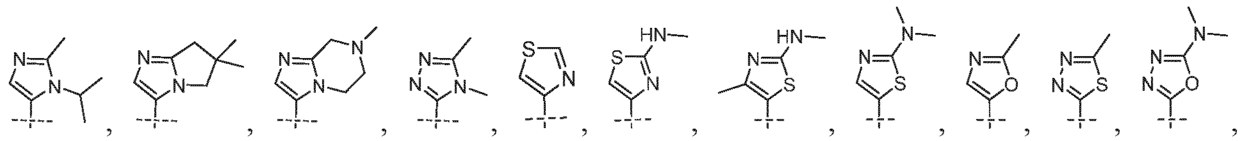

, , , , , , , , , , , ,

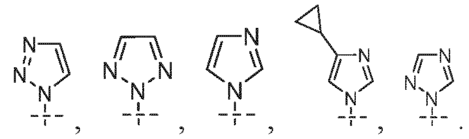

, , , , .

W is selected from unsubstituted or substituted C1-C6 alkyl, unsubstituted or substituted 3-7 membered cycloalkyl, unsubstituted or substituted 3-7 membered heterocycloalkyl, unsubstituted or substituted 5-10 membered heterocyclyl, unsubstituted or substituted -($C_{1-6}$ alkyl)-(3-7 membered cycloalkyl), unsubstituted or substituted -($C_{1-6}$ alkyl)-(3-7 membered heterocycloalkyl), unsubstituted or substituted 4-8 membered cycloalkenyl, unsubstituted or substituted 4-8 membered heterocycloalkenyl, unsubstituted or substituted -($C_{1-6}$ alkyl)-(4-8 membered cycloalkenyl), unsubstituted or substituted -($C_{1-6}$ alkyl)-(4-8 membered heterocycloalkenyl), unsubstituted or substituted 5-8 membered aryl, unsubstituted or substituted 5-8 heteroaryl, unsubstituted or substituted -($C_{1-6}$ alkyl)-(5-8 membered heteroaryl), unsubstituted or substituted -(5-8 membered heteroaryl)-($C_{1-6}$ alkyl), unsubstituted or substituted -(5-8 membered heteroaryl)-(3-7 membered cycloalkyl), unsubstituted or substituted -(5-8 membered heteroaryl)-(3-7 membered heterocycloalkyl), unsubstituted or substituted -($C_{1-6}$ alkyl)-(5-10 membered heterocyclyl); the substitution is each independently substituted by 1-5 $R_4$ groups; or two $R_4$ and the two atoms connected to each of them form a cycloalkyl or heterocyclyl and are further substituted by 0-5 R' group; wherein one or more $CH_2$ on the above-mentioned ring are optionally replaced by a corresponding number of C(O) groups, and one or more S atom or N atom on the ring are optionally oxidized to form S-oxide or N-oxide;

$R_1$ is selected from hydrogen, halogen, cyano or $C_{1-3}$ alkyl, $C_{3-6}$ cycloalkyl; preferably hydrogen or halogen.

$R_2$ at each occurrence is independently selected from halogen, hydroxyl, amino, cyano, $C_{1-4}$ alkyl, $C_{1-4}$ heteroalkyl, $C_{2-6}$ alkenyl, $C_{2-6}$ alkynyl, $C_{3-6}$ cycloalkyl, 3-6 membered heterocycloalkyl, 4-8 membered cycloalkenyl, 4-8 membered heterocycloalkenyl, -O($C_{1-6}$ alkyl), -O($C_{3-6}$ cycloalkyl), -NH($C_{1-6}$ alkyl), -NH($C_{3-6}$ cycloalkyl), -C(O)($C_{1-4}$ alkyl), -C(O)($C_{3-6}$ cycloalkyl); wherein the above substituent is further substituted by 0-5 substituents selected from the following: halogen, hydroxyl, cyano group, 5-7 membered aryl, 5-7 membered heteroaryl, $C_{3-6}$ cycloalkyl, 3-6 membered heterocycloalkyl, 3-6 membered heterocycloalkyl, -$C_{1-4}$ alkyl-$R_3$, -$OR_3$, -$NHR_3$, -$N(R_3)_2$, -$NHC_{1-4}$ alkyl-$R_3$, -$OC_{1-4}$ alkyl-$R_3$, -$CONHR_3$, -$CON(R_3)_2$, -$SR_3$, -$SOR_3$, -$SO_2R_3$;

$R_3$ is selected from 5-7 membered aryl, 5-7 membered heteroaryl, $C_{3-6}$ cycloalkyl, 3-6 membered heterocycloalkyl, 3-6 membered heterocycloalkyl; the substituent is further substituted by 0-5 groups selected from the following: halogen, hydroxyl, $C_{1-4}$ alkyl, -O($C_{1-4}$ alkyl), amino, -NH($C_{1-4}$ alkyl), -C(O)($C_{1-4}$ alkyl), -NHC(O)($C_{1-4}$ alkyl);

$R_4$ at each occurrence is independently selected from halogen, carbonyl, cyano, nitro, $C_{1-6}$ alkyl, $C_{1-6}$ heteroalkyl,

$C_{2-6}$ alkenyl, $C_{2-6}$ alkynyl, $C_{3-6}$ cycloalkyl, 3-6 membered heterocycloalkyl, 4-8 membered cycloalkenyl, 4-8 membered heterocycloalkenyl, 5-7 membered aryl, 5-7 membered heteroaryl, ($C_{1-6}$ alkyl)-O-($C_{1-6}$ alkyl), $OR_5$, $SR_5$, $S(O)R_5$, $SO_2R_5$, $C(O)R_5$, $C(O)N(R_5)_2$, $C(O)NR_5OR_5$, $C(O)NR_5SO_2R_5$, $CO(O)R_5$, $N(R_5)_2$, $NR_5C(O)R_5$, $NR_5SO_2R_5$, $NR_5SO_2NR_5C(O)OR_5$, $NR_5C(O)N(R_5)_2$, $OC(O)R_5$, $OC(O)OR_5$, $OC(O)N(R_5)_2$, $OC(O)NR_5SO_2R_5$, $SO_2N(R_5)_2$, $SO_2NR_5C(O)R_5$, $NR_5S(O)_2R_5$, $NR_5C(O)OR_5$, $SO_2NR_5C(O)OR_5$, $OSO_2N(R_5)_2$; wherein any of the alkyl, cycloalkyl, heterocycloalkyl, alkenyl, alkynyl, cycloalkenyl, heterocycloalkenyl, aryl and heteroaryl are further substituted by 0-5 $R_5$ groups;

$R_5$ at each occurrence is independently selected from hydrogen, halogen, hydroxyl, amino, carboxyl, aldehyde group, carbonyl, cyano, nitro, $C_{1-6}$ alkyl, $C_{2-6}$ alkenyl, $C_{2-6}$ alkynyl, $C_{3-6}$ cycloalkyl, 3-6 membered heterocycloalkyl, -O($C_{1-6}$ alkyl), ($C_{1-6}$ alkyl)-O-($C_{1-6}$ alkyl); the above alkyl, alkenyl, alkynyl, cycloalkyl and heterocycloalkyl are substituted by 0-5 R' groups; or two $R_5$ groups together with the two atoms to which they are connected form 3-6 membered cycloalkyl or heterocycloalkyl and substituted by 0-5 R' groups;

R' at each occurrence is independently selected from H, halogen, cyano, hydroxyl, -O-($C_{1-6}$ alkyl), -C(O)R", -C(O)OR", $C_{1-6}$ alkyl, $C_{3-6}$ cycloalkyl, $C_{1-6}$ haloalkyl, $C_{1-6}$ heteroalkyl, hydroxy $C_{1-6}$ alkyl, -N(R")(R"), NHC(O)-($C_{1-3}$ alkyl), unsubstituted or substituted cycloalkyl; or two R' together with the atoms to which they are connected form a 3-6 membered cycloalkyl or heterocycloalkyl; wherein the alkyl, cycloalkyl and heterocycloalkyl are substituted by 0-5 R" groups;

R" at each occurrence is independently selected from hydrogen, hydroxyl, and $C_{1-6}$ alkyl;

In the present invention,

The "alkyl group" refers to an aliphatic hydrocarbon group, which can be a branched or linear alkyl group. Depending on the structure, an alkyl group can be a monovalent group or a divalent group (i.e., an alkylene group), for example, in "hydroxy $C_{1-6}$ alkyl", the $C_{1-6}$ alkyl is actually a divalent group (alkylene). In the present invention, the alkyl group is preferably a "lower alkyl group" having 1 to 6 carbon atoms, even more preferably a "lower alkyl group" having 1 to 3 carbon atoms. Typical alkyl groups include, but are not limited to, methyl, ethyl, propyl, isopropyl, butyl, isobutyl, tert-butyl, pentyl, hexyl, etc.

The "halogen" is F, Cl, Br, or I;

The "halogenated alkyl group" means that at least one hydrogen atom in the alkyl group is replaced by a halogen atom. In certain embodiments, if two or more hydrogen atoms are replaced by halogen atoms, the halogen atoms are the same as or different from each other;

The "heteroalkyl group" means that at least one skeleton C atom in the alkyl is replaced by a heteroatom (N, O, S). In certain embodiments, if two or more C atoms are replaced by heteroatoms, the heteroatoms are the same as or different from each other;

The "cycloalkyl group" is a saturated or unsaturated 3-10 membered monocyclic or polycyclic aliphatic ring, and it can be a monovalent group or a bivalent group (i.e., cycloalkylene);

The "heterocycloalkyl" is a saturated or unsaturated 3-10 membered monocyclic or polycyclic aliphatic heterocyclic ring containing one or more heteroatoms selected from N, O, and S, and can be a monovalent group or bivalent group (i.e. heterocycloalkylene);

The "aryl group" means that each atom constituting the ring in the aryl ring is a carbon atom, including a single ring or a condensed polycyclic ring, and it can be a monovalent group or a bivalent group (i.e., arylene). In the present invention, the aryl ring preferably has 5 to 10 carbon atoms, and more preferably an aryl group has 5 to 7 carbon atoms.

The "aralkyl" refers to one or more hydrogen atoms in the alkyl group being replaced by an aryl group. For example, benzyl, phenethyl.

The "heteroaryl" is an aromatic group containing one or more heteroatoms selected from N, O, and S on the ring. Depending on the structure, a heteroaryl can be a monovalent group or a bivalent group (i.e., a heteroarylene). Examples of heteroaryl groups include, but are not limited to, pyridyl, pyrimidinyl, imidazolyl, pyrazolyl, pyrazinyl, triazolyl, tetrazolyl, thienyl, thiazolyl, furyl, oxazolyl, isoxazolyl, pyrrolyl, quinolyl, isoquinolyl, indolyl, benzimidazolyl, benzofuranyl, indazolyl, indolazinyl, phthalazinyl, pyridazinyl, isoindolyl, pteridyl, purinyl, oxadiazolyl, thiadiazolyl, furazyl, benzofurazyl, benzothienyl, benzothiazolyl, benzoxazolyl, quinazolinyl, etc.

The "heterocyclyl" is a monocyclic or polycyclic ring, and at least one is a saturated or partially saturated (non-aromatic) ring with one or more heteroatoms. Representative heterocyclyl includes the following three situations: 1) each ring is a non-aromatic ring, and at least one ring has heteroatoms; 2) at least one ring is a non-aromatic ring containing heteroatoms, and at least one other ring is an aromatic carbocyclic ring; 3) at least one ring is a non-aromatic ring containing heteroatoms, and at least one other ring is an heteroaromatic ring.

Preferably, the above compound of formula (I) or its deuterated compound or its pharmaceutically acceptable salt or prodrug is selected from the compounds of the following formula:

| No. | Structure | No. | Structure |
|---|---|---|---|
| 1 | 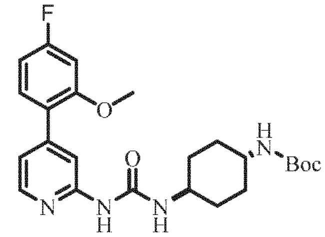 | 2 | 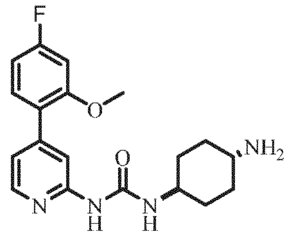 |
| 3 | 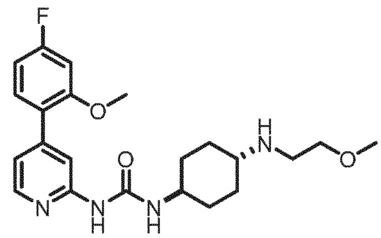 | 4 | 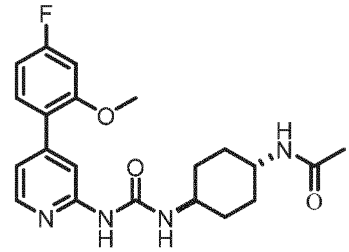 |
| 5 | 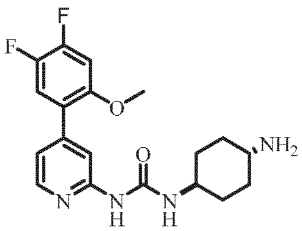 | 6 | 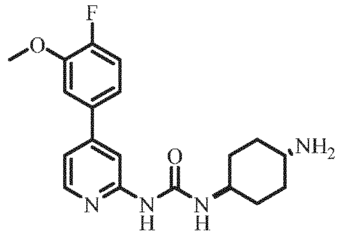 |
| 7 | 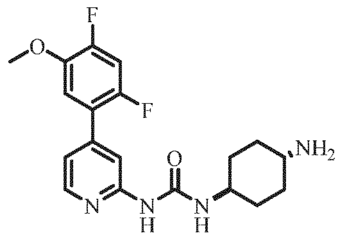 | 8 | 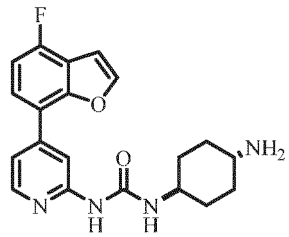 |
| 9 | 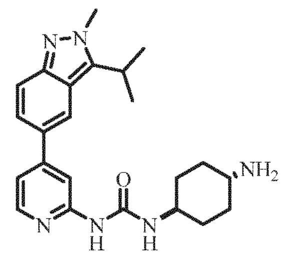 | 10 | 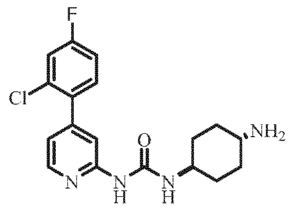 |
| 11 | 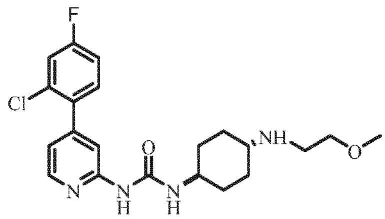 | 12 | 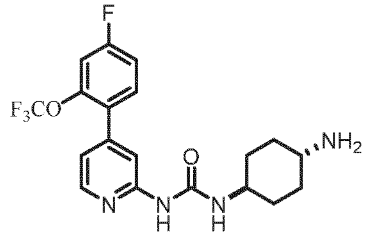 |
| 13 | 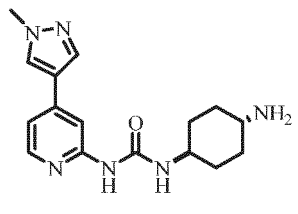 | 14 | 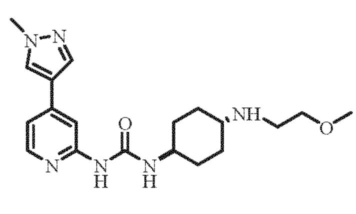 |

(continued)

| No. | Structure | No. | Structure |
|---|---|---|---|
| 15 | 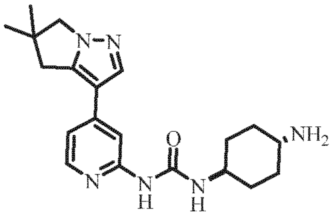 | 16 | 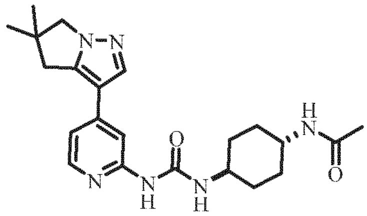 |
| 17 | 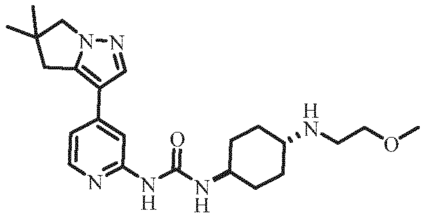 | 18 | 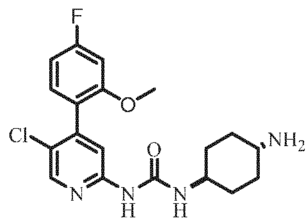 |
| 19 | 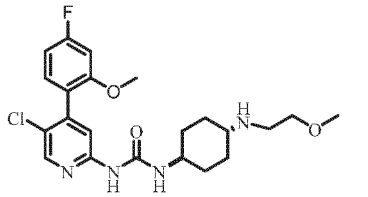 | 20 | 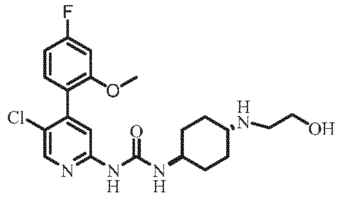 |
| 21 | 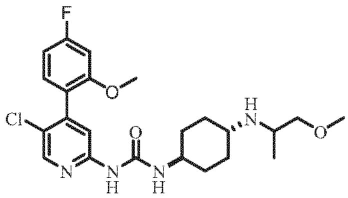 | 22 | 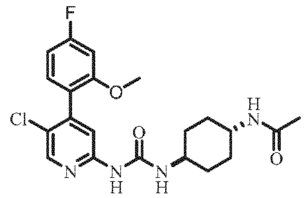 |
| 23 | 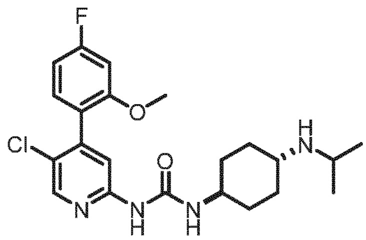 | 24 | 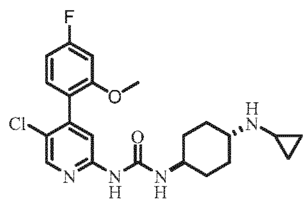 |
| 25 | 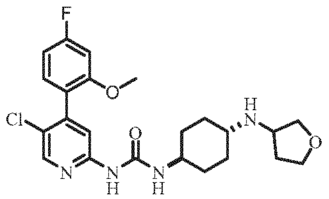 | 26 | 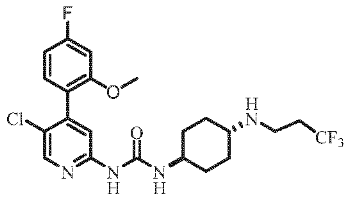 |
| 27 | 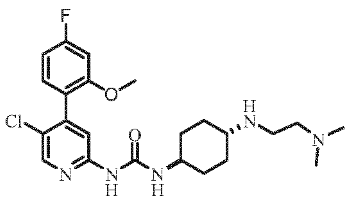 | 28 | 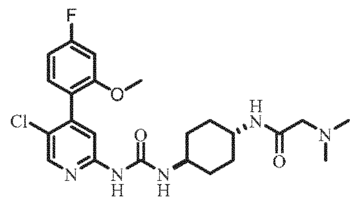 |

(continued)

| No. | Structure | No. | Structure |
|---|---|---|---|
| 29 | 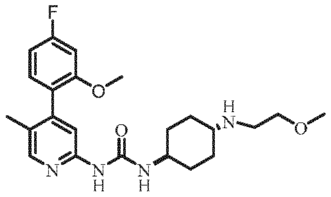 | 30 | 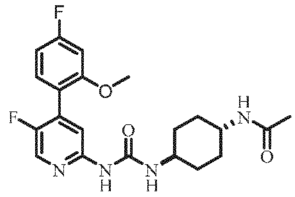 |
| 31 | 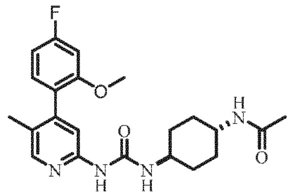 | 32 | 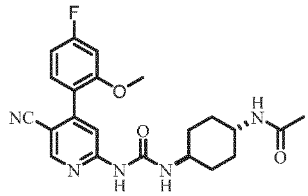 |
| 33 | 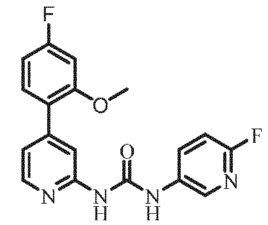 | 34 | 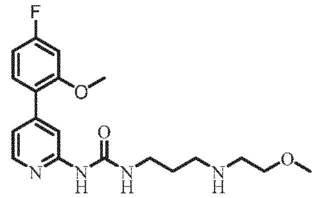 |
| 35 | 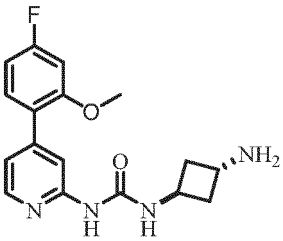 | 36 | 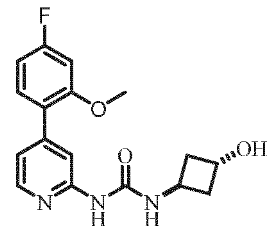 |
| 37 | 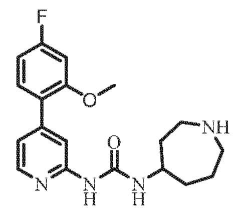 | 38 | 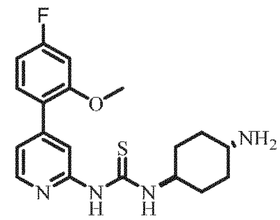 |
| 39 | 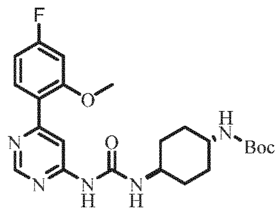 | 40 | 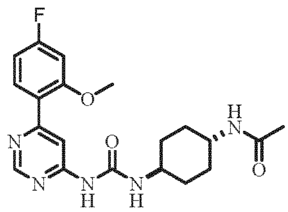 |
| 41 | 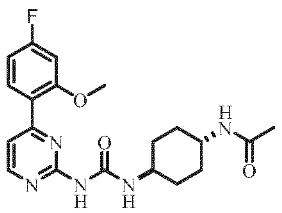 | 42 | 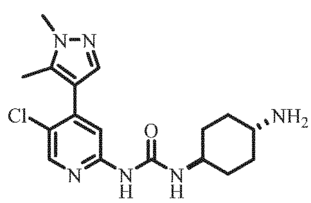 |

(continued)

| No. | Structure | No. | Structure |
|---|---|---|---|
| 43 | 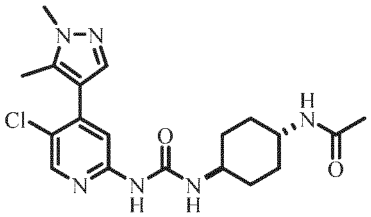 | 44 | 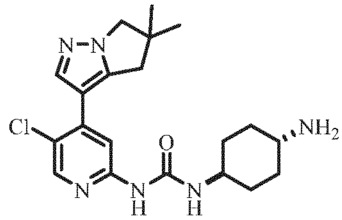 |
| 45 | 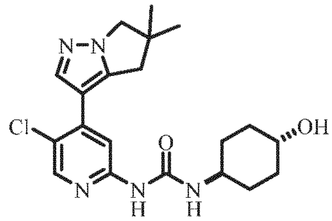 | 46 | 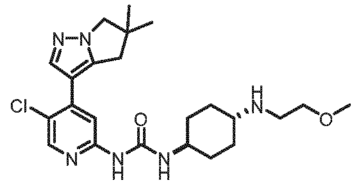 |
| 47 | 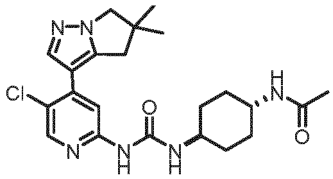 | 48 | 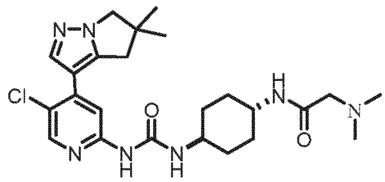 |
| 49 | 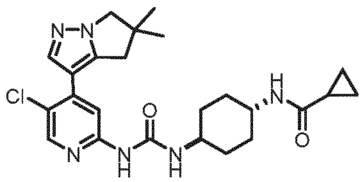 | 50 | 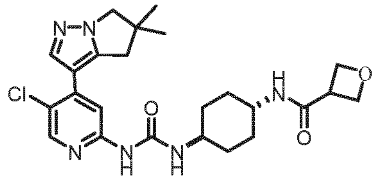 |
| 51 | 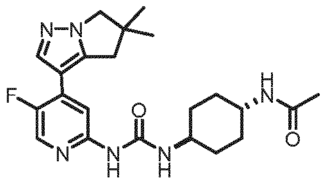 | 52 | 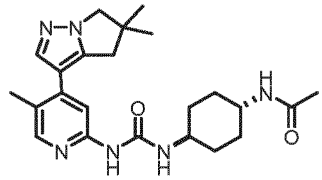 |
| 53 | 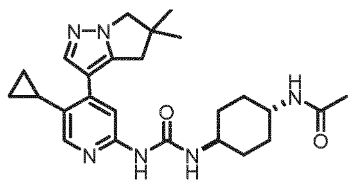 | 54 | 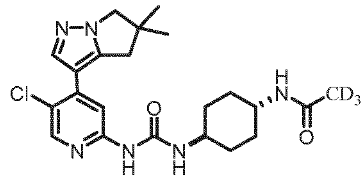 |
| 55 | 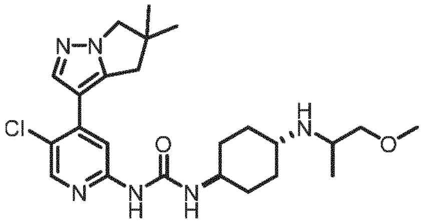 | 56 | 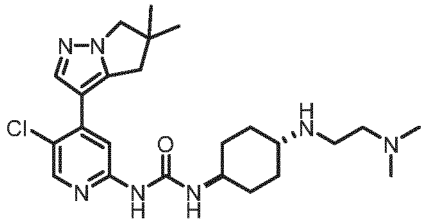 |

(continued)

| No. | Structure | No. | Structure |
|---|---|---|---|
| 57 | 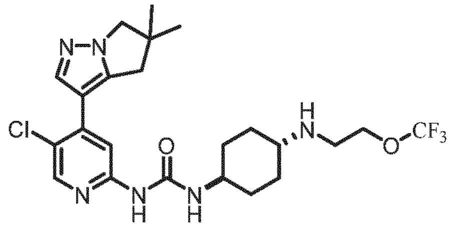 | 58 | 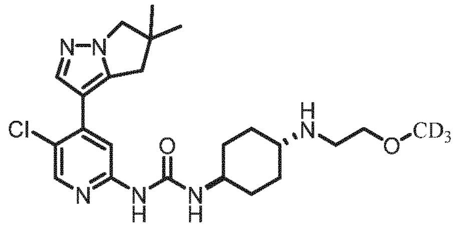 |
| 59 | 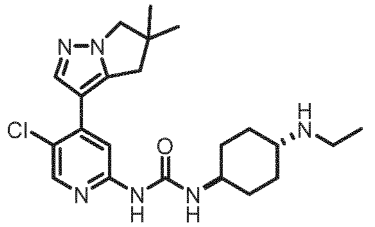 | 60 | 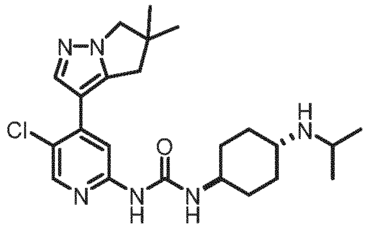 |
| 61 | 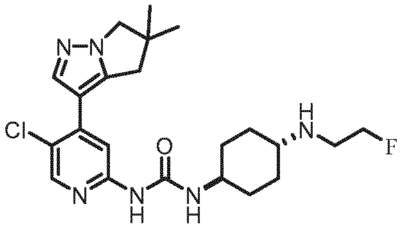 | 62 | 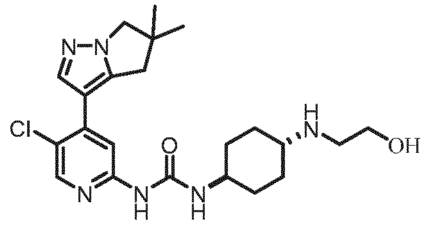 |
| 63 | 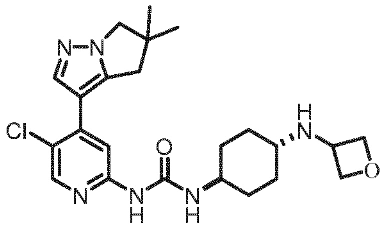 | 64 | 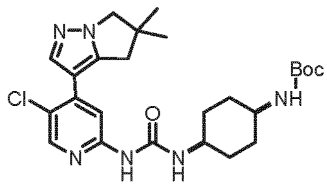 |
| 65 | 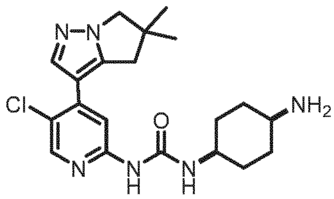 | 66 | 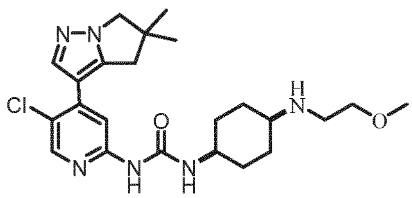 |
| 67 | 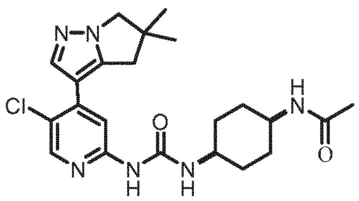 | 68 | 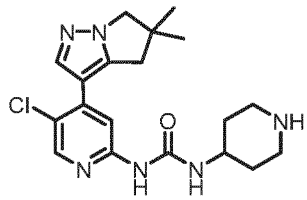 |
| 69 | 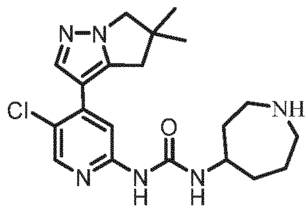 | 70 | 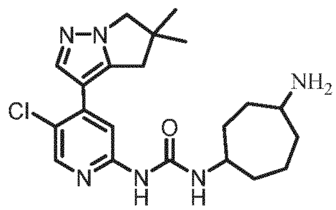 |

(continued)

| No. | Structure | No. | Structure |
|---|---|---|---|
| 71 | 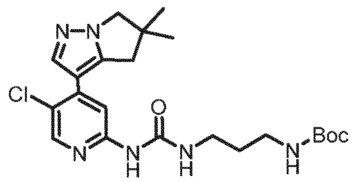 | 72 | 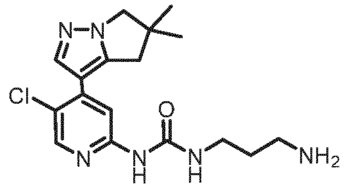 |
| 73 | 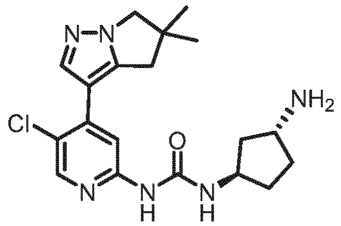 | 74 | 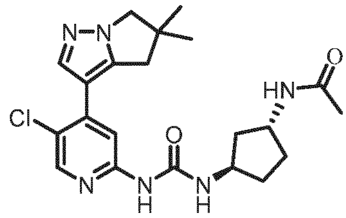 |
| 75 | 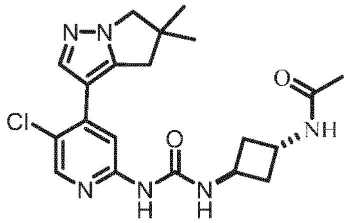 | 76 | 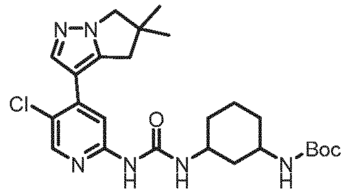 |
| 77 | 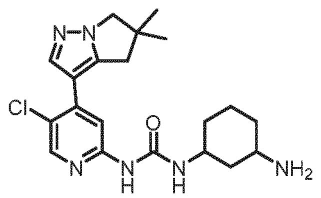 | 78 | 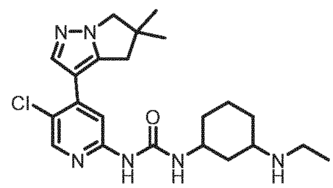 |
| 79 | 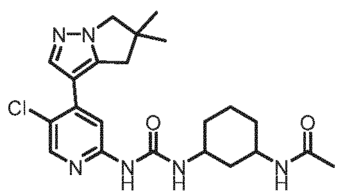 | 80 | 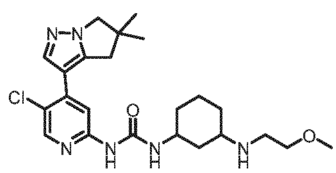 |
| 81 | 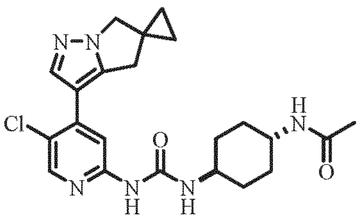 | 82 | 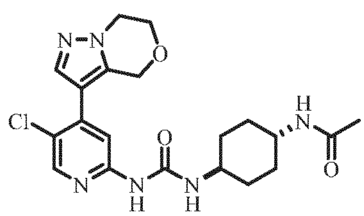 |
| 83 | 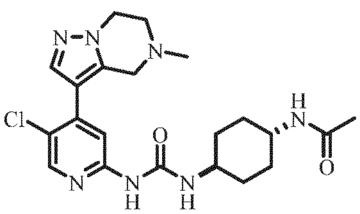 | 84 | 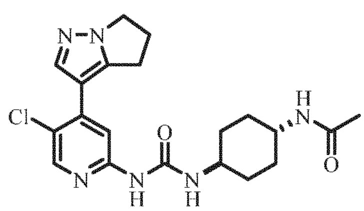 |

(continued)

| No. | Structure | No. | Structure |
|---|---|---|---|
| 85 | 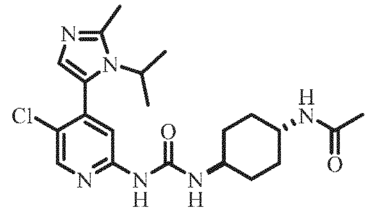 | 86 | 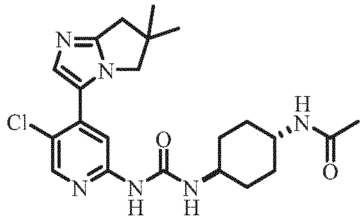 |
| 87 | 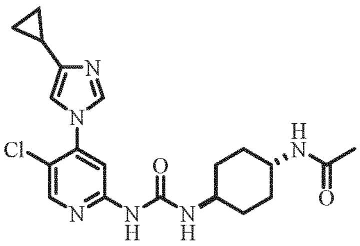 | 88 | 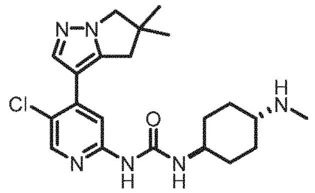 |
| 89 | 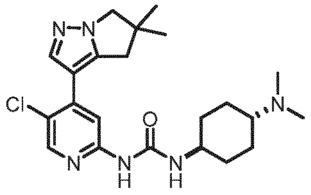 | 90 | 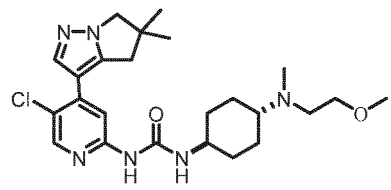 |
| 91 | 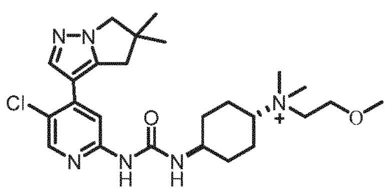 | 92 | 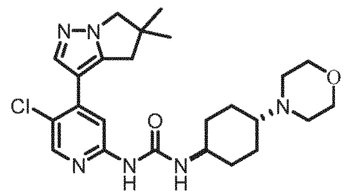 |

In the structures of the preferred compounds of the invention listed in the above table, the two carbon atoms each connected to the two para-position substituents on the cyclohexyl or cyclobutyl are not chiral centers (when there are no substituents on the cyclic group), notation of chemical bond 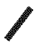 and/or 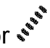 is only to indicate that the two chemical bonds connected to the two substituents at the para position have a trans or cis structure relative to the cyclohexyl and cyclobutyl groups, so the compound represented by exchanging these two chemical bonds with each other also fall within the scope of the present invention. To distinguish it from cis-trans, the present invention uses chemical bonds 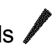 and 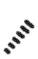 to represent the specific chiral configuration of the carbon atom. According to page 274 of "Chinese Nomenclature of Organic Compounds 2017", "According to CIP rules, 'pseudo-asymmetric carbon atoms' can use lowercase r or s to indicate their configuration", and page 284 "According to the CIP priority system, italics R, S symbol (R, S are derived from the first letter of the Latin Rectus and Sinister, meaning "right" and "left") are used to identify the configuration of the chiral center, axis and plane factors. [Now more widely used to refer to the method for expressing the configuration of various factors such as "Stereogenic"]". In this application, lowercase r and s represents cis-trans isomerism, capital R and S represents the absolute configuration of the chiral center, and chemical formulas are used to represent each specific structure of the isomer. However, it should be understood that enantiomers, diastereomers, racemates and mixtures thereof are all within the scope of the present invention.

The present invention also provides a method for preparing the above compound of formula (I) or its deuterated compound or its pharmaceutically acceptable salt or prodrug, which mainly includes the following steps:

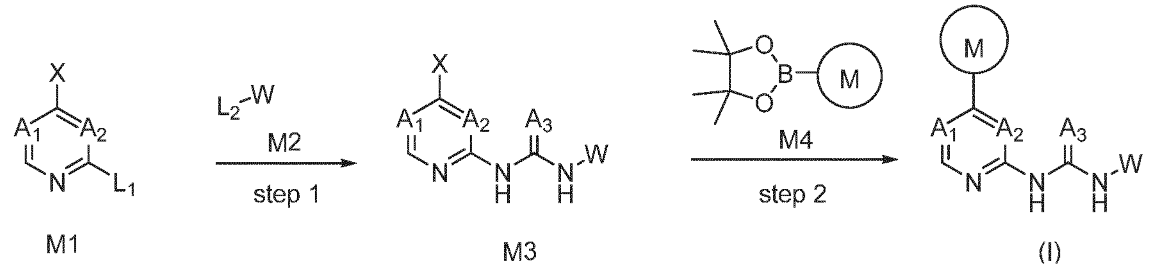


M1 M3 (I)

Wherein $A_1$, $A_2$, $A_3$, M and W are each independently defined as described above; $L_1$ and $L_2$ are amino or carboxyl; X is bromine or chlorine;

Step 1: reacting compound M1 and compound M2 in a solvent to form urea/thiourea linked intermediate M3. Specifically, when $L_1$ and $L_2$ are amino groups, the synthesis method is: compound M1 (or compound M2) firstly forms iso(thio)cyanate or chloroformate, and then reacts with the intermediate amine M2 (or M1) to form (thio)urea-linked intermediate M3; when $L_1$ and $L_2$ are each amino and carboxyl, the urea-linked intermediate M3 can also be prepared using the following synthesis method: compound M1, compound M2, DPPA and alkali (preferably $Et_3N$, DIPEA) are reacted under microwave in anhydrous toluene to form intermediate M3.

Step 2: conducting a coupling reaction between intermediate M3 and boric acid/boric acid pinacol ester M4 to obtain a compound of formula (I) in a solvent under the action of an alkali, a palladium catalyst and a ligand. The solvent is preferably one or more selected from the group of 1,4-dioxane, tetrahydrofuran, toluene, N,N-dimethylformamide, ethanol, ethylene glycol dimethyl ether and water; the palladium metal catalyst is any one of $Pd(PPh_3)_4$, $Pd_2(dba)_3$, $Pd(OAC)_2$ and $(dppf)PdCl_2$; the alkali is any one of $K_2CO_3$, $Cs_2CO_3$, KF, $K_2HPO_4$, $K_3PO_4$, $NaHCO_3$ and $Na_2CO_3$; the ligand is any one of X-Phos and $PCy_3$.

Further preferably, the present invention provides two instructive synthesis schemes (as shown in synthetic routes I and II). It will be appreciated that the reagents/reaction conditions shown in the synthetic schemes can be modified or optimized using general knowledge of organic chemistry to prepare different compounds in the invention.

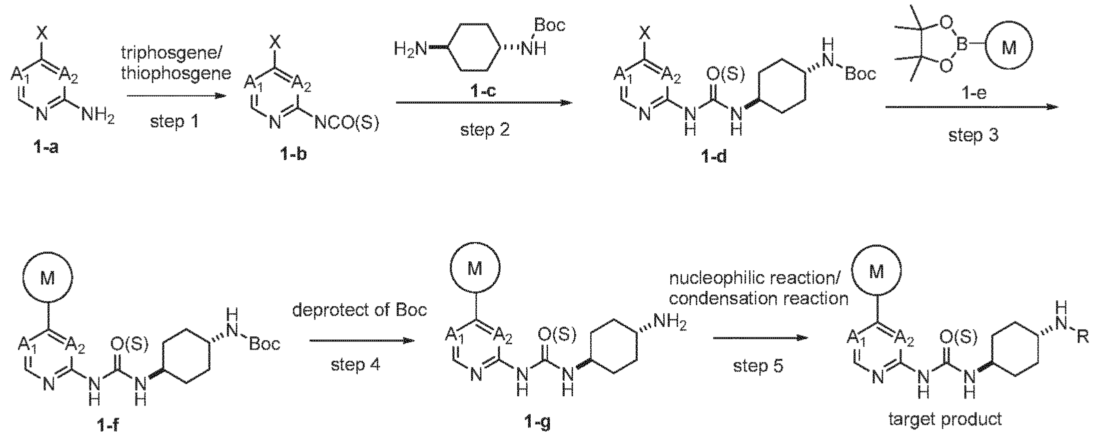


1-a 1-b 1-d 1-f 1-g

Synthetic route I

Synthetic route I includes the following synthesis steps:

Step 1: dissolving intermediate 1-a in a suitable anhydrous solvent, adding triphosgene (or thiophosgene) dropwise in an ice bath, then adding alkali dropwise, and react at room temperature to obtain iso(thio)cyanate intermediate 1-b. The solvent is preferably dichloromethane and tetrahydrofuran; the alkali is preferably triethylamine or N,N-dii sopropylethylamine.

Step 2: adding intermediate 1-b, intermediate 1-c and alkali to a suitable solvent, reacting by heating to reflux to obtain intermediate 1-d. The solvent is preferably tetrahydrofuran or toluene; the alkali is preferably triethylamine or N,N-diisopropylethylamine.

Step 3: adding intermediate 1-d and the corresponding boric acid or boric acid pinacol ester 1-e to a suitable solvent, adding metal palladium catalyst, ligand, and alkali, and heating to react under argon protection to obtain important

intermediate 1-f. The solvent is preferably one or more selected from the group of 1,4-dioxane, tetrahydrofuran, toluene, N,N-dimethylformamide, ethanol, ethylene glycol dimethyl ether and water; the palladium metal catalyst is any one of $Pd(PPh_3)_4$, $Pd_2(dba)_3$, $Pd(OAC)_2$ and $(dppf)PdCl_2$; the alkali is any one of $K_2CO_3$, $Cs_2CO_3$, KF, $K_2HPO_4$, $K_3PO_4$, $NaHCO_3$ and $Na_2CO_3$; the ligand is any one of X-Phos, dppf and $PCy_3$.

Step 4: adding intermediate 1-f to a suitable solvent, adding acid, and reacting at room temperature to obtain amino-deprotected intermediate 1-g. The solvent is preferably dichloromethane or ethyl acetate; the acid is preferably trifluoroacetic acid or hydrochloric acid solution (such as dioxane, ethyl acetate, methanol or aqueous solution).

Step 5: On the one hand, when performing a nucleophilic reaction, dissolving intermediate 1-g with exposed amino groups in a suitable solvent, adding the corresponding halide and alkali, and reacting at room temperature to obtain the target product; the solvent is preferably polar aprotic solvents, such as DMF, DMAC, DMSO or NMP; the alkali is preferably $K_2CO_3$, $Cs_2CO_3$, $Et_3N$ or DIPEA. On the other hand, when performing a condensation reaction, dissolving intermediate 1-g, the corresponding acid, condensation agent and alkali in a suitable solvent, and reacting at room temperature to obtain the target product; or dissolving intermediate 1-g with the corresponding acid chloride and alkali in a suitable solvent, reacting at room temperature to obtain the target product. The solvent is preferably DMF or THF; the condensation agent is preferably HATU, HBTU, TBTU or EDCI; the alkali is preferably $Et_3N$ or DIPEA.

Further preferably, synthetic route I includes the following synthetic steps:

Step 1: adding triphosgene (0.34 eq) or thiophosgene (1.0 eq) and anhydrous DCM to the round-bottom three-neck flask, adding 1-a (1.0 eq) dichloromethane solution dropwise in ice bath, and then adding dropwise $Et_3N$ (2.5 - 5.0 eq), reacting in ice bath or at room temperature for 2-4 hours, then rotary evaporating under reduced pressure to remove the solvent to obtain intermediate 1-b.

Step 2: adding intermediates 1-b (1.0 eq), 1-c (1.0 eq), $Et_3N$ (2.5 - 5.0 eq) and anhydrous toluene into the round bottom flask, and heating to reflux for 6-12 hours. After the reaction is completed, it is cooled to room temperature, the reaction solution is poured into water, and extracted with ethyl acetate. The aqueous phase is repeatedly extracted with ethyl acetate for 2-3 times. The organic phases are combined and washed 2-3 times with saturated NaCl aqueous solution. After drying with anhydrous $Na_2SO_4$, the organic solvent is removed by rotary evaporation under reduced pressure, and intermediate 1-d can be obtained through column chromatography separation and purification.

Step 3: adding intermediate 1-d (1.0 eq), 1-e (1.0-1.5 eq), $Pd(OAc)_2$ (0.1 eq), X-phos (0.1-0.2 eq), $K_2CO_3$ (2.5-3.0 eq) and 1,4-dioxane/water (5/1, v/v) to the round bottom flask, heating to 90°C under argon protection for 4-12 hours. After the reaction is completed, it is cooled to room temperature, the reaction solution is poured into water, and extracted with ethyl acetate. The aqueous phase is repeatedly extracted with ethyl acetate for 2-3 times. The organic phases are combined and washed 2-3 times with saturated NaCl aqueous solution. After drying with anhydrous $Na_2SO_4$, the organic solvent is removed by rotary evaporation under reduced pressure, and the intermediate 1-f can be obtained through column chromatography separation and purification.

Step 4: adding intermediate 1-f (1.0 eq) and DCM to the round-bottomed flask, adding excess dioxane hydrochloride solution, and reacting at room temperature overnight. After the reaction is completed, the solvent is removed by rotary evaporation under reduced pressure to obtain the hydrochloride salt of the intermediate 1-g, which can be directly used in the next step of the reaction.

Step 5: for nucleophilic reaction: adding intermediate 1-g (1.0 eq), corresponding halide (1.0-1.5 eq), $K_2CO_3$ (3.0-5.0 eq) and DMF to the round-bottom flask, reacting at room temperature for 12 hours. After the reaction is completed, it is cooled to room temperature, the reaction solution is poured into water, and extracted with ethyl acetate. The water phase is repeatedly extracted with ethyl acetate for 2-3 times. The organic phases are combined and washed 3 times with water and saturated NaCl aqueous solution, respectively. After drying with anhydrous $Na_2SO_4$, the organic solvent is removed by rotary evaporation under reduced pressure, and the target product can be obtained through column chromatography separation and purification.

For condensation reaction: adding intermediate 1-g (1.0 eq), corresponding acid (1.0 eq), condensation agent HATU (1.0-1.3 eq), DIPEA (2.5-3.0 eq) and DMF to the round bottom flask, reacting at room temperature for 12 hours. After the reaction is completed, it is cooled to room temperature, the reaction solution is poured into water, and extracted with ethyl acetate. The water phase is repeatedly extracted with ethyl acetate for 2-3 times. The organic phases are combined and washed 3 times with water and saturated NaCl aqueous solution, respectively. After drying with anhydrous $Na_2SO_4$, the organic solvent is removed by rotary evaporation under reduced pressure, and the target product can be obtained through column chromatography separation and purification.

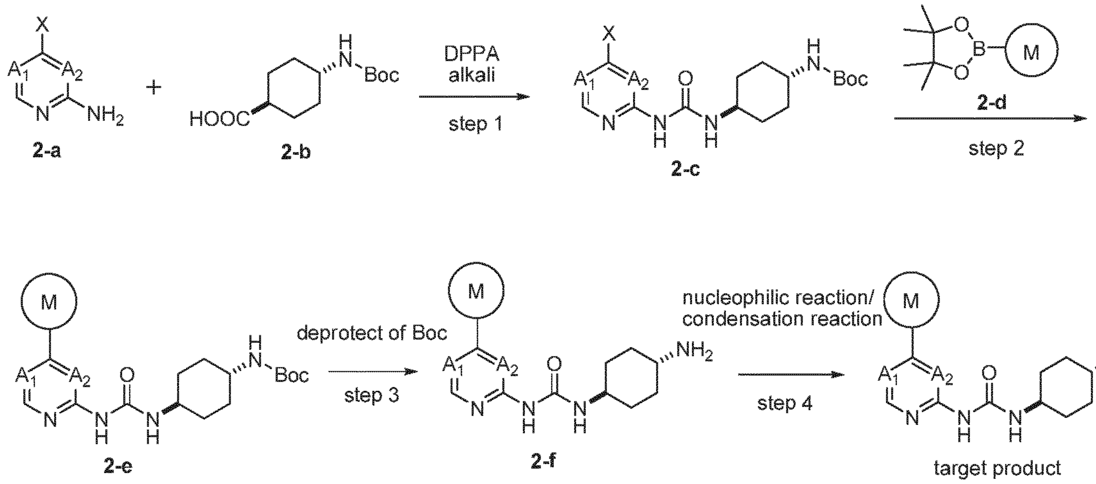


2-a 2-b 2-c 2-d 2-e 2-f

Synthetic route II

Synthetic route II includes the following synthetic steps:

Step 1: reacting by microwave to rearrange the intermediate acid 2-b in a suitable anhydrous solvent under the action of diphenyl phosphate azide (DPPA) and an alkali, and then reacting by microwave with intermediate amine 2-a to form intermediate 2-c. The solvent is preferably toluene; the alkali is preferably $Et_3N$ or DIPEA.

Step 2: adding intermediate 2-c and corresponding boric acid or boric acid pinacol ester 2-d to a suitable solvent, adding metal palladium catalyst, ligand, and alkali, and heating the reaction under argon protection to obtain important intermediate 2-e. The solvent is preferably one or more selected from the group of 1,4-dioxane, tetrahydrofuran, toluene, N,N-dimethylformamide, ethanol, ethylene glycol dimethyl ether and water; the palladium metal catalyst is any one of $Pd(PPh_3)_4$, $Pd_2(dba)_3$, $Pd(OAC)_2$ and $(dppf)PdCl_2$; the alkali is any one of $K_2CO_3$, $Cs_2CO_3$, KF, $K_2HPO_4$, $K_3PO_4$, $NaHCO_3$ and $Na_2CO_3$; the ligand is any one of X-Phos, dppf and $PCy_3$.

Step 3: adding intermediate 2-e to a suitable solvent, adding acid, and reacting at room temperature to obtain amino-deprotected intermediate 2-f. The solvent is preferably dichloromethane or ethyl acetate; the acid is preferably trifluoroacetic acid or hydrochloric acid solution (such as dioxane, ethyl acetate, methanol or aqueous solution).

Step 4: On the one hand, when performing a nucleophilic reaction, dissolving intermediate 2-f with exposed amino groups in a suitable solvent, adding the corresponding halide and alkali, and reacting at room temperature to obtain the target product; the solvent is preferably polar aprotic solvents, such as DMF, DMAC, DMSO or NMP; the alkali is preferably $K_2CO_3$, $Cs_2CO_3$, $Et_3N$ or DIPEA. On the other hand, when performing a condensation reaction, dissolving intermediate 2-f, the corresponding acid, condensation agent and alkali in a suitable solvent, and reacting at room temperature to obtain the target product; or dissolving intermediate 2-f with the corresponding acid chloride and alkali in a suitable solvent, reacting at room temperature to obtain the target product. The solvent is preferably DMF or THF; the condensation agent is preferably HATU, HBTU, TBTU or EDCI; the alkali is preferably $Et_3N$ or DIPEA.

Further preferably, synthetic route II includes the following synthesis steps:

Step 1: adding intermediate 2-b (1.2-1.5 eq), DPPA (1.3-1.5 eq) and $Et_3N$ (3.0-5.0 eq) into the microwave reaction tube, and reacting under microwave at 100°C for 2-5 minutes, then adding intermediate 2-a (1.0 eq) and reacting under microwave at 100°C for 5-30 minutes. After the reaction is completed, cooled to room temperature, the reaction solution is poured into water, and extracted with ethyl acetate. The aqueous phase is repeatedly extracted with ethyl acetate for 2-3 times. The organic phases are combined and washed 2-3 times with saturated NaCl aqueous solution. After drying with anhydrous $Na_2SO_4$, the organic solvent is removed by rotary evaporation under reduced pressure, and intermediate 2-c can be obtained through column chromatography separation and purification.

Step 2: adding intermediate 2-c (1.0 eq), 2-d (1.0-1.5 eq), $Pd(OAc)_2$ (0.1 eq), X-phos (0.1-0.2 eq), $K_2CO_3$ (2.5-3.0 eq) and 1,4-dioxane/water (5/1, v/v) to the round bottom flask, heating to 90°C under argon protection to react for 4-12 hours. After the reaction is completed, it is cooled to room temperature, the reaction solution is poured into water, and extracted with ethyl acetate. The aqueous phase is repeatedly extracted with ethyl acetate for 2-3 times. The organic phases are combined and washed 2-3 times with saturated NaCl aqueous solution. After drying with anhydrous $Na_2SO_4$, the organic solvent is removed by rotary evaporation under reduced pressure, and intermediate

2-e can be obtained through column chromatography separation and purification.

Step 3: adding intermediate 2-e (1.0 eq) and DCM to the round-bottomed flask, adding excess dioxane hydrochloride solution, and reacting at room temperature overnight. After the reaction is completed, the solvent is removed by rotary evaporation under reduced pressure to obtain the hydrochloride salt of the intermediate 2-f, which can be directly used in the next step of the reaction.

Step 4: for nucleophilic reaction: adding intermediate 2-f (1.0 eq), corresponding halide (1.0-1.5 eq), $K_2CO_3$ (3.0-5.0 eq) and DMF to the round-bottom flask, reacting at room temperature for 12 hours. After the reaction is completed, it is cooled to room temperature, the reaction solution is poured into water, and extracted with ethyl acetate. The water phase is repeatedly extracted with ethyl acetate for 2-3 times. The organic phases are combined and washed 3 times with water and saturated NaCl aqueous solution, respectively. After drying with anhydrous $Na_2SO_4$, the organic solvent is removed by rotary evaporation under reduced pressure, and the target product can be obtained through column chromatography separation and purification.

For condensation reaction: adding intermediate 2-f (1.0 eq), corresponding acid (1.0 eq), condensation agent HATU (1.0-1.3 eq), DIPEA (2.5-3.0 eq) and DMF to the round bottom flask, reacting at room temperature for 12 hours. After the reaction is completed, cooled to room temperature, the reaction solution is poured into water, and extracted with ethyl acetate. The water phase is repeatedly extracted with ethyl acetate for 2-3 times. The organic phases are combined and washed 3 times with water and saturated NaCl aqueous solution, respectively. After drying with anhydrous $Na_2SO_4$, the organic solvent is removed by rotary evaporation under reduced pressure, and the target product can be obtained through column chromatography separation and purification.

The present invention also provides a pharmaceutical composition, which is characterized in that it includes one or more of the above-mentioned compound of formula (I) or its deuterated compound or its pharmaceutically acceptable salt or prodrug, and a pharmaceutically acceptable of excipients.

The present invention also provides the use of the above-mentioned compound of formula (I) or its deuterated compound or its pharmaceutically acceptable salt or prodrug or the above-mentioned pharmaceutical composition in the preparation of CDK inhibitors (especially CDK9 inhibitors).

The present invention also provides the use of the above-mentioned compound of formula (I) or its deuterated compound or its pharmaceutically acceptable salt or prodrug or the above-mentioned pharmaceutical composition in the preparation of the medicine for treating, preventing or releasing the diseases, disorders or conditions regulated by the activity of serine kinases, or diseases, disorders or conditions affected by them, or diseases, disorders or conditions involved with the activity of cyclin dependent kinase. The disease, disorder or condition is preferably selected from the group consisting of hyperproliferative diseases (eg tumors), virus-induced infectious diseases and cardiovascular diseases.

The compound of formula (I) of the present invention or its deuterated compound or its pharmaceutically acceptable salt or prodrug, or its pharmaceutical composition is preferably used for the treatment, prevention or improvement of tumors, and the tumors are selected from hematological malignancies (including but not limited to acute myeloid leukemia, multiple myeloma, chronic lymphocytic leukemia, diffuse large B-cell lymphoma, Burkitt's lymphoma, follicular lymphoma), and solid tumors (including but not limited to neuroblastoma, skin cancer, melanoma, lung cancer, gastric cancer, breast cancer, or intestinal cancer).

The compound of formula (I) of the present invention has inhibitory activity against CDK9, and the representative compound has significant in vitro anti-tumor activity against CDK9 high-expressing tumor cells. Representative compounds have plasma stability and low clearance.

## Description of the drawings

Figure 1 shows the effect of compound 47 on the activation of CDK9 signaling pathway in WSU-DLCL2 cells.

## Specific Embodiments

Specific embodiments of the present invention will be described in detail below. It should be understood that the specific embodiments described here are only used to illustrate the present invention and are not intended to limit the present invention.

**(1) Preparation examples of the compounds**

**Example 1**

Preparation of tert-butyl((1r,4r)-4-(3-(4-(4-fluoro-2-methoxyphenyl)pyridin-2-yl)ureido) cyclohexyl) carbamate (Compound 1)

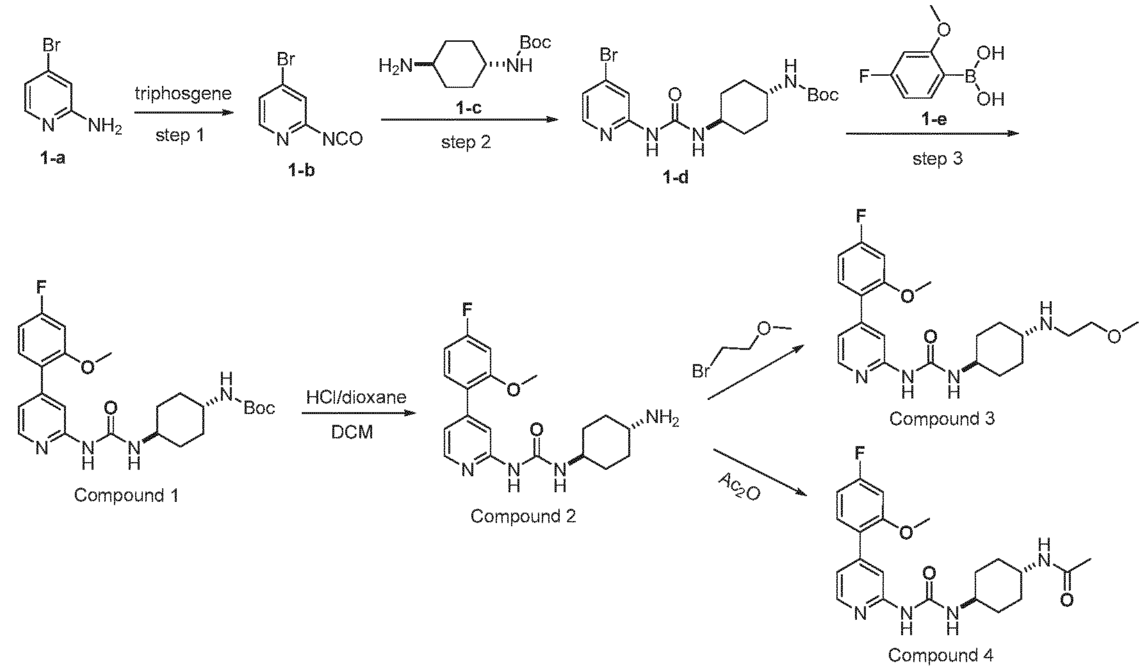


Step 1: triphosgene (583 mg, 1.97 mmol) and anhydrous DCM (40 mL) were added to a round-bottom three-neck flask, and the DCM solution (10 mL) of compound 1-a (1.0 g, 5.78 mmol) was added dropwise in an ice bath, then anhydrous $Et_3N$ (3.21 mL, 23.12 mmol) was added dropwise, and the reaction was stopped after continuing to stir for 2 hours. The solvent was evaporated to dryness under reduced pressure to obtain yellow solid 1-b, which could be directly used in the next reaction without further purification.

Step 2: intermediates 1-b obtained in step 1, 1-c (1.24 g, 5.78 mmol), $Et_3N$ (2.41 mL, 17.34 mmol) and anhydrous toluene (60 mL) were added into the round-bottom flask, and heated to reflux to react overnight. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain light yellow solid 1-d (1.1 g, yield 46.05%). $^1H$ NMR (400 MHz, chloroform-*d*) δ 8.99 (s, 1H), 8.00 (d, *J* = 5.9 Hz, 2H), 7.03 (dd, *J* = 5.5, 1.6 Hz, 1H), 7.00 (s, 1H), 4.44 (d, *J* = 7.9 Hz, 1H), 3.78 - 3.66 (m, 1H), 3.53 - 3.40 (m, 1H), 2.15 - 1.97 (m, 4H), 1.44 (s, 9H), 1.42 - 1.26 (m, 4H). MS 413.1 (M+1).

Step 3: intermediate 1-d (340 mg, 0.823 mmol), 1-e (210 mg, 1.23 mmol), $Pd(OAc)_2$ (18.5 mg, 0.082 mmol), X-phos (78.4 mg, 0.165 mmol), $K_2CO_3$ (284 mg, 2.06 mmol) and $THF/H_2O$ (12/4mL) were added into the round-bottom flask, reacted at 80°C for 4 hours under argon protection. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain target compound 1 (349 mg, yield 92.52%). $^1H$ NMR (400 MHz, chloroform-*d*) δ 9.28 (br s, 1H), 8.17 (d, *J* = 5.4 Hz, 1H), 7.45 (br s, 1H), 7.29 - 7.24 (m, 1H), 7.00 (dd, *J* = 5.4, 1.4 Hz, 1H), 6.84 (s, 1H), 6.79 - 6.69 (m, 2H), 4.43 (d, *J* = 8.2 Hz, 1H), 3.81 (s, 3H), 3.78 - 3.65 (m, 1H), 3.55 - 3.40 (m, 1H), 2.16 - 1.99 (m, 4H), 1.45 (s, 9H), 1.42 - 1.23 (m, 4H). MS 459.2 (M+1).

**Example 2**

Preparation of 1-((1r,4r)-4-aminocyclohexyl)-3-(4-(4-fluoro-2-methoxyphenyl)pyridin-2-yl)urea (compound 2)

Compound 1 (300 mg, 0.654 mmol) and DCM (5 mL) were added to the round-bottomed flask, 4.0 M dioxane hydrochloride solution (0.5 mL) was added, and reacted at room temperature overnight. After the reaction, the organic solvent was evaporated to dryness under reduced pressure, DCM was added to redissolve, 1.0 M NaOH aqueous solution was added dropwise in an ice bath to adjust the pH to weak alkalinity, extracted with DCM/$H_2O$, the organic phases were combined and washed twice with saturated NaCl aqueous solution. After drying over anhydrous $Na_2SO_4$, the solvent was evaporated to dryness under reduced pressure and the resultant was separated by column chromatography to obtain a white solid 2 (223 mg, yield 95.1%). $^1$H NMR (400 MHz, chloroform-*d*) δ 9.21 (s, 1H), 8.12 (d, *J* = 5.4 Hz, 1H), 7.86 (s, 1H), 7.24 (dd, *J* = 6.8, 1.5 Hz, 1H), 6.97 (dd, *J* = 5.4, 1.4 Hz, 1H), 6.93 (s, 1H), 6.75 - 6.65 (m, 2H), 3.79 (s, 3H), 3.76 - 3.63 (m, 1H), 2.87 - 2.77 (m, 1H), 2.35 - 2.15 (m, 2H), 2.17 - 2.07 (m, 2H), 2.02 - 1.90 (m, 2H), 1.42 - 1.32 (m, 4H). MS 359.1 (M+1).

**Example 3**

Preparation of 1-(4-(4-fluoro-2-methoxyphenyl)pyridin-2-yl)-3-((1r,4r)-4-((2-methoxyethyl)amino)cyclohexyl) urea (compound 3)

Compound 2 (100 mg, 0.279 mmol), bromoethyl methyl ether (78 mg, 0.558 mmol), $K_2CO_3$ (116 mg, 0.837 mmol) and DMF (4 mL) were added to the round bottom flask, the reaction was conducted at 50°C overnight. After the reaction was completed, the reaction solution was poured into water and extracted with ethyl acetate. The aqueous phase was extracted with ethyl acetate for 2-3 times. The organic phases were combined and washed 3 times with water and saturated NaCl aqueous solution successively. After drying with anhydrous $Na_2SO_4$, the solvent was evaporated to dryness under reduced pressure, and the resultant was purified by column chromatography to obtain white solid 3 (73 mg, yield 62.82%). $^1$H NMR (400 MHz, chloroform-*d*) δ 9.29 (d, *J* = 7.8 Hz, 1H), 8.15 (d, *J* = 5.4 Hz, 1H), 7.36 (s, 1H), 7.27 (dd, *J* = 6.9, 1.5 Hz, 1H), 6.99 (dd, *J* = 5.4, 1.5 Hz, 1H), 6.83 (s, 1H), 6.78 - 6.69 (m, 2H), 3.82 (s, 3H), 3.74 (dd, *.1* = 7.4, 3.9 Hz, 1H), 3.57 - 3.49 (m, 2H), 3.37 (s, 3H), 2.83 (t, *J* = 5.1 Hz, 2H), 2.52 (dt, *J* = 10.5, 4.9 Hz, 1H), 2.19 - 2.10 (m, 2H), 2.03 - 1.95 (m, 2H), 1.39 - 1.28 (m, 4H). MS 417.2 (M+1).

**Example 4**

Preparation of N-((1r,4r)-4-(3-(4-(4-fluoro-2-methoxyphenyl)pyridin-2-yl)ureido) cyclohexyl)acetamide (compound 4)

Compound 2 (80 mg, 0.223 mmol), $Et_3N$ (56 mg, 0.56 mmol) and DCM (5 mL) were added to the round-bottomed flask, acetic anhydride (30 mg, 0.29 mmol) was added dropwise under ice bath, and stirred at room temperature for 1 hour. After the reaction was completed, the reaction was quenched by adding a small amount of water, poured into water, and extracted with ethyl acetate. The aqueous phase was repeatedly extracted with ethyl acetate for 2-3 times. The organic phases were combined and washed with saturated NaCl aqueous solution twice. After drying with anhydrous $Na_2SO_4$, the solvent was evaporated to dryness under reduced pressure, and the resultant was purified by column chromatography to obtain white solid 4 (72 mg, 80.55%). $^1$H NMR (400 MHz, DMSO-$d_6$) δ 9.17 (s, 1H), 8.23 (d, *J* = 4.9 Hz, 1H), 7.97 (d, *J* = 2.3 Hz, 1H), 7.74 - 7.68 (m, 1H), 7.25 (dd, *J* = 4.8, 2.2 Hz, 1H), 7.15 (dd, *J* = 8.0, 2.2 Hz, 1H), 7.00 (m, 1H), 6.56 (s, 1H), 5.48 (s, 1H), 4.20 - 4.14 (m, 1H), 3.81 (s, 3H), 3.31 - 3.23 (m, 1H), 1.99 (s, 3H), 1.80 - 1.64 (m, 2H), 1.58 - 1.38 (m, 2H), 1.34-1.24 (m, 4H). MS 401.2 (M+1).

**Example 5**

Preparation of 1-((1r,4r)-4-aminocyclohexyl)-3-(4-(4,5-difluoro-2-methoxyphenyl) pyridin-2-yl)urea (compound 5)

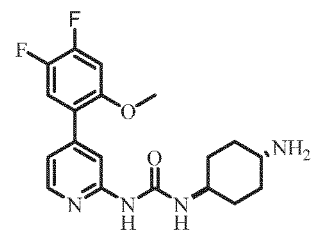

The synthesis method was the same as Example 2 except that 4,5-difluoro-2-methoxyphenylboronic acid was used instead of 4-fluoro-2- methoxyphenylboronic acid (compound 1-e). $^1$H NMR (400 MHz, DMSO-$d_6$) δ 9.15 (s, 1H), 8.19 (d, *J* = 4.9 Hz, 1H), 7.93 (d, *J* = 2.3 Hz, 1H), 7.60 (dd, *J* = 8.0, 4.9 Hz, 1H), 7.21 (dd, *J* = 4.8, 2.2 Hz, 1H), 7.09 (dd, *J* = 8.0, 5.0 Hz, 1H), 6.49 (s, 1H), 3.77 (s, 3H), 3.28 - 3.09 (m, 1H), 2.61 - 2.43 (m, 1H), 1.79 - 1.61 (m, 2H), 1.55 - 1.37 (m, 2H), 1.31 - 1.15 (m, 4H), 1.08 - 0.89 (m, 2H). MS 377.1 (M+1).

**Example 6**

Preparation of 1-((1r,4r)-4-aminocyclohexyl)-3-(4-(4-fluoro-3-methoxyphenyl)pyridin-2-yl)urea (compound 6)

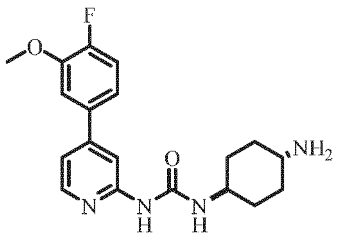

The synthesis method was the same as Example 2 except that 4-fluoro-3-methoxyphenylboronic acid was used instead of 4-fluoro-2- methoxyphenylboronic acid. $^1$H NMR (400 MHz, DMSO-$d_6$) δ 9.16 (s, 1H), 8.20 (d, *J* = 4.9 Hz, 1H), 8.11 (d, *J* = 2.3 Hz, 1H), 7.94 (t, *J* = 8.0 Hz, 1H), 7.60 (dd, *J* = 4.8, 2.2 Hz, 1H), 7.23 (dd, *J* = 4.9, 1.8 Hz, 1H), 7.11 - 7.02 (m, 1H), 6.51 (s, 1H), 3.80 (s, 3H), 3.30 - 3.11 (m, 1H), 2.62 - 2.44 (m, 1H), 1.56 - 1.41 (m, 2H), 1.33 - 1.14 (m, 6H), 1.06 - 0.87 (m, 2H). MS 359.1 (M+1).

**Example 7**

Preparation of 1-((1r,4r)-4-aminocyclohexyl)-3-(4-(2,4-difluoro-5-methoxyphenyl) pyridine-2-yl)urea (compound 7)

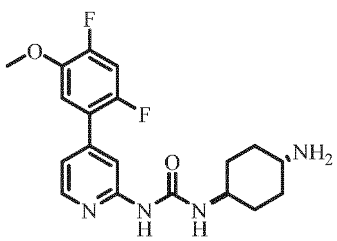

The synthesis method was the same as Example 2 except that 2,4-difluoro-5-methoxyphenylboronic acid was used instead of 4-fluoro-2- methoxyphenylboronic acid. $^1$H NMR (400 MHz, chloroform-*d*) δ 9.17 (s, 1H), 8.41 (s, 1H), 8.28 (d, *J* = 4.8 Hz, 1H), 7.43 (dt, *J* = 4.8, 2.2 Hz, 1H), 7.06 (t, *J* = 8.0 Hz, 1H), 6.87 (s, 1H), 5.71 (s, 1H), 4.82 (s, 2H), 3.81 (s, 3H), 3.24 (br s, 1H), 2.57-2.49 (m, 1H), 1.89 - 1.76 (m, 2H), 1.67 - 1.59 (m, 2H), 1.36 - 1.25 (m, 4H). MS 377.1 (M+1).

**Example 8**

Preparation of 1-((1r, 4r)-4-aminocyclohexyl)-3-(4-(4-fluoro- benzofuran-7-yl)pyridin-2-yl) urea (compound 8)

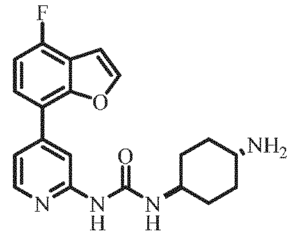

The synthesis method was the same as Example 2 except that 4-fluoro-benzofuran-7-boronic acid was used instead of 4-fluoro-2- methoxyphenylboronic acid. $^1$H NMR (400 MHz, DMSO-$d_6$) δ 9.16 (s, 1H), 8.23 (d, *J* = 4.9 Hz, 1H), 7.97 (d, *J* = 2.3 Hz, 1H), 7.91 (d, *J* = 1.3 Hz, 1H), 7.74 (dd, *J* = 7.9, 5.1 Hz, 1H), 7.25 (dd, *J* = 4.8, 2.2 Hz, 1H), 7.07 - 6.99 (m, 1H), 6.97 (dd, *J* = 4.9, 1.4 Hz, 1H), 6.56 (s, 1H), 3.32 - 3.15 (m, 1H), 2.65 - 2.49 (m, 1H), 1.80 - 1.66 (m, 2H), 1.55 - 1.43 (m, 2H), 1.38 - 1.20 (m, 6H). MS 369.1 (M+1).

**Example 9**

Preparation of 1-((1r,4r)-4-aminocyclohexyl)-3-(4-(3-isopropyl-2-methyl-2H-indazol-5-yl)pyridin-2- yl)urea (compound 9)

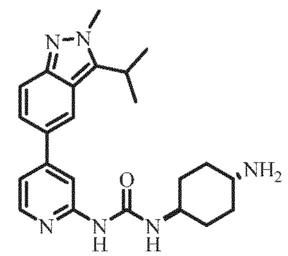

The synthesis method was the same as Example 2 except that 3-isopropyl-2-methyl-2H-indazole-5-boronic acid pinacol ester was used instead of 4-fluoro-2-methoxyphenylboronic acid. $^1$H NMR (400 MHz, DMSO-$d_6$) δ 9.14 (s, 1H), 8.21 (d, *J* = 4.9 Hz, 1H), 8.19 (d, *J* = 2.4 Hz, 1H), 8.03 - 7.94 (m, 2H), 7.45 - 7.39 (m, 1H), 7.25 (dd, *J* = 4.8, 2.2 Hz, 1H), 6.56 (s, 1H), 3.79 (s, 3H), 3.32 - 3.11 (m, 2H), 2.65 - 2.49 (m, 1H), 1.82 - 1.63 (m, 2H), 1.57 - 1.41 (m, 2H), 1.34 - 1.22 (m, 5H), 1.21 (d, *J* = 4.8 Hz, 6H), 1.08 - 0.95 (m, 2H). MS 407.2 (M+1).

**Example 10**

Preparation of 1-((1r,4r)-4-aminocyclohexyl)-3-(4-(2-chloro-4-fluorophenyl)pyridin-2-yl) urea (compound 10)

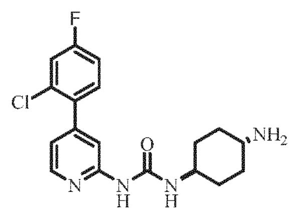

The synthesis method was the same as Example 2 except that 2-chloro-4-fluorophenyl boronic acid was used instead of 4-fluoro-2-methoxyphenyl boronic acid. $^1$H NMR (400 MHz, methanol-$d_4$) δ 8.24 (d, *J* = 5.3 Hz, 1H), 7.48 - 7.31 (m, 2H), 7.21 (td, *J* = 8.4, 2.6 Hz, 1H), 7.16 (d, *J* = 1.5 Hz, 1H), 7.02 (dd, *J* = 5.4, 1.6 Hz, 1H), 3.67 - 3.53 (m, 1H),

3.14 - 3.01 (m, 1H), 2.26 - 1.99 (m, 4H), 1.65 - 1.37 (m, 4H). MS 363.1 (M+1).

**Example 11**

Preparation of 1-(4-(2-chloro-4-fluorophenyl)pyridin-2-yl)-3-((1r,4r)-4-((2-methoxyethyl) amino)cyclohexyl)urea (compound 11)

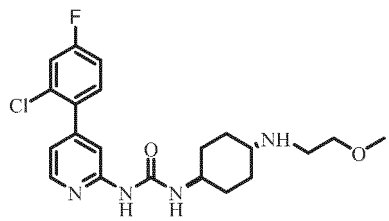

The synthesis method was the same as Example 3 except that 2-chloro-4-fluorophenyl boronic acid was used instead of 4-fluoro-2-methoxyphenyl boronic acid. $^1$H NMR (400 MHz, chloroform-*d*) δ 9.21 (d, *J* = 7.5 Hz, 1H), 8.20 (d, *J* = 5.3 Hz, 1H), 7.76 (s, 1H), 7.33 - 7.19 (m, 2H), 7.07 (td, *J* = 8.2, 2.6 Hz, 1H), 6.92 (dd, *J* = 5.4, 1.5 Hz, 1H), 6.77 (s, 1H), 3.87 - 3.63 (m, 1H), 3.62 - 3.44 (t, *J* = 5.1 Hz, 2H), 3.37 (s, 3H), 2.83 (t, *J* = 5.1 Hz, 2H), 2.61 - 2.41 (m, 1H), 2.24 - 2.06 (m, 2H), 2.05 - 1.91 (m, 2H), 1.45 - 1.14 (m, 5H). MS 421.1 (M+1).

**Example 12**

Preparation of 1-((1r,4r)-4-aminocyclohexyl)-3-(4-(4-fluoro-2-trifluoromethoxyphenyl) pyridin-2-yl) urea (compound 12)

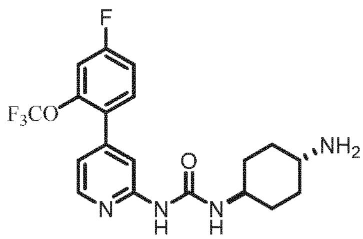

The synthesis method was the same as Example 2 except that 4-fluoro-2-trifluoromethoxyphenyl boronic acid was used instead of 4-fluoro-2- methoxyphenyl boronic acid. $^1$H NMR (400 MHz, DMSO-$d_6$) δ 9.18 (s, 1H), 8.23 (d, *J* = 4.9 Hz, 1H), 7.97 (d, *J* = 2.3 Hz, 1H), 7.71 (dd, *J* = 8.4, 5.0 Hz, 1H), 7.25 (dd, *J* = 4.8, 2.2 Hz, 1H), 7.15 (dd, *J* = 8.0, 2.0 Hz, 1H), 7.00 (m, 1H), 6.58 (s, 1H), 3.32 - 3.21 (m, 1H), 2.85 - 2.74 (m, 1H), 1.89 - 1.74 (m, 2H), 1.55 - 1.45 (m, 2H), 1.33 - 1.05 (m, 6H). MS 413.1 (M+1).

**Example 13**

Preparation of 1-((1r,4r)-4-aminocyclohexyl)-3-(4-(1-methyl-1H-pyrazol-4-yl)pyridin-2-yl) urea (compound 13)

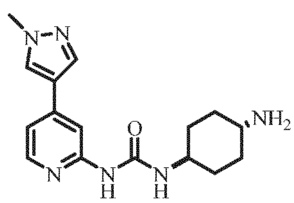

The synthesis method was the same as Example 2 except that 1-methyl-1H-pyrazole-4-boronic acid pinacol ester was used instead of 4-fluoro-2-methoxyphenyl boronic acid. $^1$H NMR (400 MHz, DMSO-$d_6$) $\delta$ 9.19 (s, 1H), 8.23 (s, 1H), 8.18 (brs, 1H), 8.09 (d, *J* = 5.4 Hz, 1H), 7.87 (s, 1H), 7.48 (s, 1H), 7.11 (d, *J* = 5.4 Hz, 1H), 3.88 (s, 3H), 3.45-3.30 (m, 2H), 3.17 (brs, 1H), 3.02-2.90 (m, 1H), 1.96-1.82 (m, 4H), 1.59 - 1.19 (m, 4H). MS 315.2 (M+1).

**Example 14**

Preparation of 1-((1r,4r)-4-((2-methoxyethyl)amino)cyclohexyl)-3-(4-(1-methyl-1H-pyrazol-4-yl)pyridine-2-yl) urea (compound 14)

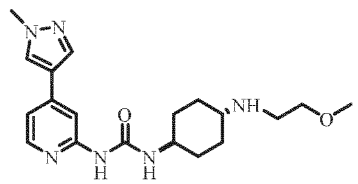

The synthesis method was the same as Example 3 except that 1-methyl-1H-pyrazole-4-boronic acid pinacol ester was used instead of 4-fluoro-2-methoxyphenyl boronic acid. $^1$H NMR (400 MHz, methanol-$d_4$) $\delta$ 8.14 - 8.06 (m, 2H), 7.92 (brs, 1H), 7.20 (brs, 1H), 7.13 (dd, *J* = 5.5, 1.6 Hz, 1H), 3.94 (s, 3H), 3.65-3.58 (m, 1H), 3.60 (t, *J* = 5.7, 4.5 Hz, 2H), 3.40 (s, 3H), 3.08 (t, *J* = 5.1 Hz, 2H), 2.91-2.80 (m, 1H), 2.23 - 2.07 (m, 4H), 1.53-1.40 (m, 4H). MS 373.2 (M+1).

**Example 15**

Preparation of 1-((1r,4r)-4-aminocyclohexyl)-3-(4-(5,5-dimethyl-5,6-dihydro-4H-pyrrole[1,2-b]pyrazole-3-yl)pyridin-2-yl) urea (compound 15)

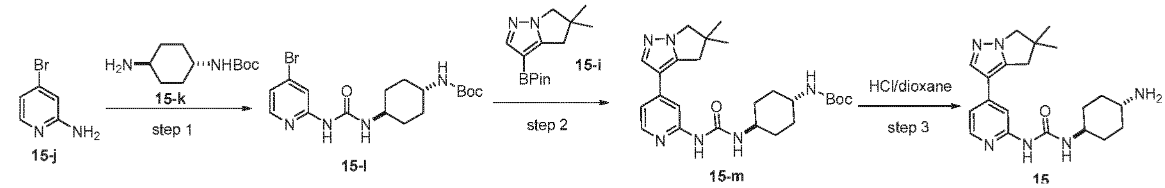


Step 1: triphosgene (422 mg, 1.97 mmol) and anhydrous DCM (40 mL) were added to a round-bottom three-neck flask, and the DCM solution (20 mL) of compound **15-j** (1.0 g, 5.78 mmol) was added dropwise in an ice bath, then anhydrous $Et_3N$ (4.02 mL, 28.90 mmol) was added dropwise, and the reaction was stopped after continuing to stir for 2 hours. After the organic solvent was removed by rotary evaporation under reduced pressure, a light yellow solid was obtained. The above intermediate, **15-k** (1.24 g, 5.78 mmol), $Et_3N$ (3.21 mL, 23.12 mmol) and anhydrous toluene (70 mL) were added to the round-bottom flask, and heated to reflux to react overnight. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain light yellow solid **15-1** (1.5 g, yield 62.79%). $^1$H NMR (400 MHz, chloroform-*d*) $\delta$ 8.99 (s, 1H), 8.00 (d, *J* = 5.9 Hz, 2H), 7.03 (dd, *J* = 5.5, 1.6 Hz, 1H), 7.00 (s, 1H), 4.44 (d, *J* = 7.9 Hz, 1H), 3.78 - 3.66 (m, 1H), 3.53 - 3.40 (m, 1H), 2.15 - 1.97 (m, 4H), 1.44 (s, 9H), 1.42 - 1.26 (m, 4H). MS 413.1 (M+1).

Step 2: intermediate **15-1** (150 mg, 0.36 mmol), **15-i** (114 mg, 0.44 mmol), $Pd_2dba_3$ (12 mg, 0.054 mmol), X-phos (34.6 mg, 0.073 mmol), $K_2HPO_4$ (190 mg, 1.09 mmol) and dioxane/$H_2O$ (10/2 mL) were added into the round-bottom flask, and reacted at 85°C for 6 hours under argon protection. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain light yellow solid **15-m** (80 mg,

yield 47.04%). $^1$H NMR (400 MHz, DMSO-$d_6$) δ 9.20 (s, 1H), 8.23 (d, *J* = 5.0 Hz, 1H), 8.15 (s, 1H), 7.95 (d, *J* = 2.2 Hz, 1H), 7.26 (dd, *J* = 4.9, 2.2 Hz, 1H), 6.64 (s, 1H), 3.95 (s, 2H), 3.33 - 3.16 (m, 1H), 2.94 (s, 2H), 2.67 - 2.52 (m, 1H), 1.92 - 1.78 (m, 2H), 1.67 - 1.53 (m, 2H), 1.46 - 1.34 (m, 11H), 1.30 (s, 6H), 1.28 - 1.09 (m, 4H). MS 469.3 (M+1).

Step 3: Compound **15-m** (80 mg, 0.17 mmol) and DCM (5 mL) were added to the round-bottomed flask, 4.0 M dioxane solution of hydrochloride (0.3 mL) was added, and reacted at room temperature overnight. After the reaction was completed, the organic solvent was evaporated to dryness under reduced pressure, methanol was added to redissolve, and 1.0M NaOH aqueous solution was added dropwise in an ice bath to adjust the pH to weak alkalinity. The solvent was evaporated to dryness under reduced pressure, and the resultant was separated by column chromatography to obtain a white solid 15 (50 mg, yield 79.48%). $^1$H NMR (400 MHz, DMSO-$d_6$) δ 9.21 (s, 1H), 8.23 (d, *J* = 4.9 Hz, 1H), 8.16 (s, 1H), 7.97 (d, *J* = 2.3 Hz, 1H), 7.25 (dd, *J* = 4.8, 2.2 Hz, 1H), 6.56 (s, 1H), 3.91 (s, 2H), 3.33 - 3.16 (m, 1H), 2.91 (s, 2H), 2.66 - 2.50 (m, 1H), 1.92 - 1.78 (m, 2H), 1.67 - 1.52 (m, 2H), 1.46 - 1.37 (m, 4H), 1.29 (s, 6H), 1.23 - 1.12 (m, 2H). MS 369.2 (M+1).

Wherein the intermediate 5,5-dimethyl-3-(4,4,5,5-tetramethyl-1,3,2-dioxaborolan-2-yl)-5,6-dihydro-4H-pyrrolo[1,2-b]pyrazole (**15-i**) was prepared as follows:

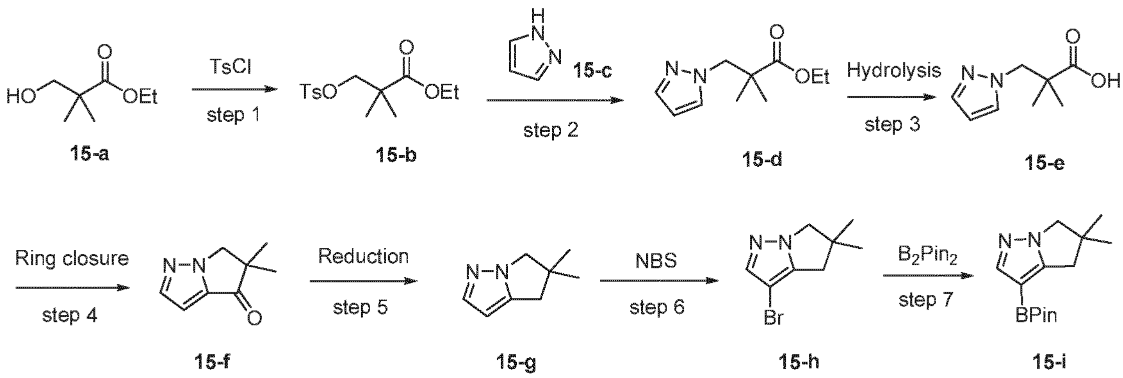


Step 1: intermediate **15-a** (25.0 g, 171.02 mmol), $Et_3N$ (34.61 g, 342.03 mmol), DMAP (1.04 g, 8.55 mmol) and anhydrous THF (200 mL) were added into the round bottom flask, and TsCl (35.86 g, 188.12 mmol) was added in three batches under ice bath. The temperature was raised to room temperature, and then the reaction was refluxed at 80°C overnight. After the reaction was completed, most of the solvent was removed by rotary evaporation under reduced pressure, the resultant was poured into water, extracted with ethyl acetate, and the water phase was repeatedly extracted with ethyl acetate 2-3 times. The organic phases were combined and washed twice with saturated NaCl aqueous solution, dried over anhydrous $Na_2SO_4$, the solvent was evaporated to dryness under reduced pressure, and purified by column chromatography to obtain colorless oil **15-b** (31.0 g, yield 60.35%). $^1$H NMR (400 MHz, chloroform-*d*) δ 7.77 (d, *J* = 8.3 Hz, 1H), 7.34 (d, *J* = 8.1 Hz, 1H), 4.07 (q, *J* = 7.1 Hz, 1H), 4.00 (s, 1H), 2.45 (s, 2H), 1.19 (t, *J* = 7.0 Hz, 1H), 1.17 (s, 6H). MS 301.1 (M+1).

Step 2: intermediate **15-b** (31.0 g, 103.21 mmol), pyrazole (9.13 g, 134.17 mmol), cesium carbonate (100.88 g, 309.62 mmol), anhydrous DMF (100 mL) were added into the round bottom flask, and reacted at 100°C overnight. After the reaction was completed, it was cooled to room temperature, most of the solvent was evaporated to dryness under reduced pressure, then the reaction solution was poured into water, extracted with ethyl acetate, and the water phase was repeatedly extracted with ethyl acetate 2-3 times. The organic phases were combined and washed three times with water and saturated NaCl aqueous solution successively, dried over anhydrous $Na_2SO_4$, the solvent was evaporated to dryness under reduced pressure, and the resultant was purified by column chromatography to obtain colorless oil 15-d (11.2 g, yield 55.3%). $^1$H NMR (400 MHz, chloroform-*d*) δ 7.44 (s, 1H), 7.34 (d, *J* = 2.3 Hz, 1H), 6.19 (t, *J* = 2.4 Hz, 1H), 4.27 (s, 2H), 4.13 (q, *J* = 7.3 Hz, 2H), 1.23 (t, *J* = 7.1 Hz, 4H), 1.17 (s, 6H). MS 197.1 (M+1).

Step 3: **15-d** (11.2 g, 57.07 mmol) and methanol (60 mL) were added to the round bottom flask, NaOH aqueous solution (5 M, 22.83 mL, 114.14 mmol) was added thereto dropwise, and reacted at 50°C overnight. After the reaction was completed, it was cooled to room temperature, 6M hydrochloric acid was used to adjust pH=3 in an ice bath, the solvent was evaporated to dryness under reduced pressure, and the resultant was separated by column chromatography to obtain the light-yellow oily substance. After standing, it solidifies into a white crystalline solid 15-e (9.2 g, yield 95.8%). $^1$H NMR (400 MHz, DMSO-$d_6$) δ 12.47 (s, 1H), 7.61 (d, *J* = 2.3 Hz, 1H), 7.41 (d, *J* = 1.8 Hz, 1H), 6.22 (t, *J* = 2.1 Hz, 1H), 4.24 (s, 2H), 1.05 (s, 6H). MS 169.1 (M+1).

Step 4: intermediate 15-e (9.2 g, 54.7 mmol) and anhydrous THF (100 mL) were added to the round bottom three-neck flask, n-BuLi (1.6 M, 70.08 mL, 112.13 mmol) was added dropwise at -78°C, the resultant was stirred for 30 minutes, warmed to -45°C and stirred for 2 hours, then warmed to 15 °C and stirred for 1 hour to complete the reaction. The reaction was quenched by dropwise adding saturated $NH_4Cl$ aqueous solution at 0°C. The reaction solution was

poured into saturated $NH_4Cl$ aqueous solution, the organic phases were seperated. The aqueous phase was extracted 3 times with ethyl acetate. The organic phases were combined and washed 2 times with saturated NaCl aqueous solution, the resultant was evaporated to dryness under reduced pressure to obtain yellow oil 15-f (3.21 g, yield 39.08%). $^1H$ NMR (400 MHz, chloroform-*d*) δ 7.79 (s, 1H), 6.65 (s, 1H), 4.28 (s, 2H), 1.38 (s, 6H). MS 151.1 (M+1).

Step 5: intermediate 15-f (3.21 g, 21.37 mmol), 85% hydrazine hydrate (6.11 mL, 106.87 mmol) and diethylene glycol (45 mL) were added into the round bottom flask, and reacted at 180°C for 1 hour. The excess hydrazine hydrate was removed by rotary evaporation under reduced pressure, then KOH was added, and reacted at 150°C for 3 hours. The reaction was cooled to room temperature, 50 mL of water was added to dilute, 2N hydrochloric acid aqueous solution was used under ice bath to adjust the pH to 4.5, the resultant was extracted with diethyl ether (5 x 50 mL), the organic phases were combined and washed with water (3 x 20 mL) and saturated NaCl aqueous solution (2 x 20 mL) successively, dried over anhydrous $Na_2SO_4$ and filtered, and the solvent was evaporated to dryness under reduced pressure to obtain yellow oily product 15-g (1.84 g, yield 63.3%). $^1H$ NMR (400 MHz, chloroform-*d*) δ 7.48 (d, *J* = 1.8 Hz, 1H), 5.92 (s, 1H), 3.86 (s, 2H), 2.67 (s, 2H), 1.27 (s, 6H). MS 137.1 (M+1).

Step 6: intermediate 15-g (1.84 g, 13.51 mmol) and DCM (20 mL) were added into the round bottom flask, NBS (2.4 g, 13.51 mmol) was added under ice bath, and reacted at room temperature overnight. After the reaction was completed, DCM (20 mL) was added to dilute the reaction solution, the resultant was washed with water (2 x 20 mL) and saturated NaCl aqueous solution (2 x 20 mL) in sequence, dried over anhydrous $Na_2SO_4$ and filtered, and the solvent was evaporated to dryness under reduced pressure to obtain yellow oily product 15-h (1.93 g, yield 66.28%). $^1H$ NMR (400 MHz, chloroform-*d*) δ 7.41 (s, 1H), 3.89 (s, 2H), 2.64 (s, 2H), 1.29 (s, 6H). 215.1 (M+1).

Step 7: intermediate 15-h (1.93 g, 8.95 mmol), AcOK (2.2 g, 22.39 mmol) and 1,4-dioxane (25 mL) were added to the round bottom flask, the oxygen was removed thoroughly under argon protection. $PCy_3$ (502 mg, 1.79 mmol) and $Pd(OAc)_2$ (201 mg, 0.90 mol) were added, and oxygen was removed thoroughly, and the reaction was conducted at 90°C for 3 hours. After cooling to room temperature, the reaction solution was poured into water, extracted with ethyl acetate, the organic phases were combined and washed twice with saturated NaCl aqueous solution, dried over anhydrous $Na_2SO_4$, the solvent was evaporated to dryness under reduced pressure, and the resultant was separated and purified by column chromatography to obtain a yellow semi-solid product 15-i (1.9 g, yield 80.94%). $^1H$ NMR (400 MHz, chloroform-*d*) δ 7.42 (s, 1H), 3.90 (s, 2H), 2.64 (s, 2H), 1.29 (s, 6H). 263.2 (M+1).

**Example 16**

Preparation of 1-((1r,4r)-4-(3-(4-(5,5-dimethyl-5,6-dihydro -4H-pyrrolo[1,2-b]pyrazol-3-yl)pyridin-2-yl)ureido)cyclohexyl)acetamide (compound 16)

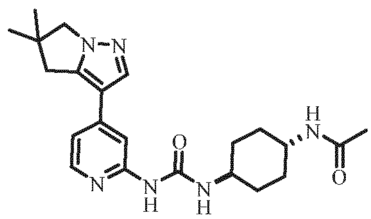

The synthesis method was the same as Example 4 except that compound 15 was used instead of compound 2. $^1H$ NMR (400 MHz, DMSO-$d_6$) δ 9.19 (s, 1H), 8.21 (d, *J* = 4.9 Hz, 1H), 8.16 (s, 1H), 7.97 (d, *J* = 2.3 Hz, 1H), 7.25 (dd, *J* = 4.8, 2.2 Hz, 1H), 6.56 (s, 1H), 5.50 (s, 1H), 4.11-4.02 (m, 1H), 3.79 (s, 2H), 3.24-3.12 (m, 1H), 2.81 (s, 2H), 2.04 (s, 3H), 1.88 - 1.71 (m, 4H), 1.34 - 1.26 (m, 4H), 1.24 (s, 6H). MS 411.2 (M+1).

**Example 17**

Preparation of 1-(4-(5,5-dimethyl-5,6-dihydro-4H-pyrrolo[1,2-b]pyrazol-3-yl)pyridin-2-yl)-3-((1r,4r)-4-((2-methoxyethyl)amino)cyclohexyl)urea (compound 17)

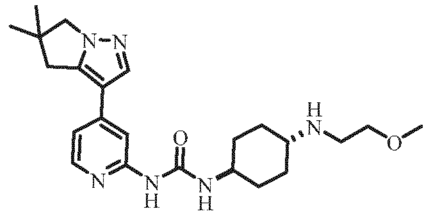

The synthesis method was the same as Example 3 except that compound 15 was used instead of compound 2. $^1$H NMR (400 MHz, DMSO-$d_6$) δ 9.20 (s, 1H), 8.23 (d, *J* = 4.9 Hz, 1H), 8.16 (s, 1H), 7.97 (d, *J* = 2.3 Hz, 1H), 7.25 (dd, *J* = 4.8, 2.2 Hz, 1H), 6.53 (s, 1H), 3.89 (s, 2H), 3.74 - 3.68 (m, 1H), 3.53 (t, *J* = 5.4 Hz, 2H), 3.27 (s, 3H), 3.26 - 3.18 (m, 1H), 2.84 (s, 2H), 2.76 (dt, *J* = 6.4, 5.5 Hz, 2H), 2.50 - 2.35 (m, 1H), 1.81 - 1.69 (m, 2H), 1.56 - 1.44 (m, 4H), 1.32 (s, 6H), 1.24 - 1.09 (m, 2H). MS 427.1 (M+1).

**Example 18**

Preparation of 1-((1r,4r)-4-aminocyclohexyl)-3-(5-chloro-4-(4-fluoro-2-methoxyphenyl) pyridin-2-yl)urea (compound 18)

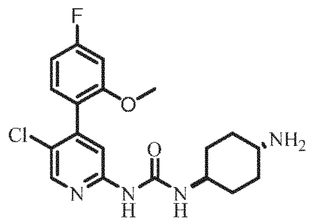

The synthesis method was the same as Example 2 except that 5-chloro-4-bromo-2-aminopyridine was used instead of 4-bromo-2-aminopyridine (compound 1-a). $^1$H NMR (400 MHz, DMSO-$d_6$) δ 9.25 (s, 1H), 8.26 (s, 1H), 7.84 (brs, 2H), 7.56-7.48 (m, 2H), 7.21 (dd, *J* = 8.4, 6.8 Hz, 1H), 7.08 (dd, *J* = 11.4, 2.5 Hz, 1H), 6.90 (td, *J* = 8.4, 2.5 Hz, 1H), 3.76 (s, 3H), 3.53 - 3.38 (m, 1H), 3.10-3.07 (m, 1H), 1.94 (d, *J* = 11.2 Hz, 4H), 1.61 - 1.14 (m, 4H). MS 393.1 (M+1).

**Example 19**

Preparation of 1-(5-chloro-4-(4-fluoro-2-methoxyphenyl)pyridin-2-yl)-3-((1r,4r)-4-((2-methoxyethyl)amino)cyclohexyl)urea (compound 19)

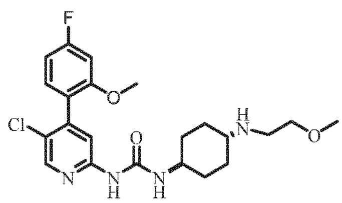

The synthesis method was the same as Example 3 except that 5-chloro-4-bromo-2-aminopyridine was used instead of 4-bromo-2-aminopyridine. $^1$H NMR (400 MHz, methanol-$d_4$) δ 8.22 (s, 1H), 7.18 (dd, *J* = 8.4, 6.6 Hz, 1H), 7.10 (s, 1H), 6.92 (dd, *J* = 11.1, 2.4 Hz, 1H), 6.80 (td, *J* = 8.3, 2.4 Hz, 1H), 3.80 (s, 3H), 3.71 - 3.57 (m, 3H), 3.43 (s, 3H), 3.22 (t, J = 5.0 Hz, 2H), 3.17 - 3.03 (m, 1H), 2.24-2.11(m, 5H), 1.66 - 1.36 (m, 4H). MS 451.1 (M+1).

**Example 20**

Preparation of 1-(5-chloro-4-(4-fluoro-2-methoxyphenyl)pyridin-2-yl)-3-((1r,4r)-4-((2-hydroxyethyl)amino)cyclohexyl)urea (compound 20)

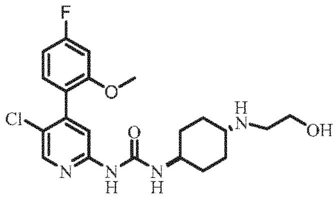

The synthesis method was the same as Example 3 except that 5-chloro-4-bromo-2-aminopyridine was used instead of 4-bromo-2-aminopyridine, and 2-bromoethanol was used instead of 2-bromoethyl methyl ether. $^1H$ NMR (400 MHz, DMSO-$d_6$) δ 9.22 (s, 1H), 8.27 (s, 1H), 7.56-7.47 (m, 2H), 7.21 (dd, *J* = 8.4, 6.8 Hz, 1H), 7.08 (dd, *J* = 11.4, 2.5 Hz, 1H), 6.91 (m, 1H), 4.80 (t, *J* = 6.4 Hz, 1H), 3.95 - 3.87 (m, 2H), 3.81 (s, 3H), 3.75 - 3.67 (m, 1H), 3.33 - 3.18 (m, 1H), 2.78 (td, *J* = 7.5, 6.6 Hz, 2H), 2.51 - 2.38 (m, 1H), 1.81 - 1.60 (m, 4H), 1.24 - 1.09 (m, 4H). MS 437.1 (M+1).

**Example 21**

Preparation of 1-(5-chloro-4-(4-fluoro-2-methoxyphenyl)pyridin-2-yl)-3-((1r,4r)-4-((1-methoxypropan-2-yl)amino)cyclohexyl)urea (compound 21)

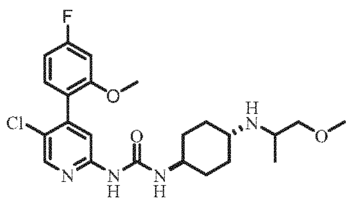

The synthesis method was the same as Example 3 except that 5-chloro-4-bromo-2-aminopyridine was used instead of 4-bromo-2-aminopyridine, and 2-bromo-1-methoxypropane was used instead of 2-bromoethyl methyl ether. $^1H$ NMR (400 MHz, DMSO-$d_6$) δ 9.21 (s, 1H), 8.26 (s, 1H), 8.16 (s, 1H), 7.71 (dd, *J* = 8.5, 5.0 Hz, 1H), 7.15 (dd, *J* = 8.0, 2.2 Hz, 1H), 7.00 (td, *J* = 8.2, 2.2 Hz, 1H), 6.56 (s, 1H), 3.81 (s, 3H), 3.72 (br s, 1H), 3.47 (d, *J* = 7.0 Hz, 2H), 3.27 (s, 3H), 3.26 - 3.18 (m, 1H), 2.97 - 2.89 (m, 1H), 2.43 - 2.35 (m, 1H), 1.78 - 1.70 (m, 2H), 1.53 - 1.44 (m, 2H), 1.19 - 1.10 (m, 2H), 0.94 (d, *J* = 6.6 Hz, 3H), 0.92 - 0.85 (m, 2H). MS 465.2 (M+1).

**Example 22**

Preparation of N-((1r,4r)-4-(3-(5-chloro-4-(4-fluoro-2-methoxyphenyl)pyridin-2-yl) ureido)cyclohexyl)acetamide (compound 22)

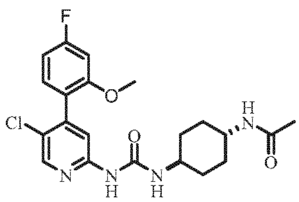

The synthesis method was the same as Example 4 except that 5-chloro-4-bromo-2-aminopyridine was used instead of 4-bromo-2-aminopyridine. $^1$H NMR (400 MHz, DMSO-$d_6$) δ 9.21 (s, 1H), 8.40 (s, 1H), 8.17 (s, 1H), 7.72 (dd, *J* = 8.5, 5.0 Hz, 1H), 7.15 (dd, *J* = 8.0, 2.2 Hz, 1H), 7.02 - 6.95 (m, 1H), 6.64 (s, 1H), 5.71 (s, 1H), 4.21 - 4.06 (m, 1H), 3.81 (s, 3H), 3.36 - 3.19 (m, 1H), 1.99 (s, 3H), 1.79 - 1.70 (m, 2H), 1.54 - 1.45 (m, 2H), 1.34 - 1.23 (m, 4H). MS 435.1 (M+1).

**Example 23**

Preparation of 1-(5-chloro-4-(4-fluoro-2-methoxyphenyl)pyridin-2-yl)-3-((1r,4r)-4-(isopropylamino)cyclohexyl)urea (compound 23)

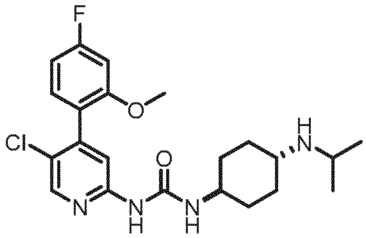

The synthesis method was the same as Example 3 except that 5-chloro-4-bromo-2-aminopyridine was used instead of 4-bromo-2-aminopyridine, and 2-bromopropane was used instead of 2-bromoethyl methyl ether. $^1$H NMR (400 MHz, DMSO-$d_6$) δ 9.15 (s, 1H), 8.38 (s, 1H), 8.16 (s, 1H), 7.69 (dd, *J* = 8.5, 5.1 Hz, 1H), 7.15 (d, *J* = 8.0 Hz, 1H), 7.07 - 6.93 (m, 1H), 6.56 (s, 1H), 3.81 (s, 3H), 3.32 - 3.18 (m, 1H), 2.87 - 2.76 (m, 1H), 2.46 - 2.33 (m, 1H), 1.80 - 1.68 (m, 2H), 1.56 - 1.43 (m, 3H), 1.32 (d, *J* = 4.9 Hz, 6H), 1.21 - 1.09 (m, 4H). MS 435.2 (M+1).

**Example 24**

Preparation of 1-(5-chloro-4-(4-fluoro-2-methoxyphenyl)pyridin-2-yl)-3-((1r,4r)-4-(cyclopropylamino)cyclohexyl)urea (compound 24)

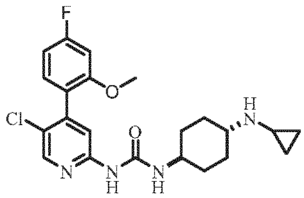

The synthesis method was the same as Example 3 except that 5-chloro-4-bromo-2-aminopyridine was used instead of 4-bromo-2-aminopyridine, and bromocyclopropane was used instead of 2-bromoethyl methyl ether. $^1$H NMR (400 MHz, DMSO-$d_6$) δ 9.16 (s, 1H), 8.42 (s, 1H), 8.15 (s, 1H), 7.69 (dd, *J* = 8.3, 4.9 Hz, 1H), 7.15 (dd, *J* = 8.0, 2.2 Hz, 1H), 6.98 (td, *J* = 8.2, 2.0 Hz, 1H), 6.59 (s, 1H), 3.80 (s, 3H), 3.35 - 3.29 (m, 1H), 2.49 - 2.30 (m, 2H), 1.93 - 1.66 (m, 5H), 1.42 - 1.37 (m, 4H), 0.58 - 0.34 (m, 4H). MS 433.1 (M+1).

**Example 25**

Preparation of 1-(5-chloro-4-(4-fluoro-2-methoxyphenyl)pyridin-2-yl)-3-((1r,4r)-4-((tetrahydrofuran-3-yl)amino)cyclohexyl)urea (compound 25)

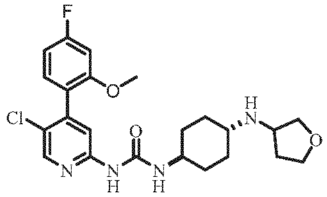

The synthesis method was the same as Example 3 except that 5-chloro-4-bromo-2-aminopyridine was used instead of 4-bromo-2-aminopyridine, and 3-bromotetrahydrofuran was used instead of 2-bromoethyl methyl ether. $^1H$ NMR (400 MHz, DMSO-$d_6$) δ 9.18 (s, 1H), 8.26 (s, 1H), 8.13 (s, 1H), 7.82 - 7.72 (m, 1H), 7.22 (dd, *J* = 8.0, 2.2 Hz, 1H), 7.14 - 7.01 (m, 1H), 6.61 (s, 1H), 4.07 - 3.98 (m, 2H), 3.88 - 3.73 (m, 6H), 3.39 - 3.26 (m, 1H), 3.10 - 3.00 (m, 1H), 2.53 - 2.41 (m, 1H), 2.17 -1.92 (m, 4H), 1.72 - 1.61 (m, 2H), 1.27 - 1.16 (m, 4H). MS 463.2 (M+1).

**Example 26**

Preparation of 1-(5-chloro-4-(4-fluoro-2-methoxyphenyl)pyridin-2-yl)-3-((1r,4r)-4-((3,3,3-trifluoropropyl)amino)cyclohexyl)urea (compound 26)

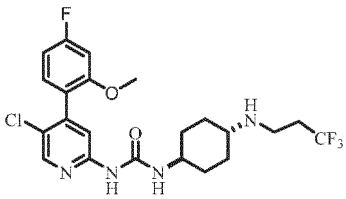

The synthesis method was the same as Example 3 except that 5-chloro-4-bromo-2-aminopyridine was used instead of 4-bromo-2-aminopyridine, and 3,3,3-trifluoro-1-bromopropane was used instead of 2-bromoethyl methyl ether. $^1H$ NMR (400 MHz, DMSO-$d_6$) δ 9.21 (s, 1H), 8.28 (s, 1H), 8.16 (s, 1H), 7.71 (dd, *J* = 8.5, 5.0 Hz, 1H), 7.17 - 6.95 (m, 2H), 6.59 (s, 1H), 3.81 (s, 3H), 3.30 - 3.18 (m, 1H), 2.59 - 2.51 (m, 2H), 2.49 - 2.37 (m, 1H), 2.01 - 1.88 (m, 2H), 1.80 - 1.40 (m, 5H), 1.32 - 1.03 (m, 4H). MS 489.1 (M+1).

**Example 27**

Preparation of 1-(5-chloro-4-(4-fluoro-2-methoxyphenyl)pyridin-2-yl)-3-((1r,4r)-4-((2-(dimethylamino)ethyl)amino)cyclohexyl)urea (compound 27)

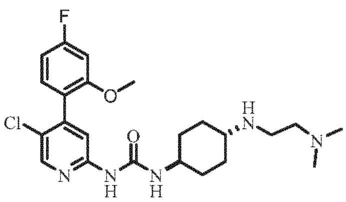

The synthesis method was the same as Example 3 except that 5-chloro-4-bromo-2-aminopyridine was used instead of 4-bromo-2-aminopyridine, and (2-bromomethyl) dimethylamine was used instead of 2-bromoethyl methyl ether. $^1H$ NMR (400 MHz, DMSO-$d_6$) δ 9.18 (s, 1H), 8.27 (s, 1H), 8.17 (s, 1H), 7.72 (m, 1H), 7.20 - 6.98 (m, 2H), 6.55 (br s, 1H), 3.82 (s, 3H), 3.32 - 3.20 (m, 1H), 2.58 (s, 6H), 2.55 - 2.37 (m, 5H), 1.81 - 1.39 (m, 5H), 1.31 - 1.09 (m, 4H). MS 464.2 (M+1).

**Example 28**

Preparation of N-((1r,4r)-4-(3-(5-chloro-4-(4-fluoro-2-methoxyphenyl)pyridin-2-yl) ureido)cyclohexyl)-2-(dimethylamino)acetamide (compound 28)

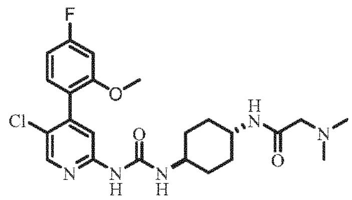

The compounds N,N-dimethylglycine (32 mg, 0.31 mmol), HATU (126 mg, 0.33 mmol) and DIPEA (0.21 mL, 1.27 mmol) and anhydrous DMF (3 mL) were added to the round bottom flask. After stirring at room temperature for 10 minutes, compound 18 (100 mg, 0.25 mmol) was added, and the reaction was continued at room temperature for 4 hours, then the reaction was completed. The reaction solution was poured into water, extracted with ethyl acetate. The organic phases were combined and washed with water (3 x 10 mL) and saturated NaCl aqueous solution (2 x 10 mL) successively, dried over anhydrous $Na_2SO_4$. The solvent was evaporated to dryness under reduced pressure, and the resultant was separated by column chromatography to obtain a light yellow solid 28 (35 mg, yield 28.77%). $^1H$ NMR (400 MHz, DMSO-$d_6$) $\delta$ 9.23 (s, 1H), 8.26 (s, 1H), 8.17 (s, 1H), 7.70 (dd, *J* = 8.5, 5.1 Hz, 1H), 7.46 (s, 1H), 7.24 - 6.96 (m, 2H), 6.55 (br s, 1H), 4.19 - 4.04 (m, 1H), 3.83 (s, 3H), 3.44 (s, 2H), 3.21 - 3.16 (m, 1H), 2.62 (s, 6H), 1.82 - 1.66 (m, 4H), 1.36 - 1.07 (m, 4H). MS 478.2 (M+1).

**Example 29**

Preparation of 1-(4-(4-fluoro-2-methoxyphenyl)-5-methylpyridin-2-yl)-3-((1r,4r)-4-((2-methoxyethyl)amino)cyclohexyl) urea (compound 29)

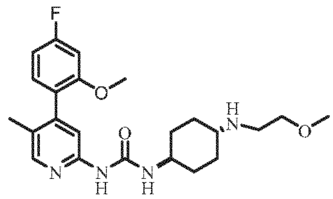

The synthesis method was the same as Example 3 except that 4-bromo-5-methylpyridin-2-amine was used instead of 4-bromo-pyridin-2-amine. $^1H$ NMR (400 MHz, DMSO-$d_6$) $\delta$ 9.16 (s, 1H), 8.08 (s, 1H), 7.88 (s, 1H), 7.71 (dd, *J* = 8.5, 5.0 Hz, 1H), 7.15 (dd, *J* = 8.0, 2.2 Hz, 1H), 7.00 (td, *J* = 8.2, 2.2 Hz, 1H), 6.54 (br s, 1H), 3.81 (s, 3H), 3.74 - 3.68 (m, 1H), 3.53 (t, *J* = 5.4 Hz, 2H), 3.27 (s, 3H), 3.26 - 3.19 (m, 1H), 2.76 (t, *J* = 5.4 Hz, 2H), 2.53 - 2.39 (m, 1H), 2.36 (s, 3H), 1.80 - 1.61 (m, 4H), 1.35 - 1.06 (m, 4H). MS 431.2 (M+1).

**Example 30**

Preparation of N-((1r,4r)-4-(3-(5-fluoro-4-(4-fluoro-2-methoxyphenyl)pyridin-2-yl)ureido) cyclohexyl)acetamide (compound 30)

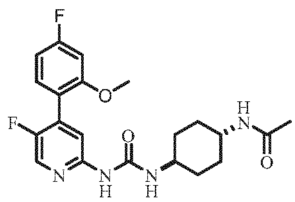

The synthesis method was the same as Example 4 except that 4-bromo-5-fluoropyridin-2-amine was used instead of 4-bromo-pyridin-2-amine. $^1H$ NMR (400 MHz, DMSO-$d_6$) δ 9.30 (s, 1H), 8.72 (d, $J$ = 8.0 Hz, 1H), 7.95 (d, $J$ = 4.9 Hz, 1H), 7.75 - 7.67 (m, 1H), 7.15 (dd, $J$ = 8.0, 2.2 Hz, 1H), 7.00 (td, $J$ = 8.2, 2.2 Hz, 1H), 6.56 (br s, 1H), 5.50 (br s, 1H), 4.21 - 4.05 (m, 1H), 3.81 (s, 3H), 3.36 - 3.18 (m, 1H), 1.99 (s, 3H), 1.81 - 1.69 (m, 2H), 1.55 - 1.43 (m, 2H), 1.37 - 1.08 (m, 4H). MS 419.1 (M+1).

**Example 31**

Preparation of N-((1r,4r)-4-(3-(5-methyl-4-(4-fluoro-2-methoxyphenyl)pyridin-2-yl) ureido)cyclohexyl)acetamide (compound 31)

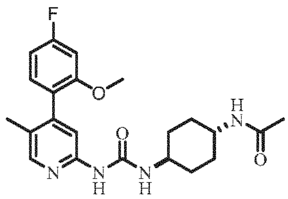

The synthesis method was the same as Example 4 except that 4-bromo-5-methylpyridin-2-amine was used instead of 4-bromo-pyridin-2-amine. $^1H$ NMR (400 MHz, DMSO-$d_6$) δ 9.18 (s, 1H), 8.05 (s, 1H), 7.85 (s, 1H), 7.68 (dd, $J$ = 8.5, 5.0 Hz, 1H), 7.12 - 6.99 (m, 2H), 6.51 (br s, 1H), 5.46 (s, 1H), 4.17 - 4.04 (m, 1H), 3.78 (s, 3H), 3.30 - 3.16 (m, 1H), 2.33 (s, 3H), 1.96 (s, 3H), 1.74 - 1.65 (m, 2H), 1.49 - 1.37 (m, 2H), 1.30 - 1.06 (m, 4H). MS 415.2 (M+1).

**Example 32**

Preparation of N-((1r,4r)-4-(3-(5-cyano-4-(4-fluoro-2-methoxyphenyl)pyridin-2-yl) ureido)cyclohexyl)acetamide (compound 32)

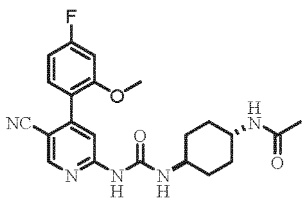

The synthesis method was the same as Example 4 except that 4-bromo-5-cyanopyridin-2-amine was used instead of 4-bromo-pyridin-2-amine. $^1H$ NMR (400 MHz, DMSO-$d_6$) δ 9.34 (s, 1H), 9.01 (s, 1H), 8.40 (s, 1H), 7.71 (dd, $J$ = 8.5, 5.0 Hz, 1H), 7.15 (dd, $J$ = 8.0, 2.2 Hz, 1H), 7.00 (td, $J$ = 8.2, 2.2 Hz, 1H), 6.56 (s, 1H), 5.50 (s, 1H), 4.21 - 4.01 (m, 1H), 3.81 (s, 3H), 3.41 - 3.12 (m, 1H), 1.99 (s, 3H), 1.83 - 1.62 (m, 2H), 1.57 - 1.37 (m, 2H), 1.37 - 1.08 (m, 4H). MS 426.2 (M+1).

**Example 33**

Preparation of 1-(4-(4-fluoro-2-methoxyphenyl)pyridin-2-yl)-3(6-fluoropyridin-3-yl)urea (compound 33)

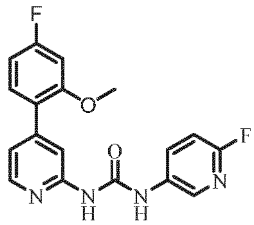

The synthesis method was the same as Example 1 except that 6-fluoropyridin-3-amine was used instead of N-Boc-trans-1,4-cyclohexanediamine (compound 1-c). $^1$H NMR (600 MHz, chloroform-*d*) δ 12.11 (s, 1H), 8.30 (s, 1H), 8.29 - 8.22 (m, 2H), 7.37 (brs, 1H), 7.30 (dd, *J* = 8.4, 6.6 Hz, 1H), 7.12 (dd, *J* = 5.4, 1.5 Hz, 1H), 6.98 - 6.88 (m, 2H), 6.84 - 6.70 (m, 2H), 3.85 (s, 3H). MS 357.1 (M+1).

**Example 34**

Preparation of 1-(4-(4-fluoro-2-methoxyphenyl)pyridin-2-yl)-3-(3-((2-methoxyethyl) amino)propyl)urea (compound 34)

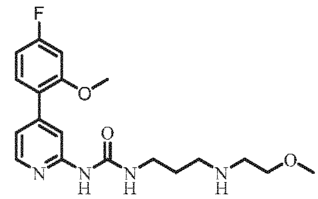

The synthesis method was the same as Example 3 except that N-Boc-1,3-propanediamine was used instead of N-Boc-trans-1,4-cyclohexanediamine. $^1$H NMR (400 MHz, DMSO-$d_6$) δ 9.21 (s, 1H), 8.23 (d, *J* = 4.9 Hz, 1H), 7.97 (d, *J* = 2.3 Hz, 1H), 7.71 (dd, *J* = 8.4, 5.0 Hz, 1H), 7.25 (dd, *J* = 4.8, 2.2 Hz, 1H), 7.19 - 7.07 (m, 2H), 7.05 - 6.93 (m, 1H), 3.81 (s, 3H), 3.61 - 3.47 (m, 5H), 3.27 (s, 3H), 2.82 - 2.69 (m, 2H), 2.56 - 2.44 (m, 2H), 1.99 (p, *J* = 6.0 Hz, 2H). MS 377.2 (M+1).

**Example 35**

Preparation of 1-((1r,3r)-3-aminocyclobutyl)-3-(4-(4-fluoro-2-methoxyphenyl)pyridin-2-yl)urea (compound 35)

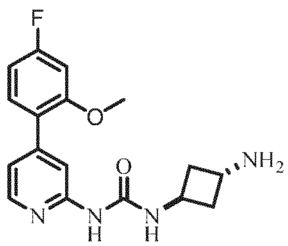

The synthesis method was the same as Example 2 except that N-Boc-trans-1,3-cyclobutanediamine was used instead of N-Boc-trans-1,4-cyclohexanediamine. $^1$H NMR (400 MHz, DMSO-$d_6$) δ 9.18 (s, 1H), 8.25 (d, *J* = 4.9 Hz, 1H), 7.99 (d, *J* = 2.3 Hz, 1H), 7.73 (dd, *J* = 8.4, 4.8 Hz, 1H), 7.27 (dd, *J* = 4.8, 2.2 Hz, 1H), 7.17 (dd, *J* = 8.0, 2.1 Hz, 1H), 7.02

(td, $J$ = 8.2, 2.2 Hz, 1H), 6.56 (d, $J$ = 10.3 Hz, 1H), 4.40 - 4.25 (m, 1H), 3.83 (s, 3H), 3.11 (ttd, $J$ = 9.2, 7.0, 2.3 Hz, 1H), 2.54 (dt, $J$ = 12.4, 9.3 Hz, 2H), 2.43 (br s, 2H), 2.37 - 2.25 (m, 2H). MS 331.1 (M+1).

**Example 36**

Preparation of 1-(4-(4-fluoro-2-methoxyphenyl)pyridin-2-yl)-3((1r,3r)-3-hydroxycyclobutyl)urea (compound 36)

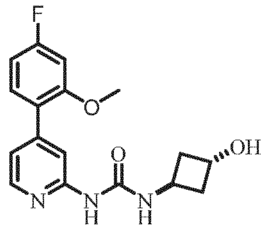

The synthesis method was the same as Example 1 except that (1r,3r)-3-aminocyclobutan-1-ol was used instead of N-Boc-trans-1,4-cyclohexanediamine. $^{1}$H NMR (400 MHz, DMSO-$d_6$) $\delta$ 9.21 (s, 1H), 8.23 (d, $J$ = 4.9 Hz, 1H), 7.97 (d, $J$ = 2.3 Hz, 1H), 7.71 (dd, $J$ = 8.4, 5.0 Hz, 1H), 7.25 (dd, $J$ = 4.8, 2.2 Hz, 1H), 7.15 (dd, $J$ = 8.0, 2.2 Hz, 1H), 7.00 (td, $J$ = 8.2, 2.2 Hz, 1H), 6.54 (d, $J$ = 10.3 Hz, 1H), 4.52 - 4.38 (m, 1H), 3.81 (s, 3H), 3.45 (d, $J$= 6.8 Hz, 1H), 3.40 - 3.28 (m, 1H), 2.67 - 2.54 (m, 2H), 2.42 - 2.28 (m, 2H). MS 332.1 (M+1).

**Example 37**

Preparation of 1-(azepan-4-yl)-3-(4-(4-fluoro-2-methoxyphenyl)pyridin-2-yl)urea (compound 37)

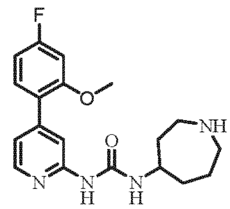

The synthesis method was the same as Example 2 except that tert-butyl 4-aminoazepane-1-carboxylate was used instead of N-Boc-trans-1,4-cyclohexanediamine. $^{1}$H NMR (400 MHz, methanol-$d_4$) $\delta$ 8.18 (dd, $J$ = 5.5, 0.8 Hz, 1H), 7.37 (dd, $J$ = 8.5, 6.6 Hz, 1H), 7.20 (s, 1H), 7.10 (dd, $J$ = 5.4, 1.5 Hz, 1H), 6.93 (dd, $J$ = 11.1, 2.5 Hz, 1H), 6.80 (td, $J$ = 8.3, 2.4 Hz, 1H), 4.04 - 3.96 (m, 1H), 3.85 (s, 3H), 3.44 - 3.33 (m, 2H), 3.29 - 3.19 (m, 2H), 2.35 - 2.13 (m, 2H), 2.10 - 1.97 (m, 2H), 1.96-1.82 (m, 2H). MS 359.2 (M+1).

**Example 38**

Preparation of 1-((1r,4r)-4-aminocyclohexyl)-3-(4-(4-fluoro-2-methoxyphenyl)pyridin-2-yl)thiourea (compound 38)

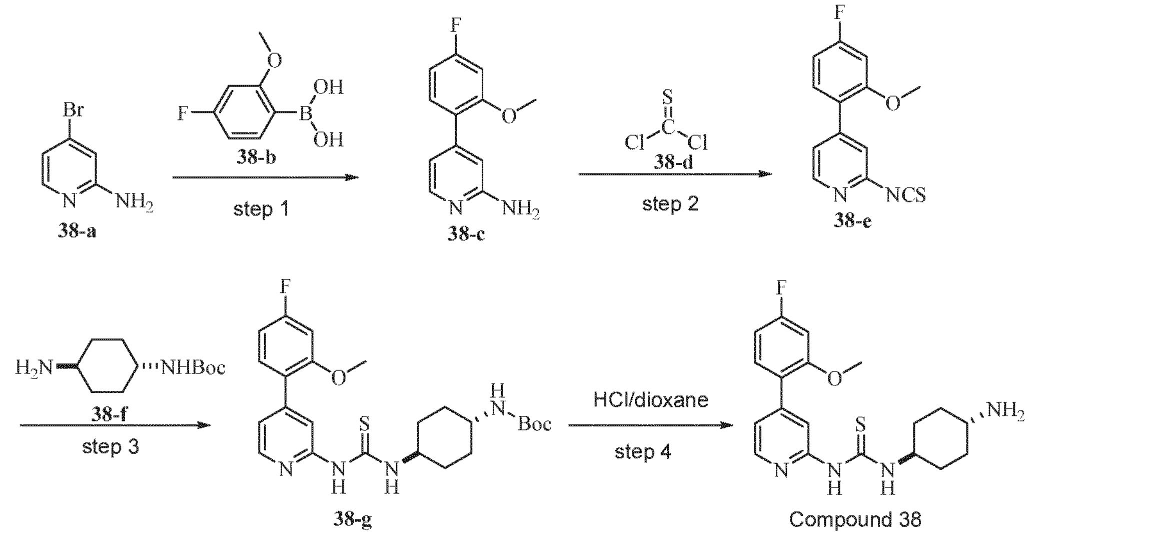


Step 1: compound 38-a (1.0 g, 5.78 mmol), 38-b (1.47g, 8.67 mmol), $Pd(OAc)_2$ (130 mg, 0.578 mml), X-phos (551 mg, 1.16 mmol), $K_2CO_3$ (2.0 g, 14.45 mmol) and $THF/H_2O$ (40/10 mL) were added into the round bottom flask, reacted at 85°C for 3 hours under argon protection. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain white solid 38-c (896 mg, yield 71.1%). $^1$H NMR (400 MHz, chloroform-*d*) δ 8.01 (d, *J* = 5.3 Hz, 1H), 7.52 - 7.44 (m, 1H), 7.29 - 7.26 (m, 1H), 6.81 - 6.78 (m, 1H), 6.76 - 6.70 (m, 2H), 4.82 (br s, 2H), 3.82 (s, 3H). MS 219.1 (M+1).

Step 2: compound 38-d (280 mg, 1.28 mmol) and anhydrous DCM (10 mL) were added into a round bottom flask, DCM solution (10 mL) of compound 38-c was added dropwise in an ice bath, and then anhydrous $Et_3N$ (0.36 mL, 2.57 mmol) was added dropwise, and the reaction was stopped after continuing to stir for 1 hour. The solvent was evaporated to dryness under reduced pressure to obtain dark brown solid 38-e, which could be directly used in the next reaction without further purification.

Step 3: intermediate 38-e obtained in step 2, compound 38-f (275 mg, 1.28 mmol), anhydrous $Et_3N$ (0.36 mL, 2.57 mmol) and anhydrous toluene (20 mL) were added into the round bottom flask, and heated to reflux overnight. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain white solid 38-g (89 mg, yield 14.6%). $^1$H NMR (400 MHz, chloroform-*d*) δ 11.66 (d, *J* = 7.9 Hz, 1H), 8.30 (s, 1H), 8.17 (d, *J* = 5.4 Hz, 1H), 7.30 - 7.24 (m, 1H), 7.07 (d, *J* = 4.9 Hz, 1H), 6.87 (s, 1H), 6.80 - 6.68 (m, 2H), 4.49 - 4.38 (m, 1H), 4.33 - 4.16 (m, 1H), 3.83 (s, 3H), 3.57 - 3.41 (m, 1H), 2.33 - 2.20 (m, 2H), 2.12 - 1.99 (m, 2H), 1.45 (s, 9H), 1.36 - 1.16 (m, 4H). MS 475.2 (M+1).

Step 4: compound 38-g (79 mg, 0.167 mmol) and dichloromethane (5 mL) were added to the round bottom flask, 4.0 M HCl dioxane solution (0.5 mL) was added dropwise thereto, and stirred at room temperature overnight. After the reaction was completed, the solvent was evaporated to dryness under reduced pressure, and then 1 M NaOH aqueous solution was added dropwise to adjust the pH to weak alkalinity. The solvent was evaporated to dryness under reduced pressure and the resultant was separated by column chromatography to obtain a white solid 38 (57.8 mg, 57.8%). $^1$H NMR (400 MHz, DMSO-$d_6$) δ 11.68 (d, *J* = 7.6 Hz, 1H), 10.56 (s, 1H), 8.19 (d, *J* = 5.4 Hz, 1H), 7.79 (s, 2H), 7.37 (dd, *J* = 8.6, 6.8 Hz, 1H), 7.33 (d, J = 1.5 Hz, 1H), 7.14 (dd, J = 5.5, 1.5 Hz, 1H), 7.09 (dd, *J* = 11.4, 2.5 Hz, 1H), 6.91 (td, *J* = 8.4, 2.5 Hz, 1H), 4.09 (s, 1H), 3.81 (s, 3H), 3.05 (s, 1H), 2.14 - 1.98 (m, 4H), 1.50 - 1.34 (m, 4H). MS 375.1 (M+1).

**Example 39**

Preparation of tert-butyl ((1r,4r)-4-(3-(6-(4-fluoro-2-methoxyphenyl)pyrimidin-4-yl) ureido)cyclohexyl)carbamate (Compound 39)

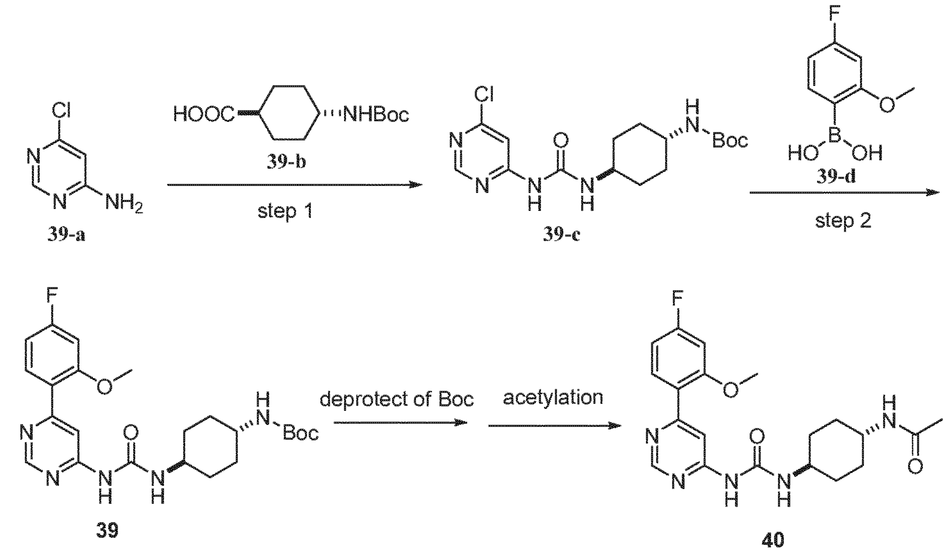


39-a 39-b 39-c 39-d 39 40

Step 1: compound 39-b (939 mg, 2.87 mmol), DPPA (1.03g, 3.74 mmol), anhydrous $Et_3N$ (1.2 mL, 8.62 mmol) and anhydrous toluene (5 mL) were added into the microwave tube. The reaction was conducted by microwave at 110°C for 5 minutes, compound 39-a (500 mg, 2.87 mmol) was added, and the reaction was continued by microwave at 110°C for 30 minutes. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain white solid 39-c (179 mg, yield 15.0%). $^1H$ NMR (400 MHz, DMSO-$d_6$) $\delta$ 8.98 (s, 1H), 8.80 (s, 1H), 8.70 (s, 1H), 7.47 (s, 1H), 4.52 - 4.37 (m, 1H), 3.90 (s, 3H), 3.78 - 3.67 (m, 1H), 3.56 - 3.40 (m, 1H), 2.19 - 1.95 (m, 4H), 1.45 (s, 9H), 1.33 - 1.17 (m, 4H). MS 414.1 (M+1).

Step 2: compound 39-c (100 mg, 0.27 mmol), 39-d (82 mg, 0.48 mmol), $Pd(OAc)_2$ (6 mg, 0.027 mml), X-phos (26 mg, 0.054 mmol), $K_2CO_3$ (83.40 mg, 0.60 mmol) and $THF/H_2O$ (8/2 mL) were added into the round bottom flask, reacted at 85°C for 3 hours under argon protection. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain white solid 39 (92.05 mg, yield 83.0%). $^1H$ NMR (400 MHz, chloroform-$d$) $\delta$ 9.04 (br s, 1H), 8.81 (s, 1H), 8.05 (dd, $J$ = 8.8, 6.9 Hz, 1H), 7.98 (s, 1H), 7.38 (s, 1H), 6.80 (td, $J$ = 8.2, 2.4 Hz, 1H), 6.73 (dd, $J$ = 10.8, 2.4 Hz, 1H), 4.43 (d, $J$ = 8.1 Hz, 1H), 3.90 (s, 3H), 3.71 (m, 1H), 3.49 (s, 1H), 2.19-2.01 (m, 4H), 1.45 (s, 9H), 1.33 - 1.14 (m, 4H). MS 460.2 (M+1).

**Example 40**

Preparation of N-((1r,4r)-4-(3-(6-(4-fluoro-2-methoxyphenyl)pyrimidin-4-yl) ureido) cyclohexyl)acetamide (compound 40)

Compound 39 (82 mg, 0.178 mmol) and DCM (5 mL) were added to the round-bottomed flask, 4.0 M HCl dioxane solution (0.5 mL) was added dropwise thereto, and stirred at room temperature overnight. After the reaction was completed, the solvent was evaporated to dryness under reduced pressure to obtain a white solid. $Et_3N$ (124 $\mu$L, 0.89 mmol) and anhydrous DCM (3 mL) were added to the above round-bottom flask, acetic anhydride (15 $\mu$L, 0.27 mmol) was added dropwise thereto, and the reaction was conducted at room temperature for 2 hours. After the reaction was completed, the solvent was evaporated to dryness under reduced pressure, and the resultant was seperated by column chromatography to obtain a white solid 40 (60 mg, yield 83.76%). $^1H$ NMR (400 MHz, DMSO-$d_6$) $\delta$ 9.24 (s, 1H), 8.34 (d, $J$ = 1.5 Hz, 1H), 7.86 (br s, 1H), 7.71 (dd, $J$ = 9.4, 5.0 Hz, 1H), 7.15 (dd, $J$ = 8.0, 2.2 Hz, 1H), 7.10 (d, $J$ = 1.3 Hz, 1H), 7.05 - 6.95 (m, 1H), 5.53 (s, 1H), 4.22 - 4.03 (m, 1H), 3.81 (s, 3H), 3.43 - 3.14 (m, 1H), 1.99 (s, 3H), 1.95 - 1.68 (m, 4H), 1.35 - 1.07 (m, 4H). MS 402.1 (M+1).

**Example 41**

Preparation of N-((1r,4r)-4-(3-(4-(4-fluoro-2-methoxyphenyl)pyrimidin-2-yl)ureido) cyclohexyl)acetamide (compound 41)

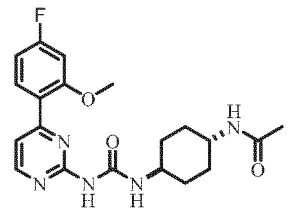

The synthesis method was the same as Example 40 except that 4-bromopyrimidin-2-amine was used instead of 6-chloropyrimidin-4-amine (39-a). $^1$H NMR (400 MHz, DMSO-$d_6$) δ 9.27 (s, 1H), 8.16 (d, *J* = 4.8 Hz, 1H), 7.75 - 7.67 (m, 2H), 7.15 (dd, *J* = 8.0, 2.2 Hz, 1H), 7.05 - 6.95 (m, 1H), 6.56 (s, 1H), 5.52 (s, 1H), 4.19 - 4.03 (m, 1H), 3.81 (s, 3H), 3.35 - 3.13 (m, 1H), 1.99 (s, 3H), 1.92 - 1.45 (m, 2H), 1.35 - 1.12 (m, 4H). MS 402.1 (M+1).

**Example 42**

Preparation of 1-((1r,4r)-4-aminocyclohexyl)-3-(5-chloro-4-(1,5-dimethyl-1H-pyrazol-4-yl)pyridin-2-yl)urea (compound 42)

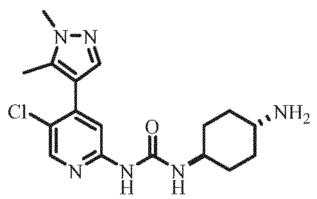

The synthesis method was the same as Example 2 except that 5-chloro-4-bromopyridin-2-amine was used instead of 4-bromopyridin-2-amine (1-a), and 1,5-dimethyl-1H-pyrazole-4-boronic acid pinacol ester was used instead of 4-fluoro-2-methoxyphenylboronic acid (1-e). $^1$H NMR (400 MHz, DMSO-$d_6$) δ 9.21 (s, 1H), 8.25 (s, 1H), 7.97 (s, 2H), 7.56-7.41 (m, 3H), 3.79 (s, 3H), 3.55 - 3.37 (m, 1H), 3.05 - 2.89 (m, 1H), 2.25 (s, 3H), 1.93-1.61 (m, 4H), 1.53 - 1.10 (m, 4H). MS 363.2 (M+1).

**Example 43**

Preparation of N-((1r,4r)-4-(3-(5-chloro-4-(1,5-dimethyl-1H-pyrazol-4-yl)pyridin-2-yl) ureido)cyclohexyl)acetamide (compound 43)

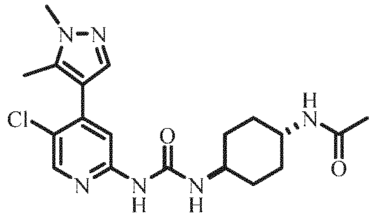

The synthesis method was the same as Example 4 except that 5-chloro-4-bromopyridin-2-amine was used instead of 4-bromopyridin-2-amine, and 1,5-dimethyl-1H-pyrazole-4-boronic acid pinacol ester was used instead of 4-fluoro-2-methoxyphenylboronic acid. $^1$H NMR (400 MHz, DMSO-$d_6$) δ 9.20 (s, 1H), 8.25 (s, 1H), 7.97 (s, 1H), 7.56-7.41

(m, 2H), 5.60 (s, 1H), 4.11 - 3.92 (m, 1H), 3.79 (s, 3H), 3.24 - 3.02 (m, 1H), 2.26 (s, 3H), 1.99 (s, 3H), 1.90-1.62 (m, 4H), 1.58 - 1.13 (m, 4H). MS 405.1 (M+1).

**Example 44**

Preparation of 1-((1r,4r)-4-aminocyclohexyl)-3-(5-chloro-4-(5,5-dimethyl-5,6-dihydro -4H-pyrrole[1,2-b]pyrazole-3-yl)pyridin-2-yl)urea (compound 44)

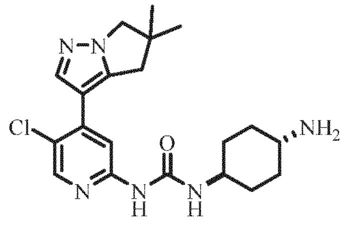

The synthesis method was the same as Example 15 except that 5-chloro-4-bromopyridin-2-amine was used instead of 4-bromo-pyridin-2-amine. $^1$H NMR (400 MHz, methanol-$d_4$) δ 8.20 (s, 1H), 8.01 (s, 1H), 7.37 (s, 1H), 3.97 (s, 2H), 3.72 - 3.56 (m, 1H), 3.35 (s, 1H), 2.95 (s, 2H), 2.23 - 1.86 (m, 4H), 1.47 - 1.37 (m, 4H), 1.34 (s, 6H). MS 403.2 (M+1).

**Example 45**

Preparation of 1-(5-chloro-4-(5,5-dimethyl-5,6-dihydro-4H-pyrrolo[1,2-b]pyrazol-3-yl)pyridin-2-yl)-3-((1r,4r)-4-hydroxycyclohexyl)urea (compound 45)

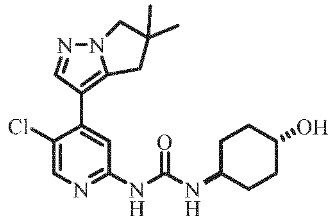

The synthesis method was the same as Example 15 except that 5-chloro-4-bromopyridin-2-amine was used instead of 4-bromopyridin-2-amine, and (1r,4r)-4-aminocyclohexan-1-ol was used instead of N-Boc-trans-1,4-cyclohexanediamine (15-k). $^1$H NMR (400 MHz, DMSO-$d_6$) δ 9.25 (s, 1H), 8.46 (s, 1H), 8.16 (s, 1H), 7.96 (s, 1H), 6.54 (s, 1H), 3.95 (s, 2H), 3.88 - 3.75 (m, 1H), 3.32 - 3.17 (m, 1H), 2.87 (s, 2H), 2.23 - 1.90 (m, 2H), 1.80 - 1.64 (m, 4H), 1.55 (d, $J$ = 8.6 Hz, 1H), 1.53 - 1.43 (m, 2H), 1.31 (s, 6H). MS 404.1 (M+1).

**Example 46**

Preparation of 1-(5-chloro-4-(5,5-dimethyl-5,6-dihydro-4H-pyrrolo[1,2-b]pyrazol-3-yl) pyridin-2-yl)-3-((1r,4r)-4-((2-methoxy)amino)cyclohexyl)urea (compound 46)

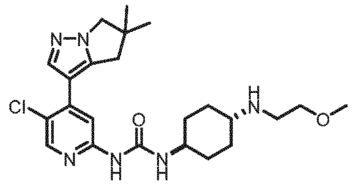

The synthesis method was the same as Example 3 except that compound 44 was used instead of compound 2. $^1$H NMR (400 MHz, chloroform-d) δ 8.77 (s, 1H), 8.40 (s, 1H), 8.14 (s, 1H), 7.96 (s, 1H), 6.92 (s, 1H), 3.95 (s, 2H), 3.79-3.67 (m, 1H), 3.57 (t, J = 5.1 Hz, 2H), 3.35 (s, 3H), 2.91 (d, J = 5.3 Hz, 2H), 2.89 (s, 2H), 2.63 (br s, 1H), 2.22 - 1.95 (m, 4H), 1.57 - 1.34 (m, 4H), 1.32 (s, 6H). MS 461.2 (M+1).

**Example 47**

Preparation of N-((1r,4r)-4-(3-(5-chloro-4-(5,5-dimethyl-5,6-dihydro-4H-pyrrolo[1,2-b] pyrazol-3-yl)pyridin-2-yl)ureido)cyclohexyl)acetamide (compound 47)

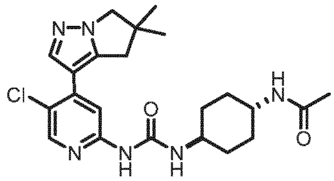

The synthesis method was the same as Example 4 except that compound 44 was used instead of compound 2. $^1$H NMR (400 MHz, chloroform-*d*) δ 8.71 (s, 1H), 8.62 (s, 1H), 8.16 (s, 1H), 7.92 (s, 1H), 6.97 (s, 1H), 5.66 (d, *J* = 8.1 Hz, 1H), 3.95 (s, 2H), 3.76 - 3.61 (m, 2H), 2.87 (s, 2H), 2.14 - 2.00 (m, 4H), 1.98 (s, 3H), 1.48 -1.35 (m, 2H), 1.32 (s, 6H), 1.30 - 1.22 (m, 2H). MS 445.1 (M+1).

**Example 48**

Preparation of N-((1r,4r)-4-(3-(5-chloro-4-(5,5-dimethyl-5,6-dihydro-4H-pyrrolo [1,2-b]pyrazol-3-yl)pyridin-2-yl)ureido)cyclohexyl)-2-(dimethylamino)acetamide (compound 48)

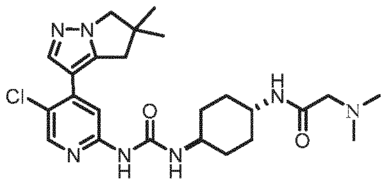

The synthesis method was the same as Example 28 except that compound 44 was used instead of compound 18. $^1$H NMR (600 MHz, acetone-$d_6$) δ 8.57 (s, 1H), 8.19 (s, 1H), 7.98 (s, 2H), 7.57 (s, 1H), 7.25 (d, *J* = 8.4 Hz, 1H), 3.95 (s, 2H), 3.77 - 3.70 (m, 1H), 3.68 - 3.60 (m, 1H), 2.97 (s, 2H), 2.91 (s, 2H), 2.29 (s, 6H), 1.95 - 1.90 (m, 2H), 1.49 - 1.35 (m, 6H), 1.34 (s, 6H). MS 488.3 (M+1).

**Example 49**

Preparation of N-((1r,4r)-4-(3-(5-chloro-4-(5,5-dimethyl-5,6-dihydro-4H-pyrrolo [1,2-b]pyrazol-3-yl)pyridin-2-yl)ureido)cyclohexyl)-2-(dimethylamino)cyclopropylcarboxamid e (compound 49)

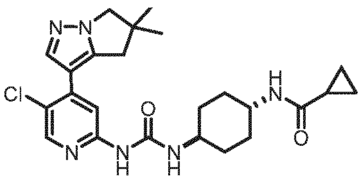

The synthesis method was the same as Example 28 except that compound 44 was used instead of compound

18, and cyclopropanecarboxylic acid was used instead of N,N-dimethylglycine. $^1$H NMR (400 MHz, DMSO-$d_6$) δ 9.24 (s, 1H), 8.46 (s, 1H), 8.16 (s, 1H), 7.96 (s, 1H), 7.44 (s, 1H), 6.54 (d, *J* = 11.9 Hz, 1H), 4.19 - 4.03 (m, 1H), 3.93 (s, 2H), 3.32 - 3.13 (m, 1H), 2.87 (s, 2H), 1.80 - 1.64 (m, 3H), 1.55 - 1.48 (m, 2H), 1.41 - 1.35 (m, 4H), 1.30 (s, 6H), 1.00 - 0.84 (m, 4H). MS 471.1 (M+1).

**Example 50**

Preparation of N-((1r,4r)-4-(3-(5-chloro-4-(5,5-dimethyl-5,6-dihydro-4H-pyrrolo[1,2-b] pyrazol-3-yl)pyridin-2-yl)ureido)cyclohexyl) oxetane-3-carboxamide (compound 50)

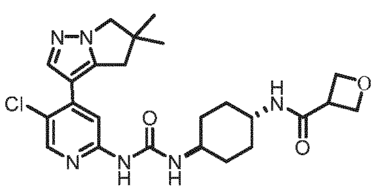

The synthesis method was the same as Example 28 except that compound 44 was used instead of compound 18, and oxetane-3-carboxylic acid was used instead of N,N-dimethylglycine. $^1$H NMR (400 MHz, DMSO-$d_6$) δ 9.14 (s, 1H), 8.50 (s, 1H), 8.24 (s, 1H), 7.97 (s, 1H), 7.44 (s, 1H), 6.95 (d, *J* = 11.1 Hz, 1H), 5.09 (dd, *J* = 12.4, 7.2 Hz, 2H), 4.84 (dd, *J* = 12.4, 7.0 Hz, 2H), 4.17 - 4.04 (m, 1H), 3.94 (s, 2H), 3.73 - 3.62 (m, 1H), 3.31 - 3.16 (m, 1H), 2.87 (s, 2H), 1.80 - 1.65 (m, 4H), 1.42 - 1.30 (m, 4H), 1.26 (s, 6H). MS 487.2 (M+1).

**Example 51**

Preparation of N-((1r,4r)-4-(3-(4-(5,5-dimethyl-5,6-dihydro-4H-pyrrolo[1,2-b]pyrazol-3-yl)-5-fluoropyridin-2-yl)ureido)cyclohexyl)acetamide (compound 51)

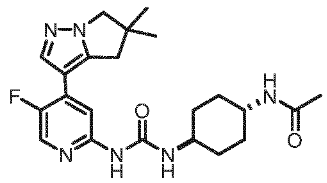

The synthesis method was the same as Example 16 except that the compound 5-fluoro-4-bromopyridin-2-amine was used instead of 4-bromo-pyridin-2-amine. $^1$H NMR (400 MHz, DMSO-$d_6$) δ 9.21 (s, 1H), 8.72 (d, *J*= 8.0 Hz, 1H), 8.16 (s, 1H), 7.98 (d, *J*= 4.9 Hz, 1H), 7.47 (s, 1H), 5.65 (d, *J* = 11.2 Hz, 1H), 4.18 - 4.06 (m, 1H), 3.97 (s, 2H), 3.33 - 3.15 (m, 1H), 2.83 (s, 2H), 1.99 (s, 3H), 1.81 - 1.65 (m, 4H), 1.55 - 1.36 (m, 4H), 1.31 (s, 6H). MS 429.1 (M+1).

**Example 52**

Preparation of N-((1r,4r)-4-(3-(4-(5,5-dimethyl-5,6-dihydro-4H-pyrrolo[1,2-b]pyrazol-3-yl)-5-methylpyridin-2-yl)ureido)cyclohexyl)acetamide (compound 52)

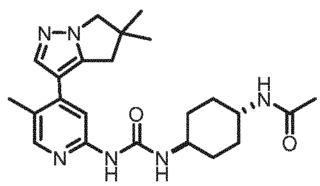

The synthesis method was the same as Example 16 except that the compound 5-methyl-4-bromopyridin-2-amine was used instead of 4-bromo-pyridin-2-amine. $^1H$ NMR (400 MHz, DMSO-$d_6$) δ 9.20 (s, 1H), 8.46 (s, 1H), 8.14 (s, 1H), 7.93 (s, 1H), 7.40 (s, 1H), 6.49 (br s, 1H), 3.93 (s, 2H), 3.32 - 3.13 (m, 2H), 2.87 (s, 2H), 2.51 (s, 1H), 1.80 - 1.60 (m, 4H), 1.47 - 1.29 (m, 4H), 1.25 (s, 6H). MS 425.2 (M+1).

**Example 53**

Preparation of N-((1r,4r)-4-(3-(4-(5,5-dimethyl-5,6-dihydro-4H-pyrrolo[1,2-b]pyrazol-3-yl)-5-cyclopropylpyridin-2-yl)ureido)cyclohexyl)acetamide (compound 53)

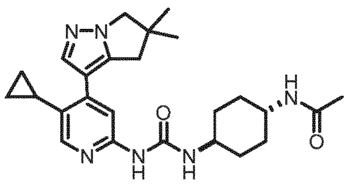

The synthesis method was the same as Example 16 except that the compound 5-cyclopropyl-4-bromopyridin-2-amine was used instead of 4-bromo-pyridin-2-amine. $^1H$ NMR (400 MHz, DMSO-$d_6$) δ 9.21 (s, 1H), 8.16 (s, 1H), 8.08 (s, 1H), 7.88 (s, 1H), 6.54 (d, *J* = 11.9 Hz, 1H), 5.49 (brs, 1H), 4.19 - 4.03 (m, 1H), 3.93 (s, 2H), 3.33 - 3.08 (m, 2H), 2.79 (s, 2H), 1.99 (s, 3H), 1.83 - 1.65 (m, 4H), 1.55 - 1.40 (m, 4H), 1.31 (s, 6H), 0.76 - 0.39 (m, 4H). MS 451.3 (M+1).

**Example 54**

Preparation of N-((1r,4r)-4-(3-(5-chloro-4-(5,5-dimethyl-5,6-dihydro-4H-pyrrolo[1,2-b] pyrazol-3-yl)pyridin-2-yl)ureido)cyclohexyl)acetamide-2,2,2-$d_3$ (compound 54)

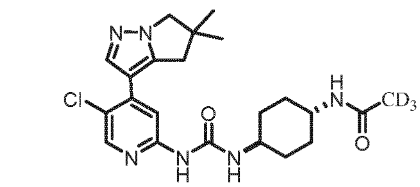

Compound 44 (60 mg, 0.149 mmol), DIPEA (62 μL, 0.372 mmol) and anhydrous DMF (1 mL) were added to the round bottomed flask, and acetyl chloride-$d_3$ (13 μL, 0.179 mmol) was added dropwise under ice bath, the resultant was reacted at room temperature for 2 hours. After the reaction was completed, methanol was added to quench the reaction, the solvent was evaporated to dryness under reduced pressure, and the resultant was seperated by column chromatography to obtain a white solid **54** (16 mg, 23.98%). $^1H$ NMR (400 MHz, DMSO-$d_6$) δ 9.23 (s, 1H), 8.46 (s, 1H), 8.16 (s, 1H), 7.93 (s, 1H), 7.43 (d, *J* = 11.4 Hz, 1H), 6.54 (d, *J* = 11.9 Hz, 1H), 4.18 - 4.04 (m, 1H), 3.95 (s, 2H), 3.31 - 3.16 (m, 1H), 2.82 (s, 2H), 1.80 - 1.62 (m, 4H), 1.44 - 1.27 (m, 2H), 1.23 (s, 6H). MS 448.2 (M+1).

**Example 55**

Preparation of 1-(5-chloro-4-(5,5-dimethyl-5,6-dihydro-4H-pyrrolo[1,2-b]pyrazol-3-yl) pyridin-2-yl)-3-((1r,4r)-4-((1-methoxypropan-2-yl)amino)cyclohexyl)urea (compound 55)

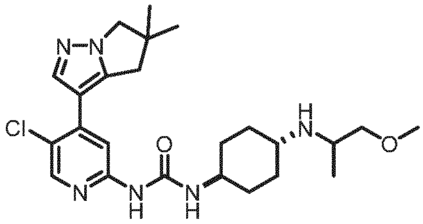

The synthesis method was the same as Example 17 except that 5-chloro-4-bromopyridin-2-amine was used instead of 4-bromopyridin-2-amine, and 2-bromo-1-methoxypropane was used instead of 1-bromo-2-methoxyethane. $^1$H NMR (400 MHz, DMSO-$d_6$) δ 9.21 (s, 1H), 8.46 (s, 1H), 8.15 (s, 1H), 7.93 (s, 1H), 6.54 (d, $J$ = 11.9 Hz, 1H), 4.70 (dd, $J$ = 10.3, 8.1 Hz, 1H), 3.94 (s, 2H), 3.47 (d, $J$ = 6.9 Hz, 2H), 3.27 (s, 3H), 3.26 - 3.18 (m, 1H), 2.99 - 2.87 (m, 1H), 2.83 (s, 2H), 2.40 - 2.34 (m, 1H), 1.83 - 1.70 (m, 2H), 1.65 - 1.44 (m, 6H), 1.21 (s, 6H), 0.98 (d, $J$ = 6.6 Hz, 3H). MS 475.1 (M+1).

**Example 56**

Preparation of 1-(5-chloro-4-(5,5-dimethyl-5,6-dihydro-4H-pyrrolo[1,2-b]pyrazol-3-yl) pyridin-2-yl)-3-((1r,4r)-4-((2-(dimethylamino)ethyl)amino)cyclohexyl)urea (compound 56)

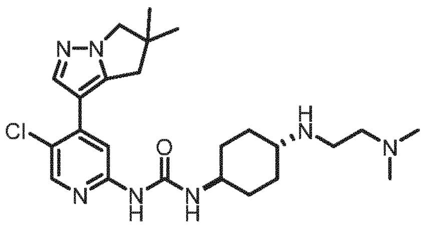

The synthesis method was the same as Example 17 except that 5-chloro-4-bromopyridin-2-amine was used instead of 4-bromopyridin-2-amine, and 2-bromo-N,N-dimethylethan-1-amine was used instead of 1-bromo-2-methoxyethane. $^1$H NMR (400 MHz, DMSO-$d_6$) δ 9.19 (s, 1H), 8.44 (s, 1H), 8.16 (s, 1H), 7.95 (s, 1H), 6.54 (br s, 1H), 3.95 (s, 2H), 3.32 - 3.15 (m, 1H), 2.87 (s, 2H), 2.67 (s, 6H), 2.55 - 2.46 (m, 2H), 2.45 - 2.36 (m, 4H), 1.79 - 1.67 (m, 4H), 1.54 - 1.42 (m, 4H), 1.25 (s, 6H). MS 474.1 (M+1).

**Example 57**

Preparation of 1-(5-chloro-4-(5,5-dimethyl-5,6-dihydro-4H-pyrrolo[1,2-b]pyrazol-3-yl) pyridin-2-yl)-3-((1r,4r)-4-((3,3,3-trifluoropropyl)amino)cyclohexyl)urea (compound 57)

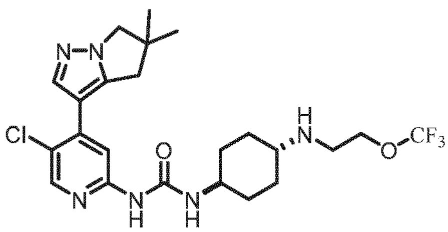

The synthesis method was the same as Example 17 except that 5-chloro-4-bromopyridin-2-amine was used instead of 4-bromopyridin-2-amine, and 1-bromo-2-(trifluoromethoxy)ethane was used instead of 1-bromo-2-methoxyethane. $^1$H NMR (400 MHz, DMSO-$d_6$) δ 9.20 (s, 1H), 8.46 (s, 1H), 8.16 (s, 1H), 7.93 (s, 1H), 6.64 (s, 1H), 3.70 (br s, 1H), 3.97 (s, 2H), 3.53 (t, $J$ = 4.8 Hz, 2H), 3.33 - 3.15 (m, 1H), 2.89 (s, 2H), 2.81 - 2.72 (m, 2H), 2.49 - 2.42 (m, 1H),

1.80 - 1.65 (m, 4H), 1.41 - 1.27 (m, 4H), 1.23 (s, 6H). MS 515.2 (M+1).

**Example 58**

Preparation of 1-(5-chloro-4-(5,5-dimethyl-5,6-dihydro-4H-pyrrolo[1,2-b]pyrazol-3-yl) pyridin-2-yl)-3-((1r,4r)-4-((2-(methoxy-*d3*)ethyl)amino)cyclohexyl)urea (compound 58)

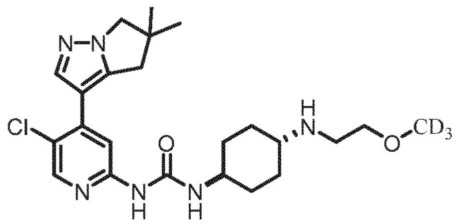

The synthesis method was the same as Example 17 except that 5-chloro-4-bromopyridin-2-amine was used instead of 4-bromopyridin-2-amine, and 1-bromo-2-(methoxy-*d3*)ethane was used instead of 1-bromo-2-methoxyethane. $^{1}H$ NMR (400 MHz, DMSO-$d_6$) $\delta$ 9.18 (s, 1H), 8.44 (s, 1H), 8.16 (s, 1H), 7.93 (s, 1H), 6.54 (d, $J$ = 11.9 Hz, 1H), 3.93 (s, 2H), 3.75 - 3.64 (m, 1H), 3.51 (t, $J$ = 5.4 Hz, 2H), 3.32 - 3.15 (m, 1H), 2.76 (dt, $J$ = 6.4, 5.5 Hz, 2H), 2.49 - 2.42 (m, 1H), 2.41 (s, 2H), 1.86 - 1.64 (m, 4H), 1.54 - 1.34 (m, 4H), 1.30 (s, 6H). MS 464.3 (M+1).

**Example 59**

Preparation of 1-(5-chloro-4-(5,5-dimethyl-5,6-dihydro-4H-pyrrolo[1,2-b]pyrazol-3-yl) pyridin-2-yl)-3-((1r,4r)-4-(ethylamino)cyclohexyl)urea (compound 59)

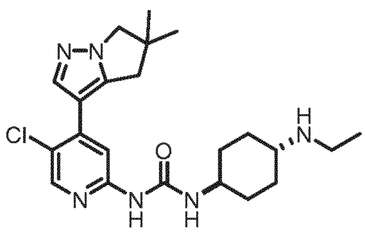

The synthesis method was the same as Example 17 except that 5-chloro-4-bromopyridin-2-amine was used instead of 4-bromopyridin-2-amine, and bromoethane was used instead of 1-bromo-2-methoxyethane. $^{1}H$ NMR (400 MHz, chloroform-*d*) $\delta$ 8.84 (s, 1H), 8.70 (s, 1H), 8.12 (s, 1H), 7.99 (s, 1H), 7.16 (s, 1H), 3.93 (s, 2H), 3.81 - 3.65 (m, 1H), 3.01 - 2.81 (m, 5H), 2.26 - 2.11 (m, 4H), 1.65 - 1.43 (m, 4H), 1.36 (t, $J$ = 7.2 Hz, 3H), 1.32 (s, 6H). MS 431.2 (M+1).

**Example 60**

Preparation of 1-(5-chloro-4-(5,5-dimethyl-5,6-dihydro-4H-pyrrolo[1,2-b]pyrazol-3-yl) pyridin-2-yl)-3-((1r,4r)-4-(isopropylamino)cyclohexyl)urea (compound 60)

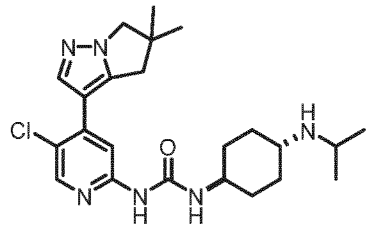

The synthesis method was the same as Example 17 except that 5-chloro-4-bromopyridin-2-amine was used instead of 4-bromopyridin-2-amine, and 2-bromopropane was used instead of 1-bromo-2-methoxyethane. $^1$H NMR (400 MHz, DMSO-$d_6$) $\delta$ 9.15 (s, 1H), 8.46 (s, 1H), 8.13 (s, 1H), 7.91 (s, 1H), 6.54 (s, 1H), 3.94 (s, 2H), 3.24 - 3.08 (m, 1H), 2.87 (s, 2H), 2.80 (m, 1H), 2.41 - 2.34 (m, 1H), 1.83 - 1.67 (m, 5H), 1.54 - 1.42 (m, 4H), 1.32 (d, $J$ = 4.9 Hz, 6H), 1.25 (s, 6H). MS 445.2 (M+1).

**Example 61**

Preparation of 1-(5-chloro-4-(5,5-dimethyl-5,6-dihydro-4H-pyrrolo[1,2-b]pyrazol-3-yl) pyridin-2-yl)-3-((1r,4r)-4-((2-fluoroethyl)amino)cyclohexyl)urea (compound 61)

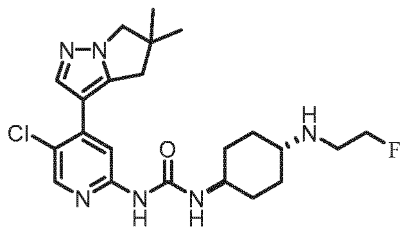

The synthesis method was the same as Example 17 except that 5-chloro-4-bromopyridin-2-amine was used instead of 4-bromopyridin-2-amine, and 1-bromo-2-fluoroethane was used instead of 1-bromo-2-methoxyethane. $^1$H NMR (400 MHz, DMSO-$d_6$) $\delta$ 9.20 (s, 1H), 8.44 (s, 1H), 8.18 (s, 1H), 7.90 (s, 1H), 6.86 (d, $J$ = 11.9 Hz, 1H), 4.68 - 4.46 (m, 2H), 3.97 (s, 2H), 3.30 - 3.19 (m, 1H), 2.84 (s, 2H), 2.82 - 2.64 (m, 2H), 2.50 - 2.42 (m, 1H), 1.81 - 1.62 (m, 5H), 1.52 - 1.34 (m, 4H), 1.20 (s, 6H). MS 449.2 (M+1).

**Example 62**

Preparation of 1-(5-chloro-4-(5,5-dimethyl-5,6-dihydro-4H-pyrrolo[1,2-b]pyrazol-3-yl) pyridin-2-yl)-3-((1r,4r)-4-((2-hydroxyethyl)amino)cyclohexyl)urea (compound 62)

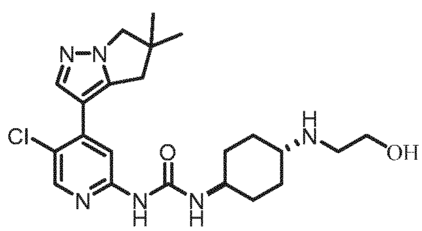

The synthesis method was the same as Example 17 except that 5-chloro-4-bromopyridin-2-amine was used instead of 4-bromopyridin-2-amine, and 2-bromoethanol was used instead of 1-bromo-2-methoxyethane. $^1$H NMR (400 MHz, DMSO-$d_6$) $\delta$ 9.18 (s, 1H), 8.46 (s, 1H), 8.16 (s, 1H), 7.87 (s, 1H), 6.56 (br s, 1H), 4.80 (t, $J$ = 6.4 Hz, 1H), 3.95 (s, 2H), 3.90 (td, $J$ = 7.5, 6.3 Hz, 2H), 3.76 - 3.64 (m, 1H), 3.32 (br s, 1H), 2.86 (s, 2H), 2.83 - 2.72 (m, 2H), 2.49 - 2.42 (m, 1H), 1.80 - 1.62 (m, 4H), 1.55 - 1.33 (m, 4H), 1.22 (s, 6H). MS 447.1 (M+1).

**Example 63**

Preparation of 1-(5-chloro-4-(5,5-dimethyl-5,6-dihydro-4H-pyrrolo[1,2-b]pyrazol-3-yl) pyridin-2-yl)-3-((1r,4r)-4-(isopropylamino)cyclohexyl)urea (compound 63)

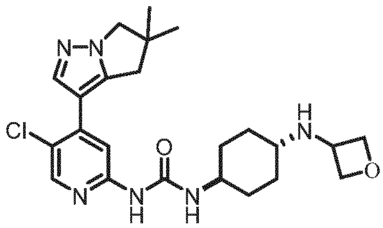

The synthesis method was the same as Example 17 except that 5-chloro-4-bromopyridin-2-amine was used instead of 4-bromopyridin-2-amine, and 3-bromooxetane was used instead of 1-bromo-2-methoxyethane. $^1$H NMR (400 MHz, DMSO-$d_6$) δ 9.23 (s, 1H), 8.47 (s, 1H), 8.16 (s, 1H), 7.90 (s, 1H), 6.59 (br s, 1H), 5.02 - 4.71 (m, 4H), 3.96 (s, 2H), 3.91 - 3.80 (m, 1H), 3.74 - 3.65 (m, 1H), 3.30 - 3.16 (m, 1H), 2.89 (s, 2H), 2.40 - 2.33 (m, 1H), 1.81 - 1.60 (m, 4H), 1.54 - 1.32 (m, 4H), 1.24 (s, 6H). MS 459.2 (M+1).

**Example 64**

Preparation of tert-butyl (1s,4s)-4-(3-(5-chloro-4-(5,5-dimethyl-5,6-dihydro-4H-pyrrolo [1,2-b]pyrazol-3-yl)pyridin-2-yl)ureido)cyclohexylcarbamate (compound 64)

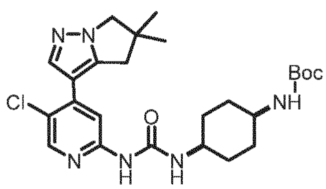

The synthesis method was the same as Example 15-m except that 5-chloro-4-bromopyridin-2-amine was used instead of 4-bromopyridin-2-amine and N-Boc-cis-1,4-cyclohexanediamine was used instead of N-Boc-trans-1,4-cyclohexanediamine. $^1$H NMR (400 MHz, chloroform-$d$) δ 9.34 (s, 1H), 8.76 (s, 1H), 8.18 (s, 1H), 8.02 (s, 1H), 6.81 (s, 1H), 4.55 (d, $J$ = 7.7 Hz, 1H), 4.02 (s, 1H), 3.97 (s, 2H), 3.58 (s, 1H), 2.91 (s, 2H), 1.82 - 1.67 (m, 6H), 1.62 - 1.49 (m, 2H), 1.46 (s, 9H), 1.34 (s, 6H). MS 503.3 (M+1).

**Example 65**

Preparation of 1-((1s,4s)-4-aminocyclohexyl)-3-(5-chloro-4-(5,5-dimethyl-5,6-dihydro-4H-pyrrolo[1,2-b]pyrazol-3-yl)pyridin-2-yl)urea (compound 65)

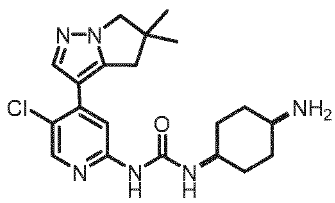

The synthesis method was the same as Example 15 except that 5-chloro-4-bromopyridin-2-amine was used

instead of 4-bromopyridin-2-amine and N-Boc-cis-1,4-cyclohexanediamine was used instead of N-Boc-trans-1,4-cyclohexanediamine. $^1$H NMR (400 MHz, chloroform-*d*) δ 9.44 (s, 1H), 9.38 (s, 1H), 8.18 (s, 1H), 8.02 (s, 1H), 6.94 (s, 1H), 4.12 - 4.01 (m, 1H), 3.95 (s, 2H), 2.91 (s, 2H), 2.86 - 2.75 (m, 1H), 1.96 - 1.51 (m, 8H), 1.33 (s, 6H). MS 403.1 (M+1).

**Example 66**

Preparation of 1-(5-chloro-4-(5,5-dimethyl-5,6-dihydro-4H-pyrrolo[1,2-b]pyrazol-3-yl) pyridin-2-yl)-3-((1s,4s)-4-((2-methoxyethyl)amino)cyclohexyl)urea (compound 66)

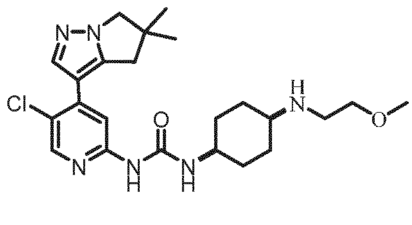

The synthesis method was the same as Example 17 except that 5-chloro-4-bromopyridin-2-amine was used instead of 4-bromopyridin-2-amine and N-Boc-cis-1,4-cyclohexanediamine was used instead of N-Boc-trans-1,4-cyclohexanediamine. $^1$H NMR (400 MHz, chloroform-*d*) δ 9.24 (s, 1H), 9.15 (s, 1H), 8.18 (s, 1H), 8.01 (s, 1H), 6.99 (s, 1H), 4.05 (s, 1H), 3.95 (s, 2H), 3.56 (t, *J* = 5.1 Hz, 2H), 3.36 (s, 3H), 2.92 (s, 2H), 2.90 - 2.85(m, 2H), 2.66 (s, 1H), 2.54 (s, 1H), 1.86 - 1.70 (m, 4H), 1.68 - 1.42 (m, 4H), 1.33 (s, 6H). MS 461.2 (M+1).

**Example 67**

Preparation of N-((1s,4s)-4-(3-(5-chloro-4-(5,5-dimethyl-5,6-dihydro-4H-pyrrolo[1,2-b] pyrazol-3-yl)pyridin-2-yl)ureido)cyclohexyl)acetamide (compound 67)

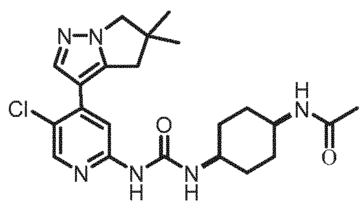

The synthesis method was the same as Example 16 except that 5-chloro-4-bromopyridin-2-amine was used instead of 4-bromopyridin-2-amine and N-Boc-cis-1,4-cyclohexanediamine was used instead of N-Boc-trans-1,4-cyclohexanediamine. $^1$H NMR (400 MHz, chloroform-*d*) δ 9.75 (s, 1H), 9.50 (s, 1H), 8.17 (s, 1H), 8.05 (s, 1H), 6.91 (s, 1H), 5.54 (d, *J* = 7.9 Hz, 1H), 4.07 (s, 1H), 3.96 (s, 2H), 3.92 - 3.81 (m, 1H), 2.92 (s, 2H), 2.00 (s, 3H), 1.90 - 1.83 (m, 4H), 1.78 - 1.69 (m, 2H), 1.54 - 1.37 (m, 2H), 1.33 (s, 6H). MS 445.2 (M+1).

**Example 68**

Preparation of 1-(5-chloro-4-(5,5-dimethyl-5,6-dihydro-4H-pyrrolo[1,2-b]pyrazol-3-yl) pyridin-2-yl)-3-(piperidin-4-yl)urea (compound 68)

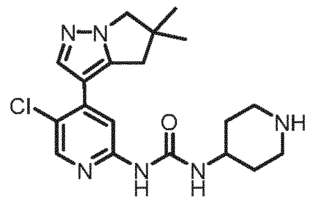

The synthesis method was the same as Example 15 except that 5-chloro-4-bromopyridin-2-amine was used instead of 4-bromopyridin-2-amine and N-Boc-piperidin-4-amine was used instead of N-Boc-trans-1,4-cyclohexanediamine. $^1$H NMR (400 MHz, DMSO-$d_6$) δ 9.14 (s, 1H), 8.50 (s, 1H), 8.24 (s, 1H), 7.97 (s, 1H), 7.73 (d, $J$ = 11.1 Hz, 2H), 3.94 (s, 2H), 3.84 - 3.76 (m, 1H), 3.22 (d, $J$ = 12.6 Hz, 2H), 2.97 (t, $J$ = 11.9 Hz, 2H), 2.87 (s, 2H), 2.10 - 1.92 (m, 2H), 1.58 - 1.46 (m, 2H), 1.26 (s, 6H). MS 389.2 (M+1).

**Example 69**

Preparation of 1-(azepan-4-yl)-3-(5-chloro-4-(5,5-dimethyl-5,6-dihydro-4H-pyrrolo[1,2-b] pyrazol-3-yl)pyridin-2-yl)urea (compound 69)

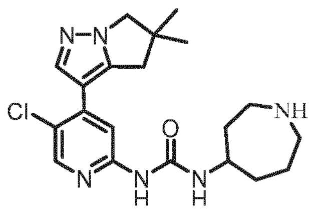

The synthesis method was the same as Example 15 except that 5-chloro-4-bromopyridin-2-amine was used instead of 4-bromopyridin-2-amine and N-Boc-azepan-4-amine was used instead of N-Boc-trans-1,4-cyclohexanediamine. $^1$H NMR (400 MHz, DMSO-$d_6$) δ 9.18 (s, 1H), 8.46 (s, 1H), 8.16 (s, 1H), 7.94 (s, 1H), 6.54 (d, $J$ = 10.8 Hz, 1H), 3.93 (s, 2H), 3.47 - 3.35 (m, 1H), 3.34 - 3.11 (m, 4H), 2.89 (s, 2H), 276 - 2.68 (m, 1H), 1.93 - 1.32 (m, 6H), 1.26 (s, 6H). MS 403.1 (M+1).

**Example 70**

Preparation of 1-(4-aminocycloheptyl)-3-(5-chloro-4-(5,5-dimethyl-5,6-dihydro-4H-pyrrolo[1,2-b]pyrazole-3-yl)pyridin-2-yl)urea (compound 70)

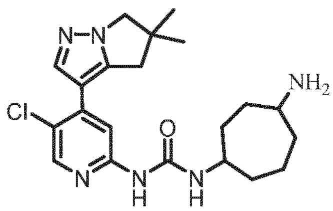

The synthesis method was the same as Example 15 except that 5-chloro-4-bromopyridin-2-amine was used instead of 4-bromopyridin-2-amine and N-Boc-1,4-cycloheptanediamine was used instead of N-Boc-trans-1,4-cyclohexanediamine. $^1$H NMR (400 MHz, DMSO-$d_6$) δ 9.23 (s, 1H), 8.46 (s, 1H), 8.14 (s, 1H), 7.95 (s, 1H), 6.54 (d, $J$ = 11.0 Hz, 1H), 3.95 (s, 2H), 3.47 - 3.34 (m, 1H), 2.87 (s, 2H), 2.50 - 2.42 (m, 1H), 1.87 - 1.68 (m, 2H), 1.62 - 1.56 (m, 4H), 1.41 - 1.26 (m, 6H), 1.20 (s, 6H). MS 417.2 (M+1).

**Example 71**

Preparation of tert-butyl (3-(3-(5-chloro-4-(5,5-dimethyl-5,6-dihydro-4H-pyrrolo[1,2-b] pyrazol-3-yl)pyridin-2-yl)ureido)propyl)carbamate (compound 71)

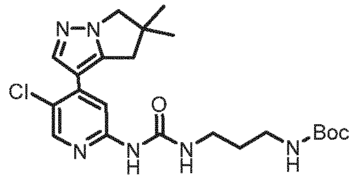

The synthesis method was the same as Example 15m except that 5-chloro-4-bromopyridin-2-amine was used instead of 4-bromopyridin-2-amine and N-Boc-1,3-propanediamine was used instead of N-Boc-trans-1,4-cyclohexanediamine. $^{1}$H NMR (400 MHz, chloroform-*d*) δ 9.04 (s, 1H), 8.35 (s, 1H), 8.18 (s, 1H), 8.00 (s, 1H), 6.82 (s, 1H), 5.08 (s, 1H), 3.96 (s, 2H), 3.45 (q, *J* = 6.2 Hz, 2H), 3.26 - 3.14 (m, 2H), 2.91 (s, 2H), 1.78 - 1.62 (m, 2H), 1.41 (s, 9H), 1.34 (s, 6H). MS 463.2 (M+1).

**Example 72**

Preparation of 1-(3-aminopropyl)-3-(5-chloro-4-(5,5-dimethyl-5,6-dihydro-4H-pyrrolo [1,2-b]pyrazol-3-yl)pyridin-2-yl)urea (compound 72)

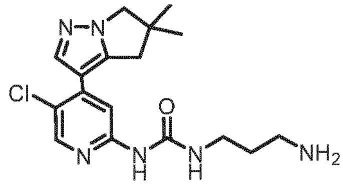

The synthesis method was the same as Example 15 except that 5-chloro-4-bromopyridin-2-amine was used instead of 4-bromopyridin-2-amine and N-Boc-1,3-propanediamine was used instead of N-Boc-trans-1,4-cyclohexanediamine. $^{1}$H NMR (400 MHz, methanol-$d_4$) δ 8.23 (s, 1H), 7.99 (s, 1H), 7.27 (s, 1H), 3.97 (s, 2H), 3.42 (t, *J*= 6.6 Hz, 3H), 3.06 - 2.86 (m, 4H), 1.90 (p, *J* = 6.9 Hz, 2H), 1.34 (s, 6H). MS 363.1 (M+1).

**Example 73**

Preparation of 1-((1S,3S)-3-aminocyclopentyl)-3-(_5-chloro-4-(5,5-dimethyl-5,6-dihydro-4H-pyrrole[1,2-b]pyrazole-3-yl)pyridin-2-yl)urea (compound 73)

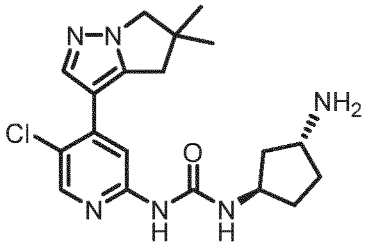

The synthesis method was the same as Example 15 except that 5-chloro-4-bromopyridin-2-amine was used instead of 4-bromopyridin-2-amine and tert-butyl (1S,3S)-3- aminocyclopentylcarbamate was used instead of N-Boc-trans-1,4-cyclohexanediamine. $^{1}$H NMR (400 MHz, methanol-$d_4$) δ 8.21 (s, 1H), 8.00 (s, 1H), 7.35 (s, 1H), 4.31 (p, *J* =

6.3 Hz, 1H), 3.97 (s, 2H), 3.68 (p, *J* = 6.7 Hz, 1H), 2.95 (s, 2H), 2.29 - 2.16 (m, 2H), 2.10 - 1.92 (m, 3H), 1.67 - 1.48 (m, 2H), 1.34 (s, 6H). MS 389.1 (M+1).

**Example 74**

Preparation of N-((1S,3S)-3-(3-(5-chloro-4-(5,5-dimethyl-5,6-dihydro-4H-pyrrolo[1,2-b] pyrazol-3-yl)pyridin-2-yl)ureido)cyclopentyl)acetamide (compound 74)

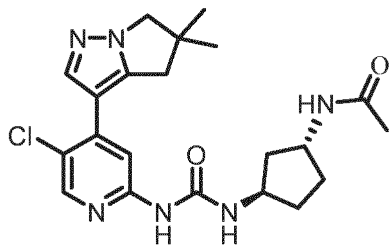

The synthesis method was the same as Example 16 except that 5-chloro-4-bromopyridin-2-amine was used instead of 4-bromopyridin-2-amine and tert-butyl (1S,3S)-3- aminocyclopentylcarbamate was used instead of N-Boc-trans-1,4-cyclohexanediamine. $^1$H NMR (400 MHz, chloroform-*d*) δ 9.02 (s, 1H), 8.71 (s, 1H), 8.16 (s, 1H), 7.95 (s, 1H), 7.07 (s, 1H), 6.08 (d, *J* = 7.4 Hz, 1H), 4.48 - 4.31 (m, 2H), 3.95 (s, 2H), 2.89 (s, 2H), 2.30 - 2.17 (m, 2H), 2.10 - 1.99 (m, 1H), 1.97 (s, 3H), 1.93 - 1.84 (m, 1H), 1.66 - 1.52 (m, 1H), 1.51 - 1.40 (m, 1H), 1.32 (s, 6H). MS 431.1 (M+1).

**Example 75**

Preparation of N-((1r,3r)-3-(3-(5-chloro-4-(5,5-dimethyl-5,6-dihydro-4H-pyrrolo[1,2-b] pyrazol-3-yl)pyridin-2-yl)ureido)cyclobutyl)acetamide (compound 75)

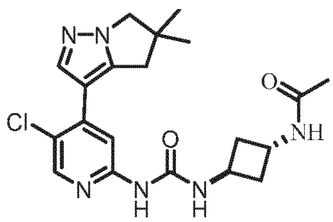

The synthesis method was the same as Example 16 except that 5-chloro-4-bromopyridin-2-amine was used instead of 4-bromopyridin-2-amine and N-Boc-trans-1,3-cyclobutanediamine was used instead of N-Boc-trans-1,4-cyclohexanediamine. $^1$H NMR (400 MHz, DMSO-$d_6$) δ 9.31 (s, 1H), 8.46 (s, 1H), 8.16 (s, 1H), 7.95 (s, 1H), 6.58 - 6.47 (m, 1H), 5.53 - 5.46 (m, 1H), 4.35 - 4.23 (m, 2H), 3.95 (s, 2H), 2.86 (s, 2H), 2.57 - 2.47 (m, 2H), 2.34 - 2.20 (m, 2H), 1.99 (s, 3H), 1.27 (s, 6H). MS 417.1 (M+1).

**Example 76**

Preparation of tert-butyl (3-(3-(5-chloro-4-(5,5-dimethyl-5,6-dihydro-4H-pyrrolo[1,2-b] pyrazol-3-yl)pyridin-2-yl)ureido)cyclohexyl)carbamate (compound 76)

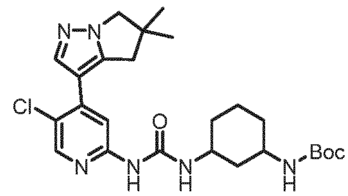

The synthesis method was the same as Example 15-m except that 5-chloro-4-bromopyridin-2-amine was used instead of 4-bromopyridin-2-amine and N-Boc-1,3-cyclohexanediamine was used instead of N-Boc-trans-1,4-cyclohexanediamine. $^1$H NMR (400 MHz, chloroform-*d*) δ 7.69 (br s, 1H), 7.53 - 7.35 (m, 2H), 7.23 - 7.12 (m, 1H), 6.98 (s, 2H), 2.93 - 2.84 (m, 1H), 2.45 - 2.21 (m, 2H), 1.85 - 1.72 (m, 3H), 1.50 - 1.30 (m, 4H), 1.26 (s, 9H), 1.14 -1.09 (m, 1H), 0.95 (s, 6H). MS 503.2 (M+1).

**Example 77**

Preparation of 1-(3-aminocyclohexyl)-3-(5-chloro-4-(5,5-dimethyl-5,6-dihydro-4H-pyrrolo[1,2-b]pyrazol-3-yl)pyridin-2-yl)urea (compound 77)

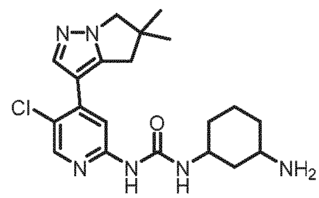

The synthesis method was the same as Example 15 except that 5-chloro-4-bromopyridin-2-amine was used instead of 4-bromopyridin-2-amine and N-Boc-1,3-cyclohexanediamine was used instead of N-Boc-trans-1,4-cyclohexanediamine. $^1$H NMR (400 MHz, DMSO-$d_6$) δ 9.31 (s, 1H), 8.46 (s, 1H), 8.16 (s, 1H), 7.91 (s, 1H), 6.54 (d, *J* = 10.5 Hz, 1H), 3.96 (s, 2H), 3.48 - 3.34 (m, 1H), 2.89 (s, 2H), 2.63 - 2.49 (m, 1H), 2.30 (br s, 2H), 2.06 - 1.92 (m, 1H), 1.83 - 1.49 (m, 5H), 1.46 - 1.34 (m, 2H), 1.23 (s, 6H). MS 403.1 (M+1).

**Example 78**

Preparation of 1-(5-chloro-4-(5,5-dimethyl-5,6-dihydro-4H-pyrrolo[1,2-b]pyrazol-3-yl) pyridin-2-yl)-3-(3-(ethylamino)cyclohexyl)urea (compound 78)

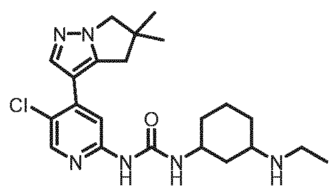

The synthesis method was the same as Example 17 except that 5-chloro-4-bromopyridin-2-amine was used instead of 4-bromopyridin-2-amine, N-Boc-1,3-cyclohexanediamine was used instead of N-Boc-trans-1,4-cyclohexanediamine, and bromoethane was used instead of 2-bromo-1-methoxyethane. $^1$H NMR (400 MHz, DMSO-$d_6$) δ 9.23 (s, 1H), 8.48 (s, 1H), 8.16 (s, 1H), 7.93 (s, 1H), 6.54 (br s, 1H), 3.94 (s, 2H), 3.49 - 3.32 (m, 1H), 3.29 - 3.16 (m, 1H), 3.07 - 2.91 (m, 1H), 2.85 (s, 2H), 2.50 - 2.42 (m, 1H), 1.94 - 1.81 (m, 1H), 1.74 - 1.49 (m, 6H), 1.43 - 1.30 (m, 2H), 1.29 (t, *J* = 5.7 Hz, 3H), 1.20 (s, 6H). MS 431.2 (M+1).

**Example 79**

Preparation of N-(3-(3-(5-chloro-4-(5,5-dimethyl-5,6-dihydro-4H-pyrrolo[1,2-b]pyrazol-3-yl)pyridin-2-yl)ureido)cyclohexyl)acetamide (compound 79)

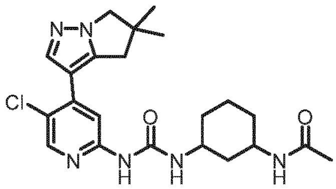

The synthesis method was the same as Example 16 except that 5-chloro-4-bromopyridin-2-amine was used instead of 4-bromopyridin-2-amine and N-Boc-1,3-cyclohexanediamine was used instead of N-Boc-trans-1,4-cyclohexanediamine. $^1$H NMR (400 MHz, chloroform-*d*) δ 8.98 (s, 1H), 8.36 (s, 1H), 8.13 (s, 1H), 7.99 (s, 1H), 7.08 (s, 1H), 5.90 (s, 1H), 3.95 (s, 2H), 3.91 - 3.70 (m, 2H), 2.89 (s, 2H), 2.33 (d, *J* = 11.5 Hz, 1H), 2.06 (d, *J* = 15.3 Hz, 1H), 1.87 - 1.76 (m, 4H), 1.58 - 1.40 (m, 2H), 1.33 (s, 6H). MS 445.2 (M+1).

**Example 80**

Preparation of 1-(5-chloro-4-(5,5-dimethyl-5,6-dihydro-4H-pyrrolo[1,2-b]pyrazol-3-yl) pyridin-2-yl)-3-(3-((2-methoxyethyl)amino)cyclohexyl)urea (compound 80)

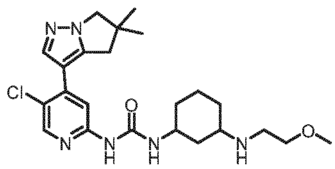

The synthesis method was the same as Example 17 except that 5-chloro-4-bromopyridin-2-amine was used instead of 4-bromopyridin-2-amine and N-Boc-1,3-cyclohexanediamine was used instead of N-Boc-trans-1,4-cyclohexanediamine. $^1$H NMR (400 MHz, DMSO-$d_6$) δ 9.24 (s, 1H), 8.44 (s, 1H), 8.16 (s, 1H), 7.94 (s, 1H), 6.54 (d, *J* = 10.3 Hz, 1H), 3.94 (s, 2H), 3.75 - 3.66 (m, 2H), 3.51 - 3.36 (m, 2H), 3.27 (s, 3H), 2.88 (s, 2H), 2.84 - 2.76 (m, 2H), 2.49 - 2.42 (m, 1H), 1.94 - 1.46 (m, 6H), 1.42 - 1.31 (m, 2H), 1.22 (s, 6H). MS 461.2 (M+1).

**Example 81**

Preparation of N-((1r,4r)-4-(3-(5-chloro-4-(4'H,6'H-spiro[cyclopropane-1,5'-pyrrolo[1,2-b] pyrazole]-3'-yl)pyridin-2-yl)ureido)cyclohexyl)acetamide (compound 81)

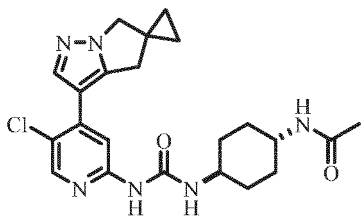

The synthesis method was the same as Example 16 except that 5-chloro-4-bromopyridin-2-amine was used instead of 4-bromopyridin-2-amine and 4',6'-dihydrospiro[cyclopropane-1,5'-pyrrolo[1,2-b]pyrazole]-3'-boronic acid pinacol ester was used instead of compound 15-i. $^1H$ NMR (400 MHz, DMSO-$d_6$) δ 9.23 (s, 1H), 8.46 (s, 1H), 8.17 (s, 1H), 7.92 (s, 1H), 6.94 (s, 1H), 5.64 (br s, 1H), 4.19 - 4.01 (m, 1H), 3.97 (s, 2H), 3.31 - 3.16 (m, 1H), 2.84 (s, 2H), 2.03 (s, 3H), 1.85 - 1.56 (m, 4H), 1.46 - 1.26 (m, 4H), 0.44 - 0.12 (m, 4H). MS 443.1 (M+1).

**Example 82**

Preparation of N-((1r,4r)-4-(3-(5-chloro-4-(6,7-dihydro-4H-pyrazolo[5,1-c][1,4]oxazine-3-yl)pyridin-2-yl)ureido)cyclohexyl)acetamide (compound 82)

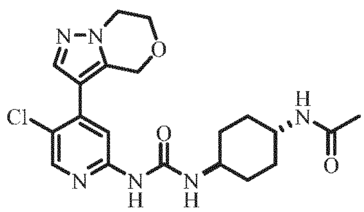

The synthesis method was the same as Example 16 except that 5-chloro-4-bromopyridin-2-amine was used instead of 4-bromopyridin-2-amine and 6,7-dihydro-4H-pyrazolo[5,1-c][1,4]oxazine-3-boronic acid pinacol ester was used instead of compound 15-i. $^1H$ NMR (400 MHz, DMSO-$d_6$) δ 9.20 (s, 1H), 8.45 (s, 1H), 8.14 (s, 1H), 8.12 (s, 1H), 6.84 (s, 1H), 5.69 (s, 1H), 4.63 (s, 2H), 4.19 - 4.00 (m, 1H), 3.93 - 3.78 (m, 4H), 3.34 - 3.11 (m, 1H), 1.98 (s, 3H), 1.82 - 1.61 (m, 4H), 1.36 - 1.16 (m, 4H). MS 433.2 (M+1).

**Example 83**

Preparation of N-((1r,4r)-4-(3-(5-chloro-4-(5-methyl-4,5,6,7-tetrahydropyrazolo[1,5-a] pyrazin-3-yl)pyridin-2-yl)ureido)cyclohexyl)acetamide (compound 83)

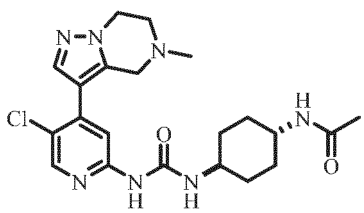

The synthesis method was the same as Example 16 except that 5-chloro-4-bromopyridin-2-amine was used instead of 4-bromopyridin-2-amine and 5-methyl-4,5,6,7-tetrahydropyrazolo[1,5-a]pyrazine-3-boronic acid pinacol ester was used instead of compound 15-i. $^1H$ NMR (400 MHz, DMSO-$d_6$) δ 9.21 (s, 1H), 8.44 (s, 1H), 8.13 (s, 1H), 8.10 (s, 1H), 6.54 (d, $J$ = 11.7 Hz, 1H), 5.47 (d, $J$ = 11.2 Hz, 1H), 4.20 - 4.04 (m, 1H), 4.02 - 3.91 (m, 2H), 3.80 (s, 2H), 3.34 - 3.15 (m, 1H), 3.08 - 2.96 (m, 2H), 2.45 (s, 3H), 1.98 (s, 3H), 1.84 - 1.62 (m, 4H), 1.47 - 1.24 (m, 4H). MS 446.2 (M+1).

**Example 84**

Preparation of N-((1r,4r)-4-(3-(5-chloro-4-(5,6-dihydro-4H-pyrrolo[1,2-b]pyrazol-3-yl) pyridin-2-yl)ureido)cyclohexyl)acetamide (compound 84)

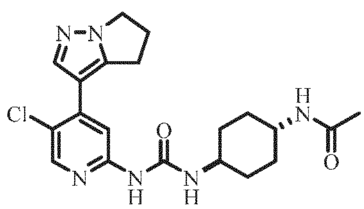

The synthesis method was the same as Example 16 except that 5-chloro-4-bromopyridin-2-amine was used instead of 4-bromo-pyridin-2-amine, and ethyl bromopropionate was used instead of compound 15-b. $^1$H NMR (400 MHz, DMSO-$d_6$) δ 9.17 (s, 1H), 8.46 (s, 1H), 8.16 (s, 2H), 6.57 (s, 1H), 5.49 (s, 1H), 4.21 - 4.02 (m, 1H), 3.71 (t, $J$ = 7.0 Hz, 2H), 3.36 - 3.16 (m, 1H), 2.95 - 2.81 (t, $J$ = 7.1 Hz, 2H), 2.68 - 2.50 (m, 2H), 1.99 (s, 3H), 1.80 - 1.63 (m, 4H), 1.57 - 1.40 (m, 4H). MS 417.1 (M+1).

**Example 85**

Preparation of N-((1r,4r)-4-(3-(5-chloro-4-(1-isopropyl-2-methyl-1H-imidazol-5-yl) pyridin-2-yl)ureido)cyclohexyl)acetamide (compound 85)

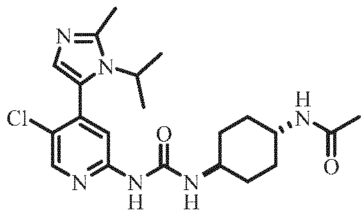

The synthesis method was the same as Example 16 except that 5-chloro-4-bromopyridin-2-amine was used instead of 4-bromo-pyridin-2-amine, and 1-isopropyl-2-methyl-1H-imidazol-5-boronic acid pinacol ester was used instead of compound 15-i. $^1$H NMR (400 MHz, DMSO-$d_6$) δ 9.16 (s, 1H), 8.46 (s, 1H), 8.15 (s, 1H), 6.94 (br s, 1H), 6.75 (s, 1H), 5.52 (s, 1H), 4.85 (hept, $J$ = 4.2 Hz, 1H), 4.20 - 3.98 (m, 1H), 3.34 - 3.13 (m, 1H), 2.52 (s, 3H), 1.99 (s, 3H), 1.80 - 1.66 (m, 4H), 1.57 (d, $J$ = 4.4 Hz, 6H), 1.53 - 1.37 (m, 4H). MS 433.2 (M+1).

**Example 86**

Preparation of N-((1r,4r)-4-(3-(5-chloro-4-(6,6-dimethyl-6,7-dihydro-5H-pyrrolo [1,2-a]imidazol-3-yl)pyridin-2-yl)ureido)cyclohexyl)acetamide (compound 86)

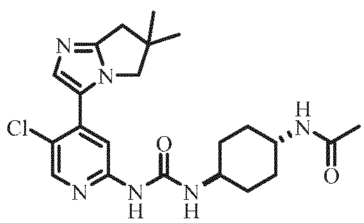

The synthesis method was the same as Example 16 except that 5-chloro-4-bromopyridin-2-amine was used instead of 4-bromo-pyridin-2-amine, and 6,6-dimethyl-6,7-dihydro-5H-pyrrolo[1,2-a]imidazol-3-boronic acid pinacol ester was used instead of compound 15-i. $^1$H NMR (400 MHz, DMSO-$d_6$) δ 9.21 (s, 1H), 8.46 (s, 1H), 8.16 (s, 1H), 6.65 (s, 1H), 6.56 (br s, 1H), 5.49 (br s, 1H), 4.20 - 4.04 (m, 1H), 3.96 (s, 2H), 3.37 - 3.12 (m, 1H), 2.87 (s, 2H), 1.99 (s, 3H), 1.80 - 1.67 (m, 4H), 1.55 - 1.42 (m, 4H), 1.30 (s, 6H). MS 445.1 (M+1).

**Example 87**

Preparation of N-((1r,4r)-4-(3-(5-chloro-4-(4-cyclopropyl-1H-imidazol-1-yl)pyridin-2-yl) ureido)cyclohexyl)acetamide (compound 87)

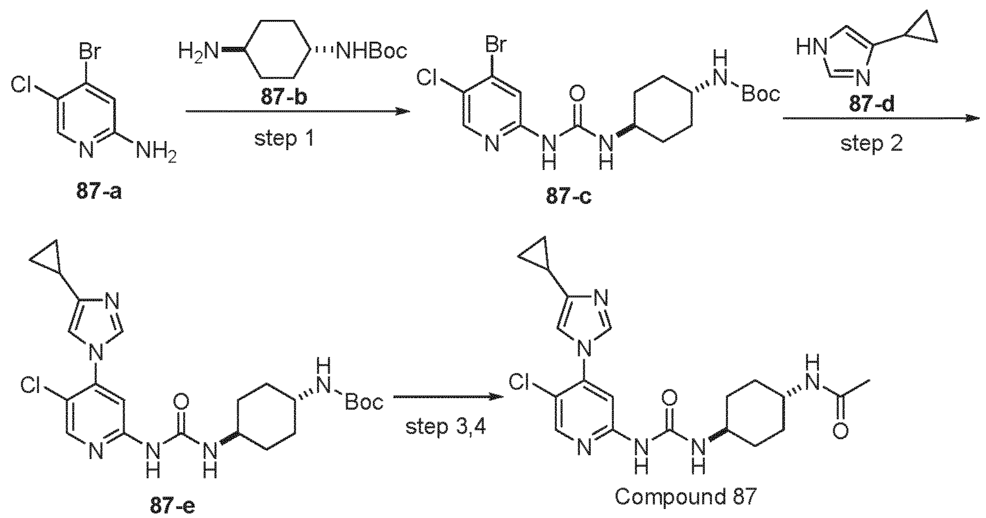


87-a 87-c 87-e Compound 87

Step 1: triphosgene (973 mg, 3.28 mmol) and anhydrous DCM (50 mL) were added to a round-bottom three-neck flask, and the DCM solution (30 mL) of compound **87-a** (2.0 g, 9.64 mmol) was added dropwise in an ice bath, then anhydrous $Et_3N$ (5.36 mL, 28.90 mmol) was added dropwise, and the reaction was stopped after continuing to stir for 2 hours. The solvent was evaporated to dryness under reduced pressure to obtain a brown solid, which was directly used in the next reaction without further purification. The above obtained intermediate, **87-b** (2.07 g, 9.64 mmol), $Et_3N$ (4.02 mL, 28.92 mmol) and anhydrous toluene (80 mL) were added to the round-bottom flask, and heated to reflux to react overnight. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain light yellow solid 87-c (2.7 g, yield 62.55%). $^1H$ NMR (400 MHz, DMSO-$d_6$) $\delta$ 9.22 (s, 1H), 8.34 (s, 1H), 8.09 (s, 1H), 7.08 (d, $J$ = 7.6 Hz, 1H), 6.74 (d, $J$ = 8.0 Hz, 1H), 3.37 (d, $J$ = 3.8 Hz, 1H), 3.23 - 3.09 (m, 1H), 1.93 - 1.70 (m, 4H), 1.37 (s, 9H), 1.28 - 1.13 (m, 4H). MS 447.1 (M+1).

Step 2: intermediate 87-c (400 mg, 0.893 mmol), 4-cyclopropyl-1H-imidazole (126 mg, 1.16 mmol), $Cs_2CO_3$ (437 mg, 1.34 mmol), CuO (7 mg, 0.089 mmol), 4,7-dimethoxy-1,10-phenanthroline (43 mg, 0.179 mmol), PEG-3350 (200 mg) and butyl cyanide (10 mL) were added into the round bottom flask, the reaction was conducted at 120°C for 16 hours. After the reaction was completed, it was cooled to room temperature, the reaction solution was poured into water, and extracted with ethyl acetate. The aqueous phase was repeatedly extracted with ethyl acetate for 2-3 times. The organic phases were combined and washed 3 times with saturated NaCl aqueous solution. After drying with anhydrous $Na_2SO_4$, the solvent was evaporated to dryness under reduced pressure, and the resultant was purified by column chromatography to obtain yellow solid 87-e (110 mg, yield 25.92%). $^1H$ NMR (400 MHz, DMSO-$d_6$) $\delta$ 9.20 (s, 1H), 8.26 (s, 1H), 8.11 (s, 1H), 7.80 (s, 1H), 7.16 (d, $J$ = 1.9 Hz, 1H), 6.59 (br s, 1H), 5.45 - 5.32 (m, 1H), 4.23 - 4.04 (m, 1H), 3.30 - 3.11 (m, 1H), 2.35 - 2.20 (m, 1H), 1.89 - 1.78 (m, 2H), 1.69 - 1.49 (m, 2H), 1.46 (s, 9H), 1.34 - 1.11 (m, 6H), 1.09 - 0.95 (m, 2H). MS 475.2 (M+1).

Steps 3-4: intermediate **87-e** (100 mg, 0.21 mmol) and DCM (5 mL) were added to the round-bottomed flask, 4.0 M dioxane solution of hydrochloride (0.5 mL) was added dropwise, and reacted at room temperature overnight. After the reaction was completed, the solvent was evaporated to dryness under reduced pressure to obtain a yellow solid. DCM (5 mL) and $Et_3N$ (146 mL, 1.05 mmol) were added to the round bottomed flask to redissolve the above intermediate, acetic anhydride (18 $\mu$L, 0.32 mmol) was added dropwise in an ice bath, and reacted at room temperature for 2 hours. After the reaction was completed, the solvent was evaporated to dryness under reduced pressure, and the resultant was seperated and purified by column chromatography to obtain a compound 87 (45 mg, two-step yield 51.27%). $^1H$ NMR (400 MHz, DMSO-$d_6$) $\delta$ 9.23 (s, 1H), 8.27 (s, 1H), 8.10 (s, 1H), 7.80 (s, 1H), 7.16 (d, $J$ = 2.1 Hz, 1H), 6.54 (s, 1H), 5.49 (s, 1H), 4.21 - 4.02 (m, 1H), 3.32 - 3.14 (m, 1H), 2.19 - 2.10 (m, 1H), 1.99 (s, 3H), 1.78 - 1.68 (m, 2H), 1.54 - 1.41

(m, 2H), 1.34 - 1.11 (m, 6H), 1.09 - 0.95 (m, 2H). MS 417.2 (M+1).

**Example 88**

Preparation of 1-(5-chloro-4-(5,5-dimethyl-5,6-dihydro-4H-pyrrolo[1,2-b]pyrazol-3-yl) pyridin-2-yl)-3-((1r,4r)-4-(methylamino)cyclohexyl)urea (compound 88)

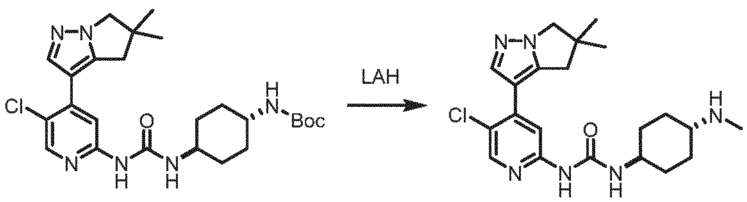


88-a 88

Compound 88-a (60 mg, 0.12 mmol) and anhydrous THF (5 mL) were added to the round bottomed flask, $LiAlH_4$ (14 mg, 0.26 mmol) was added thereto under ice bath, the resultant was stirred at room temperature for 10 minutes, and then warmed to 75°C to react for 4 hours. After the reaction was completed, it was cooled to room temperature, potassium sodium tartrate aqueous solution was added dropwise in an ice bath, the resultant was stirred vigorously at room temperature for 10 minutes, and a large amount of white solid was produced, filtered under reduced pressure, and the filter cake was washed with ethyl acetate. The filtrate was collected and the solvent was evaporated to dryness under reduced pressure, and the resultant was separated and purified by column chromatography to obtain light yellow solid 88 (20 mg, yield 40.22%). $^1$H NMR (400 MHz, chloroform-*d*) δ 8.56 (br s, 1H), 8.14 (s, 1H), 7.99 (s, 1H), 7.07 (br s, 1H), 5.46 - 5.38 (m, 1H), 3.94 (s, 2H), 3.82 - 3.67 (m, 1H), 2.93 (s, 2H), 2.87 - 2.77 (m, 2H), 2.64 (s, 3H), 2.24 - 2.19 (m, 2H), 1.76 - 1.68 (m, 2H), 1.48 - 1.38 (m, 4H), 1.32 (s, 6H). MS 417.2 (M+1).

**Example 89**

Preparation of 1-(5-chloro-4-(5,5-dimethyl-5,6-dihydro-4H-pyrrolo[1,2-b]pyrazol-3-yl) pyridin-2-yl)-3-((1r,4r)-4-(dimethylamino)cyclohexyl)urea (compound 89)

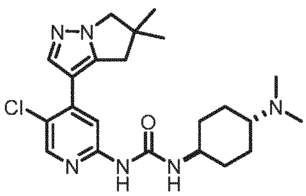

The synthesis method was the same as Example 15 except that 5-chloro-4-bromopyridin-2-amine was used instead of 4-bromopyridin-2-amine and N,N-dimethyl-trans-1,4-cyclohexanediamine was used instead of N-Boc-trans-1,4-cyclohexanediamine. $^1$H NMR (400 MHz, chloroform-*d*) δ 8.79 (s, 1H), 8.47 (s, 1H), 8.16 (s, 1H), 7.93 (s, 1H), 7.02 (br s, 1H), 3.95 (s, 2H), 3.72 - 3.64 (m, 1H), 3.25 - 3.19 (m, 1H), 2.91 (s, 2H), 2.52 (s, 6H), 2.24 - 2.01 (m, 4H), 1.60 - 1.45 (m, 4H), 1.32 (s, 6H). MS 431.2 (M+1).

**Example 90 - 91**

Preparation of 1-(5-chloro-4-(5,5-dimethyl-5,6-dihydro-4H-pyrrolo[1,2-b]pyrazol-3-yl) pyridin-2-yl)-3-((1r,4r)-4-((2-methoxyethyl)(methyl)amino)cyclohexyl)urea (compound 90) and (1r,4r)-4-(3-(5-chloro-4-(5,5-dimethyl-5,6-dihydro-4H-pyrrolo[1,2-b]pyrazol-3-yl)pyridin-2-yl)ureido)-N-(2-methoxyethyl)-N,N-dimethylcyclohexyl-1-ammonium salt (compound 91)

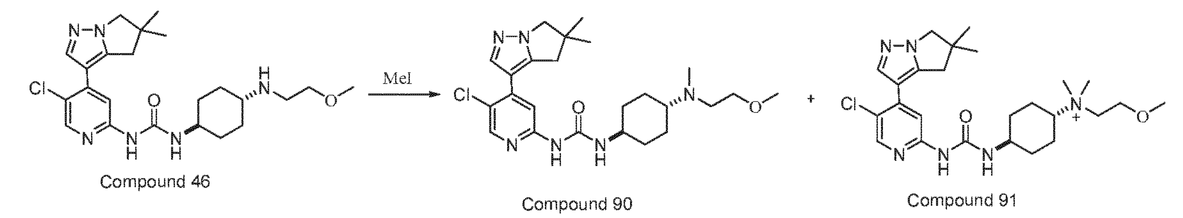


Compound 46 Compound 90 Compound 91

Compound 46 (100 mg, 0.217 mmol), DIPEA (90 μL, 0.542 mmol) and anhydrous DMF (5 mL) were added to the double-neck round bottomed flask, and methyl iodide (14 μL, 0.217 mmol) was added dropwise under ice bath, the resultant was continually stirred for 2 hours. The methyl iodide (14 μL, 0.217 mmol) was continually added dropwise, and the resultant was warmed to room temperature to react for 2 hours. NaOH aqueous solution (0.2 mL) was added to quench the reaction, the solvent was evaporated to dryness under reduced pressure and then the resultant was separated and purified by column chromatography to obtain light yellow solid 90 (18 mg, yield 17.47%) and yellow solid 91 (8 mg, yield 7.53%). Compound **90:** $^1$H NMR (400 MHz, chloroform-*d*) δ 8.75 (br s, 1H), 8.48 (s, 1H), 8.14 (s, 1H), 7.93 (s, 1H), 6.98 (s, 1H), 3.94 (s, 2H), 3.80 - 3.75 (m, 2H), 3.72 - 3.64 (m, 1H), 3.38 (s, 3H), 3.22 - 3.15 (m, 1H), 3.10 (t, *J* = 5.4 Hz, 2H), 2.90 (s, 2H), 2.65 (s, 3H), 2.26 - 2.12 (m, 4H), 1.59 - 1.51 (m, 4H), 1.32 (s, 6H). MS 475.2 (M+1). Compound **91:** $^1$H NMR (400 MHz, chloroform-*d*) δ 8.97 (br s, 1H), 8.14 (s, 1H), 7.96 (s, 1H), 7.23 (br s, 1H), 4.13 - 4.01 (m, 1H), 3.92 (s, 2H), 3.90 - 3.85 (m, 2H), 3.84 - 3.77 (m, 2H), 3.39 (s, 3H), 3.30 (s, 6H), 2.91 (s, 2H), 2.42 - 2.21 (m, 4H), 1.86 - 1.77 (m, 2H), 1.66 - 1.54 (m, 2H), 1.30 (s, 6H). MS 490.3 (M+1).

**Example 92**

Preparation of 1-(5-chloro-4-(_5,5-dimethyl-5,6-dihydro-4H-pyrrolo[1,2-b]pyrazol-3-yl) pyridin-2-yl)-3-((1r, 4r)-4-morpholinylcyclohexyl)urea (compound 92)

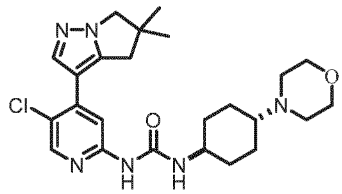

The synthesis method was the same as Example 15 except that 5-chloro-4-bromopyridin-2-amine was used instead of 4-bromopyridin-2-amine, and (1r, 4r)-4-morpholinylcyclohexyl-1-amine was used instead of N-Boc-trans-1,4-cyclohexanediamine. $^1$H NMR (400 MHz, DMSO-$d_6$) δ 9.20 (s, 1H), 8.46 (s, 1H), 8.16 (s, 2H), 6.59 (br s, 1H), 3.95 (s, 2H), 3.60 - 3.52 (m, 4H), 3.32 - 3.15 (m, 1H), 2.88 (s, 2H), 2.64 - 2.51 (m, 1H), 2.49 - 2.41 (m, 4H), 2.30 - 2.11 (m, 2H), 1.97 - 1.83 (m, 4H), 1.59 - 1.45 (m, 2H), 1.30 (s, 6H). MS 473.2 (M+1).

**(2) Examples of biological activity detection**

**Experimental Example 1: Evaluation Experiment of CDK Enzyme Inhibitory Activity at the Molecular Level**

Experimental method: Recombinant human CDK9/cyclin T1 (Carna bioscience, Cat#04-110) was diluted with dilution buffer (20 mM Tris HCl (pH 7.5), 0.02% Triton X-100, 0.01% BSA, 2 mM DTT, 0.5 mM Na3VO4, 10% Glycerol) to 2.5 ng/μl, the substrates CDK7/9 tide (ThermoFisher, Cat#PV5090) and ATP (sigmaaldrich, Cat#A1852) were diluted with assay buffer (25 mM Tris HCl (pH7.5), 10 mM MgCl2, 0.5 mM EGTA (pH 8), 0.5 mM Na3VO4, 5mMb-glycerol phosphate, 0.1% Triton X-100, 2.5 mM DTT) to final concentrations of 100 μM and 10 μM respectively, aliquot and set aside. 2 μl of diluted recombinant human CDK9/cyclin T1, 1 μl of test compound, and 2 μl of diluted substrate CDK7/9 tide containing ATP were added to the 384-well plate. Incubation was conducted at 37°C for 120 min and then 5 μl of ADP-Glo (Promega ADP-Glo Kinase Assay kit, Cat#V9102) was added, incubated at room temperature for 45 minutes, then 10 μl of kinase detection reagent (Promega ADP-Glo Kinase Assay kit, Cat#V9102) was added, and incubated at room temperature for 30 minutes, and then the luminescence was measured with a BioTek microplate reader. The positive control was the well in which enzyme and substrate were co-incubated, and the negative control was the well

in which dilution buffer and substrate were co-incubated. The inhibition rate was calculated according to the following formula.

$$\left(1-\frac{lum_{sample}-lum_{negative\ control}}{lum_{positive\ control}-lum_{negative\ control}}\right)\times 100\%$$

CDK 1,4,6,7 enzyme (CDK1/cyclin B, CDK4/cyclin D1, CDK6/cyclin D1, CDK7/cyclin H) activity was measured using a 5 μL enzyme system (50 mM HEPES, 10 mM MgCl2, 1 mM EGTA, 2 mM DTT and 0.01% Tween 20, appropriate enzyme) either. A mixture of 2.5 μL of compound, 2.5 μL of substrate and ATP (final concentration of substrate 50 nM, final concentration of ATP 200 μM) was incubated at room temperature in the dark for 60 minutes. Then 5 μL EDTA stop solution diluted in 1x detection buffer (final concentration 6 mM) was added to each well, then an antibody diluted in 1x detection buffer (final concentration 2 nM) was added to incubate for 1 hour at room temperature in the dark, and then the corresponding enzyme activity inhibition rate or $IC_{50}$ was calculated after measurement.

Experimental results: The inhibitory activities of the compounds prepared in the preparation examples of the present invention, as well as the positive control compound AZD4573 and the positive control compound Dinaciclib on CDK9 enzyme activity are shown in Table 1.

Table 1. Inhibitory effects of compounds on CDK9 enzyme activity

| No. | Inhibition rate at different concentrations (%) | | No. | Inhibition rate at different concentrations (%) | |
|---|---|---|---|---|---|
| | 100 nM | 10 nM | | 100 nM | 10 nM |
| Example 1 | 97.3 | 48.5 | Example 48 | 74.0 | 54.0 |
| Example 2 | 88.0 | 57.0 | Example 49 | 89.2 | 58.0 |
| Example 3 | 96.7 | 50.2 | Example 50 | 95.1 | 53.2 |
| Example 4 | 93.2 | 45.1 | Example 51 | 92.8 | 60.2 |
| Example 5 | 88.2 | 46.2 | Example 52 | 78.5 | 39.7 |
| Example 6 | 74.1 | 32.0 | Example 53 | 59.8 | 40.3 |
| Example 7 | 81.1 | 34.3 | Example 54 | 97.4 | 70.8 |
| Example 8 | 61.3 | 23.1 | Example 55 | 93.1 | 61.3 |
| Example 9 | 55.1 | 23.0 | Example 56 | 95.2 | 44.9 |
| Example 10 | 94.6 | -12.6 | Example 57 | 86.5 | 55.0 |
| Example 11 | 93.4 | 24.7 | Example 58 | 98.2 | 65.1 |
| Example 12 | 69.1 | 19.2 | Example 59 | 62.0 | 72.0 |
| Example 13 | 72.3 | -17.5 | Example 60 | 87.3 | 49.0 |
| Example 14 | 72.3 | -7.6 | Example 61 | 85.1 | 58.3 |
| Example 15 | 91.5 | 51.1 | Example 62 | 90.9 | 54.0 |
| Example 16 | 95.7 | 72.0 | Example 63 | 69.3 | 40.2 |
| Example 17 | 90.2 | 68.2 | Example 64 | 56.0 | 28.0 |
| Example 18 | 86.0 | 84.0 | Example 65 | 84.0 | 58.0 |
| Example 19 | 87.4 | 57.1 | Example 66 | 69.0 | 57.0 |
| Example 20 | 88.3 | 38.9 | Example 67 | 85.0 | 68.0 |
| Example 21 | 91.0 | 45.2 | Example 68 | 89.0 | 44.0 |
| Example 22 | 93.5 | 62.9 | Example 69 | 79.5 | 33.8 |
| Example 23 | 87.4 | 37.2 | Example 70 | 83.7 | 40.0 |

(continued)

| No. | Inhibition rate at different concentrations (%) | | No. | Inhibition rate at different concentrations (%) | |
|---|---|---|---|---|---|
| | 100 nM | 10 nM | | 100 nM | 10 nM |
| Example 24 | 73.1 | 37.8 | Example 71 | 64.0 | 34.0 |
| Example 25 | 61.2 | 28.7 | Example 72 | 69.0 | 45.0 |
| Example 26 | 82.5 | 39.3 | Example 73 | 86.0 | 70.0 |
| Example 27 | 78.4 | 44.2 | Example 74 | 92.0 | 63.0 |
| Example 28 | 84.3 | 29.5 | Example 75 | 83.0 | 51.0 |
| Example 29 | 57.0 | 19.4 | Example 76 | 91.0 | 25.0 |
| Example 30 | 55.3 | 23.1 | Example 77 | 92.0 | 60.3 |
| Example 31 | 51.3 | 20.7 | Example 78 | 80.0 | 68.0 |
| Example 32 | 59.8 | 18.1 | Example 79 | 87.0 | 53.0 |
| Example 33 | 81.0 | 11.9 | Example 80 | 56.0 | 48.0 |
| Example 34 | 54.0 | 7.0 | Example 81 | 91.5 | 58.3 |
| Example 35 | 62.7 | 30.0 | Example 82 | 79.2 | 38.0 |
| Example 36 | 62.8 | 16.5 | Example 83 | 68.3 | 40.2 |
| Example 37 | 60.0 | 10.0 | Example 84 | 66.3 | 39.5 |
| Example 38 | 27.0 | 51.0 | Example 85 | 72.0 | 28.9 |
| Example 39 | 55.0 | 28.0 | Example 86 | 88.3 | 50.1 |
| Example 40 | 59.5 | 25.3 | Example 87 | 75.3 | 43.8 |
| Example 41 | 70.2 | 38.1 | Example 88 | 94.2 | 55.7 |
| Example 42 | 75.0 | 44.0 | Example 89 | 92.7 | 63.7 |
| Example 43 | 87.3 | 49.0 | Example 90 | 98.0 | 61.0 |
| Example 44 | 96.0 | 83.0 | Example 91 | 88.0 | 63.0 |
| Example 45 | 94.3 | 48.9 | Example 92 | 93.7 | 58.9 |
| Example 46 | 94.0 | 55.0 | AZD4573 | $IC_{50}$ = 5.3 nM | |
| Example 47 | 84.0 | 71.0 | Dinaciclib | $IC_{50}$ = 3.0 nM | |
| Note: The inhibitory rate/$IC_{50}$ value of the compound against the phosphorylation of the kinase substrate was repeated twice independently and expressed as the average value. | | | | | |

Experimental conclusion: It can be seen from Table 1 that in the biological activity evaluation, the 2-heteroaromatic ring substituted urea compounds of the present invention all have high inhibitory activity against CDK9, and the inhibition rates of CDK9 enzyme activity at a lower concentration of 100 nM are all more than 50%, in which 39 compounds have high inhibitory activity against CDK9 (enzyme activity inhibition rate > 50% @ 10 nM), which reflects that this type of compounds has good CDK9 inhibitory activity advantages.

The inhibitory activities of compound 2, compound 3, compound 46, compound 47 and compound 67 against CDK9, CDK1, CDK4, CDK6 and CDK7 enzyme activities are shown in Table 2. The results show that 2-heteroaromatic ring substituted urea compounds have the advantage of high CDK9 subtype selectivity.

Table 2. Inhibitory activities of different CDK subtypes of compounds

| Compound | CDK9/CycT1 $IC_{50}$ (nM) | CDK1/Cyc B inhibition rate % | | CDK4/Cyc D1 inhibition rate % | | CDK6/Cyc D 1 inhibition rate % | | CDK7/Cyc H inhibition rate % | |
|---|---|---|---|---|---|---|---|---|---|
| | | 100nM | 10nM | 100nM | 10nM | 100nM | 10nM | 100nM | 10nM |
| Example 2 | 5.7 | 7.8 | 1.3 | 1.8 | -7.0 | 13.8 | 2.6 | -3.8 | -6.6 |
| Example 3 | 6.2 | -1.4 | -1.9 | 25.1 | 7.2 | 18.9 | 9.9 | -2.6 | -24.5 |
| Example 46 | 12.0 | 11.6 | -5.1 | 20.9 | 11.1 | 21.6 | 6.2 | 59 | 24 |
| Example 47 | 5.1 | 33.4 | 5.0 | 41.1 | 13.6 | 26.7 | 4.6 | 50 | 15 |
| Example 67 | 5.3 | 47.4 | 4.7 | 18.0 | 9.5 | 19.1 | 4.0 | 44 | -4 |

**Experimental Example 2: Experiment on growth inhibition of CDK9-positive tumor cells by representative compounds**

Experimental method: WSU-DLCL2 cells in good condition were seeded in a 96- well plate at 20,000/well. The test compound was gradient diluted with PBS and then added to the wells. After incubating with the cells for 72 hours, 10 μl of CCK-8 reagent was added to each well. After further incubation, the absorbance was measured at 460 nm and the 4- parameter curve fitting of Softmax Pro software was used to calculate the proliferation inhibition $IC_{50}$ of the test compound.

Table 3. Inhibitory activity of compound 47 and AZD4573 on tumor cell proliferation in vitro

| Compound | WSU-DLCL2 ($IC_{50}$, nM) | U2932 ($IC_{50}$, nM) | Pfeiffer ($IC_{50}$, nM) | Will2 ($IC_{50}$, nM) | 422 ($IC_{50}$, nM) |
|---|---|---|---|---|---|
| Example 47 | <4.5 | 0.932 | 0.052 | 2.162 | 0.089 |
| AZD4573 | <3 | 2.322 | 2.427 | 5.749 | 1.809 |

The results in Table 3 show that the representative compound 47 of the present invention has potent inhibitory activity on the growth of various tumor cells in vitro. The $IC_{50}$ value can reach low nanomolar or even picomolar levels, and is superior to AZD4573, a positive control compound currently in clinical phase II. In particular, the $IC_{50}$ value of the growth inhibition of Pfeiffer and 422 cells reached the picomolar level, which was 46.67 and 20.33 times higher than that of the positive control compound respectively, fully demonstrating that the 2-heteroaromatic ring substituted urea compound of the present invention has significant advantage in vitro anti-tumor activity.

**Experimental Example 3: Effect of Preferred Compound 47 on Activation of CDK9 Signaling Pathway in Tumor Cells**

Western Blot was used to detect the effect of compounds on the activation of CDK9 signaling pathway in WSU-DLCL2 cells.

Experimental method: An appropriate amount of WSU-DLCL2 cells were seeded in a 6-well plate. After 24 hours of exposure to the test compound, the cells were collected by centrifugation. A corresponding amount of 1X loading buffer (50mM Tris-HCl (pH6.8), 2% SDS, 0.1% bromophenol blue, 10% glycerol) was added, the sample was boiled at 100°C after lysis. The same volume of sample was taken. After SDS-PAGE electrophoresis, the protein on the gel was transferred to a nitrocellulose membrane. The corresponding band was cut according to the size of the protein and blocked TBST containing 5% skimmed milk powder (20mM Tris- HCl, 150mM NaCl, 0.1% Tween20, adjust pH to 7.4) for 1 hour, and primary antibody was incubated overnight at 4°C. TBST was used to elute excess primary antibody for 10 minutes each time for three times, the secondary antibody was incubated at room temperature for 1 hour, and then TBST was used to elute excess secondary antibody for 10 minutes each time for three times. Finally, the strips were colored and photographed using a Bio-Rad colorimeter.

The experimental results are shown in Figure 1. The preferred compound 47 inhibited the phosphorylation of

Pol II S2 position in a concentration-dependent manner after 24 hours of action, indicating that it can also significantly inhibit the activity of CDK9 at the cellular level. Under the action of 50 nM of compound 47, the phosphorylation of Pol II S2 position is significantly down-regulated when compared with the control group. And under a concentration of 100nM, the phosphorylation of this site is basically completely inhibited. In addition, it can be seen that the expression of the corresponding downstream protein Mcl-1, which is regulated by CDK9 transcription, is also inhibited in a concentration-dependent manner. Under the action of the compound concentration of 50nM, Mcl-1 has a significant decrease. When the compound concentration is increased to 100 nM, Mcl-1 expression was almost completely blocked.

**Experimental Example 4: Preliminary in vivo metabolism results**

After compound 46, compound 47 and positive compound AZD4573 were administered to mice intravenously (iv) and orally (po) respectively, blood samples were collected at different time points, and LC-MS/MS was used to determine the concentration of the compound in the mouse plasma after administration of the test substance. The relevant pharmacokinetic parameters were calculated to examine the oral bioavailability and pharmacokinetic properties of the compound in mice. The results are shown in Table 4.

Table 4 PK parameters of compounds 46, 47 and AZD4573 in mice

| Compound | Path of administ ration | $T_{1/2}$ | $T_{max}$ | $C_{max}$ | $AUC_{last}$ | $AUC_{INF_obs}$ | $CL_{_obs}$ | $MRT_{INF_obs}$ | $Vss_{_obs}$ | F |
|---|---|---|---|---|---|---|---|---|---|---|
| | | (h) | (h) | (ng/mL) | (h*ng/mL) | (h*ng/mL) | (mL/min/kg) | (h) | (mL/kg) | (%) |
| Example 46 | p.o. | 2.38 | 1.00 | 382 | 601 | 602 | - | 1.70 | - | 24.8 |
| | i.v. | 2.09 | - | - | 728 | 729 | 68.6 | 0.84 | 3469 | |
| Example 47 | p.o. | 1.50 | 0.50 | 58.2 | 98.8 | 100 | - | 1.57 | - | 6.18 |
| | i.v. | 3.84 | - | - | 480 | 491 | 102 | 2.34 | 14318 | |
| AZD4573 | p.o. | 0.27 | 0.25 | 219 | 73.2 | 73.6 | - | 0.42 | - | 5.28 |
| | i.v. | 0.11 | - | - | 416 | 419 | 119 | 0.11 | 820 | |

As can be seen from Table 4, the plasma exposure and oral bioavailability of compounds 46 and 47 are better than those of the positive compound AZD4573, and their plasma clearance rates are lower, indicating better druggability.

Furthermore, the identification results of metabolites of compound 47 and the positive compound AZD4573 in mouse plasma (as shown in the following chemical reaction formula and Tables 5-6) show that compound 47 has higher plasma stability, lower body clearance rate and better drugability than the positive compound.

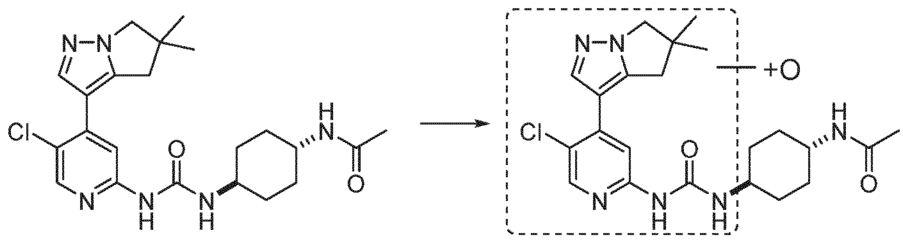

M0 M1

Metabolic pathways of compound 47 in mouse plasma

Table 5. Metabolites of compound 47 in mouse plasma

| No. | Metabolite name | m/z | Chemical formula | Deviation | | Time (min) | MS Area (254nm) | |
|---|---|---|---|---|---|---|---|---|
| | | | | mDa | PPM | | po | iv |
| M0 | Original form | 445.2044 | C22H29ClN6O2 | -7.5 | -16.8 | 6.78 | 811 | 62967 (532) |
| M1 | monooxidation | 461.2034 | C22H29ClN6O3 | -3.4 | -7.3 | 5.06 | 141 | 1876 (39) |

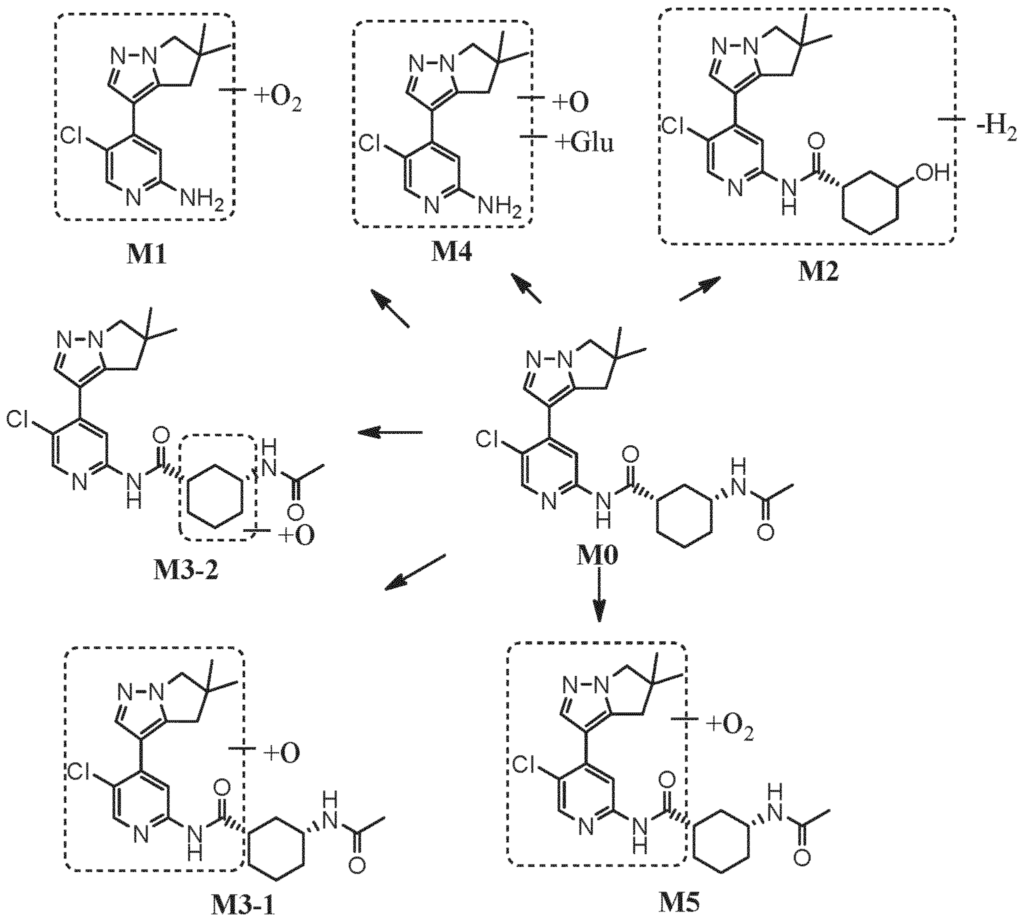

M1 M4 M2 M3-2 M0 M3-1 M5

Note: Glu : Glucuronidation.

Metabolic pathways of compound AZD4573 in mouse plasma

Table 6. Metabolites of compound AZD4573 in mouse plasma

| No. | Metabolite name | m/z | Chemical formula | Deviation | | Time (min) | MS Area (254nm) | |
|---|---|---|---|---|---|---|---|---|
| | | | | mDa | PPM | | po | iv |
| M0 | Original form | 430.199 | C22H28ClN5O2 | -1.8 | -4.1 | 7.00 | 2900 (132) | 51904 (540) |
| M1 | Hydrolyze and double oxidation | 295.097 | C13H15ClN4O2 | 0.5 | 1.9 | 2.46 | 419 | 442 |
| M2-1 | N-dealkylation and dehydrogenation | 387.155 | C20H23ClN4O2 | -3.8 | -9.7 | 6.58 | - | 196 |
| M2-2 | N-dealkylation and dehydrogenation | 387.163 | C20H23ClN4O2 | 4.0 | 10.5 | 7.51 | 52.3 | 182 |
| M3-1 | monooxidation | 446.192 | C22H28ClN5O3 | -4.2 | -9.4 | 6.49 | 291 | 363 |
| M3-2 | monooxidation | 446.195 | C22H28ClN5O3 | -1.0 | -2.2 | 5.29 | 1878 (22) | 3811 (63) |
| M4 | Hydrolysis and monooxidation and glucuronidation | 455.134 | C19H23ClN4O7 | 0.7 | 1.5 | 4.29 | 1069 | 345 |
| M5 | Double oxidation | 462.190 | C22H28ClN5O4 | -0.4 | -0.8 | 4.75 | 190 | 644 |

In summary, it can be seen that the compounds containing 2-heteroaromatic ring substituted ureas in the examples of the present invention have excellent inhibitory activity against CDK9, and at the same time have the advantages of high selectivity for CDKs subtypes, as well as better druggability, and are expected to solve the current clinical problems of off-target toxicity and druggability of CDK inhibitors caused by poor selectivity. The representative compound of the present invention (Example 47) has a very high inhibitory effect on the in vitro proliferation of a variety of CDK9-positive malignant tumor cells. The $IC_{50}$ value can reach low nanomolar or even picomolar levels, which is significantly better than AZD4573, a compound currently in clinical researching. It shows that 2-heteroaromatic ring substituted urea compounds have excellent anti-tumor activity advantages. In addition, preliminary pharmacokinetic data indicate that the representative compound of the present invention (Example 47) has better druggability than the positive compound AZD4573, such as higher plasma stability, higher plasma exposure and lower clearance rate.

The preferred embodiments of the present invention are described in detail above with reference to the accompanying drawings. However, the present invention is not limited to the specific details of the above embodiments. Within the scope of the technical concept of the present invention, various simple modifications can be made to the technical solution of the present invention. Such simple modifications all belong to the protection scope of the present invention.

## Claims

1. A urea compound containing 2-heteroaromatic ring substitution of formula (I), its enantiomers, diastereomers, racemates or mixtures thereof, or pharmaceutically acceptable salts thereof, solvate, metabolite or prodrug:

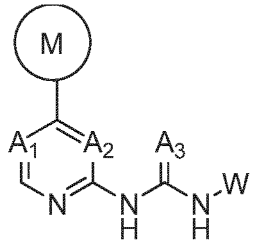

(I)

in which

$A_1$ and $A_2$ are each independently selected from N or C-$R_1$;
$A_3$ is selected from O or S;
M is selected from unsubstituted or substituted 5-8 membered aryl, unsubstituted or substituted 5-8 membered heteroaryl, unsubstituted or substituted 5-10 membered heterocyclyl; the substitution means that each of the above groups is independently substituted by 1-5 $R_2$ groups; or two $R_2$ and the two atoms connected to each of them form a 5-7 membered cycloalkyl, heterocyclyl or spirocyclyl, and further substituted by 0-5 R' group; wherein one or more $CH_2$ on the above-mentioned ring are optionally replaced by a corresponding number of C(O) groups, and one or more S atom or N atom on the ring are optionally oxidized to form S-oxide or N-oxide;
W is selected from unsubstituted or substituted C1-C6 alkyl, unsubstituted or substituted 3-7 membered cycloalkyl, unsubstituted or substituted 3-7 membered heterocycloalkyl, unsubstituted or substituted 5-10 membered heterocyclyl, unsubstituted or substituted -($C_{1-6}$ alkyl)-(3-7 membered cycloalkyl), unsubstituted or substituted -($C_{1-6}$ alkyl)-(3-7 membered heterocycloalkyl), unsubstituted or substituted 4-8 membered cycloalkenyl, unsubstituted or substituted 4-8 membered heterocycloalkenyl, unsubstituted or substituted -($C_{1-6}$ alkyl)-(4-8 membered cycloalkenyl), unsubstituted or substituted -($C_{1-6}$ alkyl)-(4-8 membered heterocycloalkenyl), unsubstituted or substituted 5-8 membered aryl, unsubstituted or substituted 5-8 heteroaryl, unsubstituted or substituted -($C_{1-6}$ alkyl)-(5-8 membered heteroaryl), unsubstituted or substituted -(5-8 membered heteroaryl)-($C_{1-6}$ alkyl), unsubstituted or substituted -(5-8 membered heteroaryl)-(3-7 membered cycloalkyl), unsubstituted or substituted -(5-8 membered heteroaryl)-(3-7 membered heterocycloalkyl), unsubstituted or substituted -($C_{1-6}$ alkyl)-(5-10 membered heterocyclyl); the substitution means each independently substituted by 1-5 $R_4$ groups; or two $R_4$ and the two atoms connected to each of them form a cycloalkyl or heterocyclyl and are further substituted by 0-5 R' group; wherein one or more $CH_2$ on the above-mentioned ring are optionally replaced by a corresponding number of C(O) groups, and one or more S atom or N atom on the ring are optionally oxidized to form S-oxide or N-oxi de;
$R_1$ is selected from hydrogen, halogen, cyano or $C_{1-3}$ alkyl, $C_{3-6}$ cycloalkyl;
$R_2$ at each occurrence is independently selected from halogen, hydroxyl, amino, cyano, $C_{1-4}$ alkyl, $C_{1-4}$ heteroalkyl, $C_{2-6}$ alkenyl, $C_{2-6}$ alkynyl, $C_{3-6}$ cycloalkyl, 3-6 membered heterocycloalkyl, 4-8 membered cycloalkenyl, 4-8 membered heterocycloalkenyl, -O($C_{1-6}$ alkyl), -O($C_{3-6}$ cycloalkyl), -NH($C_{1-6}$ alkyl), -NH($C_{3-6}$ cycloalkyl), -C(O)($C_{1-4}$ alkyl), -C(O)($C_{3-6}$ cycloalkyl); wherein the above substituent is further substituted by 0-5 substituents selected from the following: halogen, hydroxyl, cyano group, 5-7 membered aryl, 5-7 membered heteroaryl, $C_{3-6}$ cycloalkyl, 3-6 membered heterocycloalkyl, 3-6 membered heterocycloalkyl, -$C_{1-4}$ alkyl-$R_3$, -$OR_3$, -$NHR_3$, -N$(R_3)_2$, -NH$C_{1-4}$ alkyl-$R_3$, -O$C_{1-4}$ alkyl-$R_3$, -CONH$R_3$, -CON$(R_3)_2$, -S$R_3$, -SO$R_3$, -$SO_2R_3$;
$R_3$ is selected from 5-7 membered aryl, 5-7 membered heteroaryl, $C_{3-6}$ cycloalkyl, 3-6 membered heterocycloalkyl, 3-6 membered heterocycloalkyl; the substituent is further substituted by 0-5 groups selected from the following: halogen, hydroxyl, $C_{1-4}$ alkyl, -O($C_{1-4}$ alkyl), amino, -NH($C_{1-4}$ alkyl), -C(O)($C_{1-4}$ alkyl), -NHC(O)($C_{1-4}$ alkyl);
$R_4$ at each occurrence is independently selected from halogen, carbonyl, cyano, nitro, $C_{1-6}$ alkyl, $C_{1-6}$ heteroalkyl, $C_{2-6}$ alkenyl, $C_{2-6}$ alkynyl, $C_{3-6}$ cycloalkyl, 3-6 membered heterocycloalkyl, 4-8 membered cycloalkenyl, 4-8 membered heterocycloalkenyl, 5-7 membered aryl, 5-7 membered heteroaryl, ($C_{1-6}$ alkyl)-O-($C_{1-6}$ alkyl), $OR_5$, $SR_5$, S(O)$R_5$, $SO_2R_5$, C(O)$R_5$, C(O)N$(R_5)_2$, C(O)N$R_5$O$R_5$, C(O)N$R_5$$SO_2R_5$, CO(O)$R_5$, N$(R_5)_2$, N$R_5$C(O)$R_5$, N$R_5$$SO_2R_5$, N$R_5$$SO_2$N$R_5$C(O)O$R_5$, N$R_5$C(O)N$(R_5)_2$, OC(O)$R_5$, OC(O)O$R_5$, OC(O)N$(R_5)_2$, OC(O)N$R_5$$SO_2R_5$, $SO_2$N$(R_5)_2$, $SO_2$N$R_5$C(O)$R_5$, N$R_5$S$(O)_2R_5$, N$R_5$C(O)O$R_5$, $SO_2$N$R_5$C(O)O$R_5$, O$SO_2$N$(R_5)_2$; wherein any of the alkyl, cycloalkyl, heterocycloalkyl, alkenyl, alkynyl, cycloalkenyl, heterocycloalkenyl, aryl and heteroaryl are further substituted by 0-5 $R_5$ groups;
$R_5$ at each occurrence is independently selected from hydrogen, halogen, hydroxyl, amino, carboxyl, aldehyde group, carbonyl, cyano, nitro, $C_{1-6}$ alkyl, $C_{2-6}$ alkenyl, $C_{2-6}$ alkynyl, $C_{3-6}$ cycloalkyl, 3-6 membered heterocy-

cloalkyl, -O($C_{1-6}$ alkyl), ($C_{1-6}$ alkyl)-O-($C_{1-6}$ alkyl); the above alkyl, alkenyl, alkynyl, cycloalkyl and heterocycloalkyl are substituted by 0-5 R' groups; or two $R_5$ groups together with the two atoms to which they are connected form 3-6 membered cycloalkyl or heterocycloalkyl and substituted by 0-5 R' groups;

R' at each occurrence is independently selected from H, halogen, cyano, hydroxyl, -O-($C_{1-6}$ alkyl), -C(O)R", -C(O)OR", $C_{1-6}$ alkyl, $C_{3-6}$ cycloalkyl, $C_{1-6}$ haloalkyl, $C_{1-6}$ heteroalkyl, hydroxy $C_{1-6}$ alkyl, -N(R")(R"), NHC(O)-($C_{1-3}$ alkyl), unsubstituted or substituted cycloalkyl; or two R' together with the atoms to which they are connected form a 3-6 membered cycloalkyl or heterocycloalkyl; wherein the alkyl, cycloalkyl and heterocycloalkyl are substituted by 0-5 R" groups;

R" at each occurrence is independently selected from hydrogen, hydroxyl, and $C_{1-6}$ alkyl.

**2.** The urea compounds containing 2-heteroaromatic ring substitution according to claim 1, their enantiomers, diastereomers, racemates or mixtures thereof, or pharmaceutically acceptable salts thereof, solvate, metabolite or prodrug, wherein the urea compound is a compound represented by formula (II):

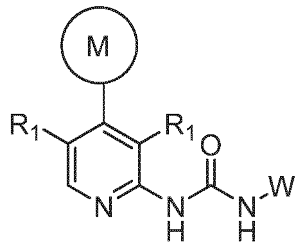

(II)

in which:

M is selected from unsubstituted or substituted phenyl, unsubstituted or substituted pyridyl, unsubstituted or substituted pyrimidinyl, unsubstituted or substituted pyrazolyl, unsubstituted or substituted imidazolyl, unsubstituted or substituted thiazole group; the substitution means that the above groups are each independently substituted by 1-5 $R_2$ groups; or two $R_2$ and the two atoms connected to each of them form a 5-7 membered cycloalkyl or heterocyclyl and further substituted by 0-5 R' groups; wherein one or more $CH_2$ on the above-mentioned ring are optionally replaced by a corresponding number of C(O) groups, and one or more S atom or N atom on the ring are optionally oxidized to form S-oxide or N-oxide;

W is selected from unsubstituted or substituted C1-C6 alkyl, unsubstituted or substituted 3-7 membered cycloalkyl, unsubstituted or substituted 3-7 membered heterocycloalkyl, unsubstituted or substituted 5-10 membered heterocyclyl, unsubstituted or substituted -($C_{1-6}$ alkyl)-(3-7 membered cycloalkyl), unsubstituted or substituted -($C_{1-6}$ alkyl)-(3-7 membered heterocycloalkyl), unsubstituted or substituted 4-8 membered cycloalkenyl, unsubstituted or substituted 4-8 membered heterocycloalkenyl, unsubstituted or substituted -($C_{1-6}$ alkyl)-(4-8 membered cycloalkenyl), unsubstituted or substituted -($C_{1-6}$ alkyl)-(4-8 membered heterocycloalkenyl), unsubstituted or substituted 5-8 membered aryl, unsubstituted or substituted 5-8 heteroaryl, unsubstituted or substituted -($C_{1-6}$ alkyl)-(5-8 membered heteroaryl), unsubstituted or substituted -(5-8 membered heteroaryl)-($C_{1-6}$ alkyl), unsubstituted or substituted -(5-8 membered heteroaryl)-(3-7 membered cycloalkyl), unsubstituted or substituted -(5-8 membered heteroaryl)-(3-7 membered heterocycloalkyl), unsubstituted or substituted -($C_{1-6}$ alkyl)-(5-10 membered heterocyclyl); the substitution means each independently substituted by 1-5 $R_4$ groups; or two $R_4$ and the two atoms connected to each of them form a cycloalkyl or heterocyclyl and are further substituted by 0-5 R' group; wherein one or more $CH_2$ on the above-mentioned ring are optionally replaced by a corresponding number of C(O) groups, and one or more S atom or N atom on the ring are optionally oxidized to form S-oxide or N-oxide;

$R_1$ is selected from hydrogen, halogen, cyano or $C_{1-3}$ alkyl, $C_{3-6}$ cycloalkyl

$R_2$ at each occurrence is independently selected from halogen, hydroxyl, amino, cyano, $C_{1-4}$ alkyl, $C_{1-4}$ heteroalkyl, $C_{2-6}$ alkenyl, $C_{2-6}$ alkynyl, $C_{3-6}$ cycloalkyl, 3-6 membered heterocycloalkyl, 4-8 membered cycloalkenyl, 4-8 membered heterocycloalkenyl, -O($C_{1-6}$ alkyl), -O($C_{3-6}$ cycloalkyl), -NH($C_{1-6}$ alkyl), -NH($C_{3-6}$ cycloalkyl), -C(O)($C_{1-4}$ alkyl), -C(O)($C_{3-6}$ cycloalkyl); wherein the above substituent is further substituted by 0-5 substituents selected from the following: halogen, hydroxyl, cyano group, 5-7 membered aryl, 5-7 membered heteroaryl, $C_{3-6}$ cycloalkyl, 3-6 membered heterocycloalkyl, 3-6 membered heterocycloalkyl, -$C_{1-4}$ alkyl-$R_3$, -$OR_3$, -$NHR_3$, -$N(R_3)_2$, -$NHC_{1-4}$ alkyl-$R_3$, -$OC_{1-4}$ alkyl-$R_3$, -$CONHR_3$, -$CON(R_3)_2$, -$SR_3$, -$SOR_3$, -$SO_2R_3$;

$R_3$ is selected from 5-7 membered aryl, 5-7 membered heteroaryl, $C_{3-6}$ cycloalkyl, 3-6 membered heterocycloalkyl, 3-6 membered heterocycloalkyl; the substituent is further substituted by 0-5 groups selected from the following: halogen, hydroxyl, $C_{1-4}$ alkyl, -O($C_{1-4}$ alkyl), amino, -NH($C_{1-4}$ alkyl), -C(O)($C_{1-4}$ alkyl), -NHC(O)($C_{1-4}$ alkyl);

$R_4$ at each occurrence is independently selected from halogen, carbonyl, cyano, nitro, $C_{1-6}$ alkyl, $C_{1-6}$ heteroalkyl, $C_{2-6}$ alkenyl, $C_{2-6}$ alkynyl, $C_{3-6}$ cycloalkyl, 3-6 membered heterocycloalkyl, 4-8 membered cycloalkenyl, 4-8 membered heterocycloalkenyl, 5-7 membered aryl, 5-7 membered heteroaryl, ($C_{1-6}$ allcyl)-O-($C_{1-6}$ alkyl), $OR_5$, $SR_5$, $S(O)R_5$, $SO_2R_5$, $C(O)R_5$, $C(O)N(R_5)_2$, $C(O)NR_5OR_5$, $C(O)NR_5SO_2R_5$, $CO(O)R_5$, $N(R_5)_2$, $NR_5C(O)R_5$, $NR_5SO_2R_5$, $NR_5SO_2NR_5C(O)OR_5$, $NR_5C(O)N(R_5)_2$, $OC(O)R_5$, $OC(O)OR_5$, $OC(O)N(R_5)_2$, $OC(O)NR_5SO_2R_5$, $SO_2N(R_5)_2$, $SO_2NR_5C(O)R_5$, $NR_5S(O)_2R_5$, $NR_5C(O)OR_5$, $SO_2NR_5C(O)OR_5$, $OSO_2N(R_5)_2$; wherein any of the alkyl, cycloalkyl, heterocycloalkyl, alkenyl, alkynyl, cycloalkenyl, heterocycloalkenyl, aryl and heteroaryl are further substituted by 0-5 $R_5$ groups;

$R_5$ at each occurrence is independently selected from hydrogen, halogen, hydroxyl, amino, carboxyl, aldehyde group, carbonyl, cyano, nitro, $C_{1-6}$ alkyl, $C_{2-6}$ alkenyl, $C_{2-6}$ alkynyl, $C_{3-6}$ cycloalkyl, 3-6 membered heterocycloalkyl, -O($C_{1-6}$ alkyl), ($C_{1-6}$ alkyl)-O-($C_{1-6}$ alkyl); the above alkyl, alkenyl, alkynyl, cycloalkyl and heterocycloalkyl are substituted by 0-5 R' groups; or two $R_5$ groups together with the two atoms to which they are connected form 3-6 membered cycloalkyl or heterocycloalkyl and substituted by 0-5 R' groups;

R' at each occurrence is independently selected from H, halogen, cyano, hydroxyl, -O-($C_{1-6}$ alkyl), -C(O)R", -C(O)OR", $C_{1-6}$ alkyl, $C_{3-6}$ cycloalkyl, $C_{1-6}$ haloalkyl, $C_{1-6}$ heteroalkyl, hydroxy $C_{1-6}$ alkyl, -N(R")(R"), NHC(O)-($C_{1-3}$ alkyl), unsubstituted or substituted cycloalkyl; or two R' together with the atoms to which they are connected form a 3-6 membered cycloalkyl or heterocycloalkyl; wherein the alkyl, cycloalkyl and heterocycloalkyl are substituted by 0-5 R" groups;

R" at each occurrence is independently selected from hydrogen, hydroxyl, and $C_{1-6}$ alkyl.

3. The urea compounds containing 2-heteroaromatic ring substitution according to claim 2, their enantiomers, diastereomers, racemates or mixtures thereof, or pharmaceutically acceptable salts thereof, solvate, metabolite or prodrug, wherein

M is selected from the group consisting of

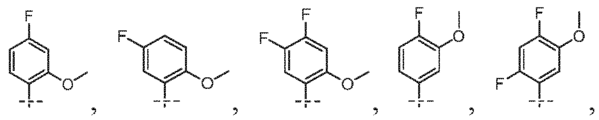
, , , , ,

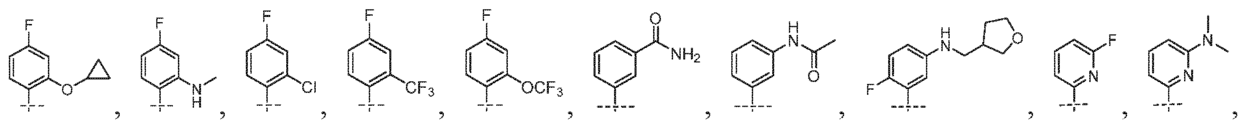
, , , , , , , , , ,

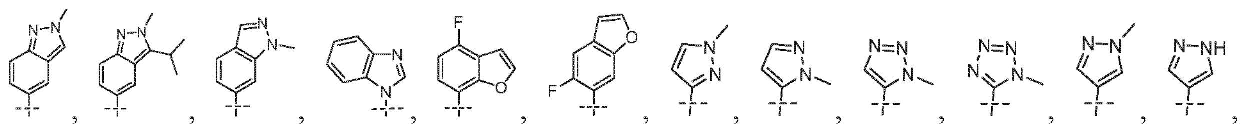
, , , , , , , , , , , ,

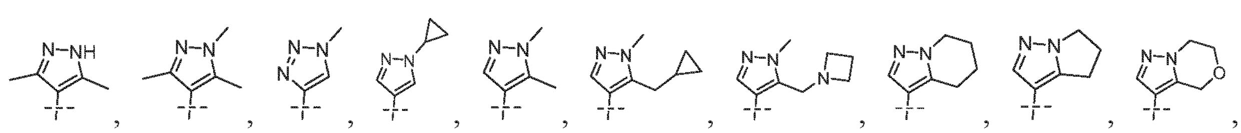
, , , , , , , , , ,

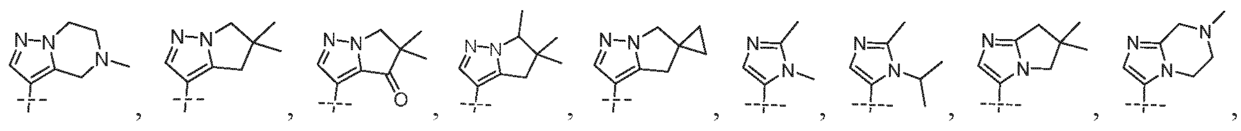
, , , , , , , , ,

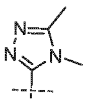, 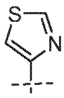, 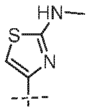, 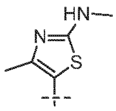, 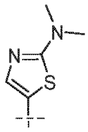, 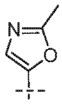, 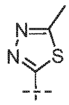, 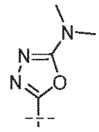, 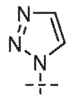, 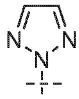, 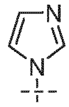, 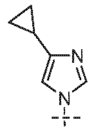,

and

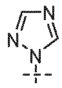,

$R_1$ is hydrogen or halogen.

**4.** The urea compounds containing 2-heteroaromatic ring substitution according to claim 1, their enantiomers, diastereomers, racemates or mixtures thereof, or pharmaceutically acceptable salts thereof, solvate, metabolite or prodrug, wherein
the compound of formula (I) or its deuterated compound or its pharmaceutically acceptable salt or prodrug is selected from the following compounds:

| No. | Structure | No. | Structure |
|---|---|---|---|
| 1 | 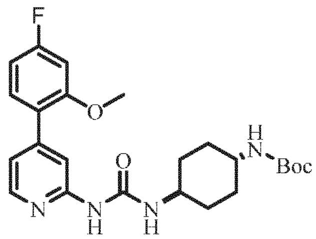 | 2 | 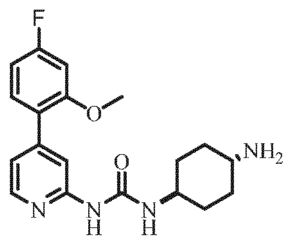 |
| 3 | 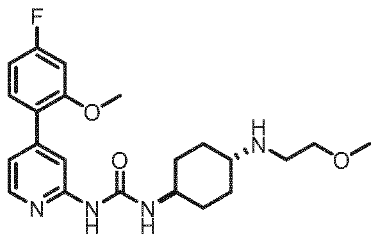 | 4 | 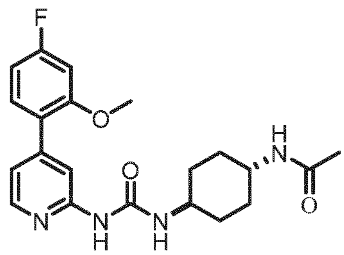 |
| 5 | 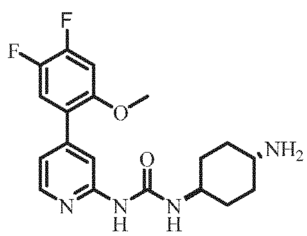 | 6 | 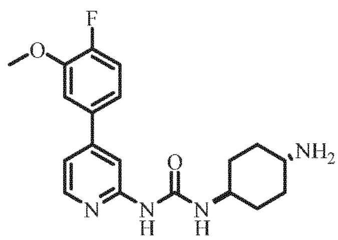 |
| 7 | 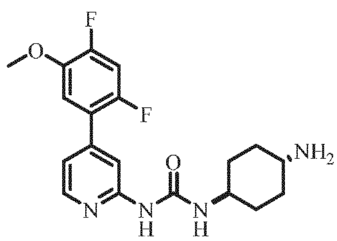 | 8 | 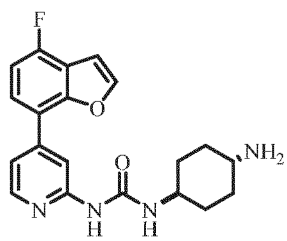 |

(continued)

| No. | Structure | No. | Structure |
|---|---|---|---|
| 9 | 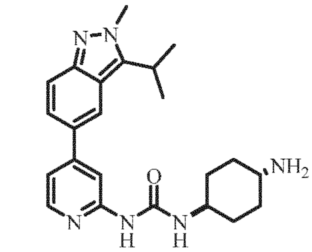 | 10 | 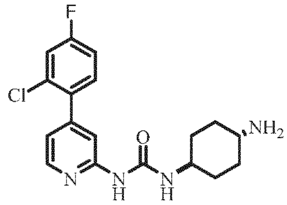 |
| 11 | 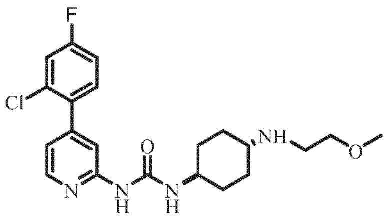 | 12 | 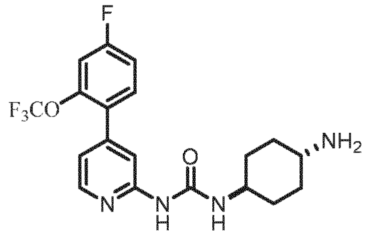 |
| 13 | 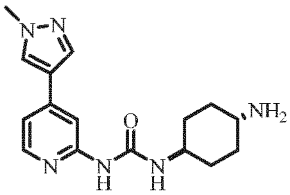 | 14 | 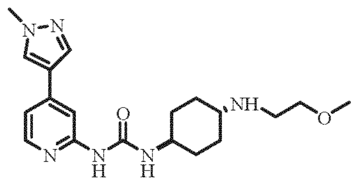 |
| 15 | 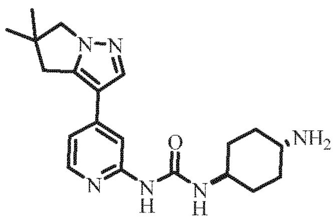 | 16 | 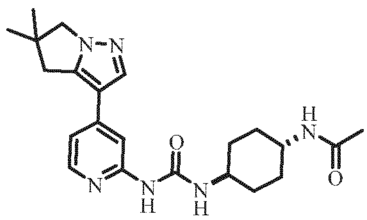 |
| 17 | 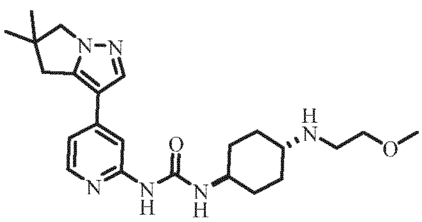 | 18 | 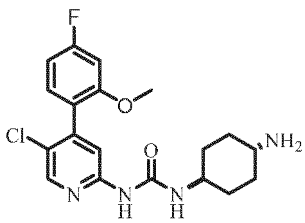 |
| 19 | 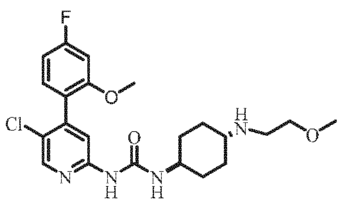 | 20 | 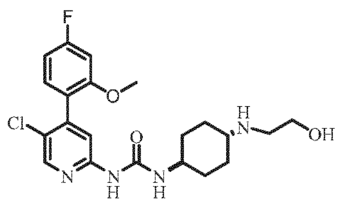 |
| 21 | 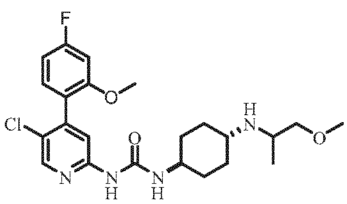 | 22 | 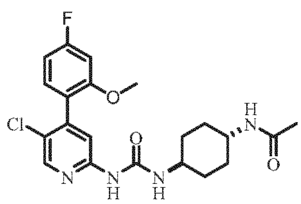 |

(continued)

| No. | Structure | No. | Structure |
|---|---|---|---|
| 23 | 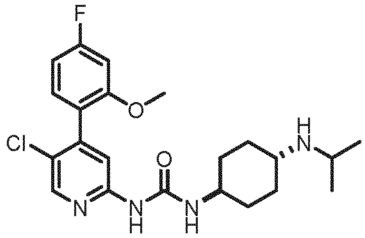 | 24 | 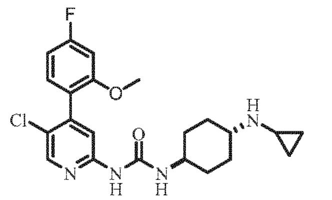 |
| 25 | 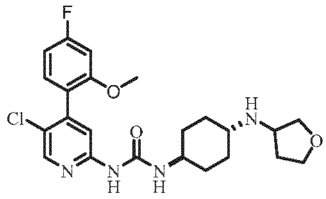 | 26 | 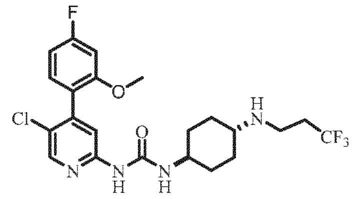 |
| 27 | 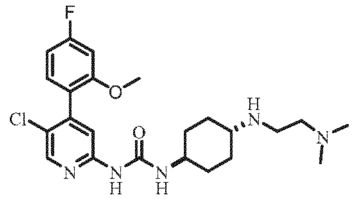 | 28 | 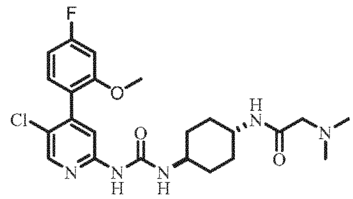 |
| 29 | 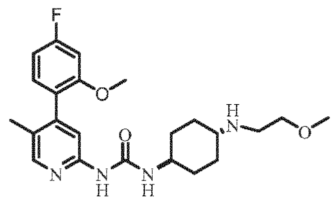 | 30 | 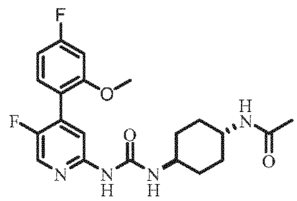 |
| 31 | 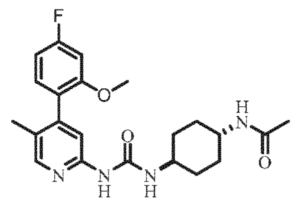 | 32 | 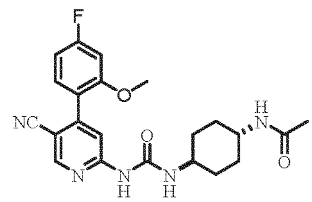 |
| 33 | 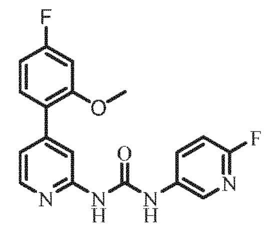 | 34 | 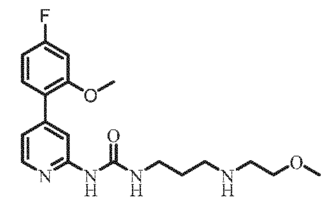 |
| 35 | 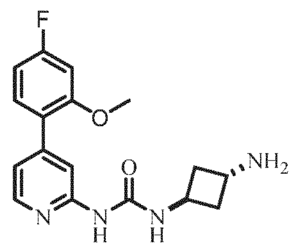 | 36 | 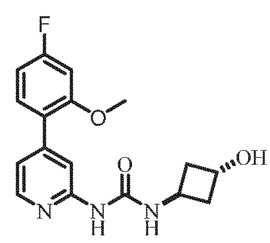 |

(continued)

| No. | Structure | No. | Structure |
|---|---|---|---|
| 37 | 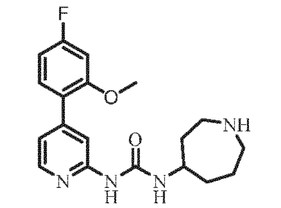 | 38 | 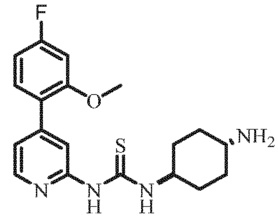 |
| 39 | 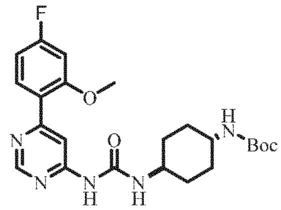 | 40 | 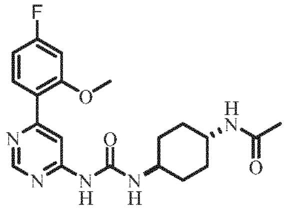 |
| 41 | 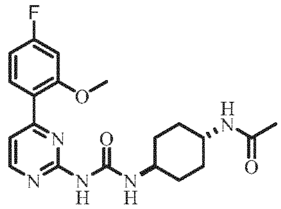 | 42 | 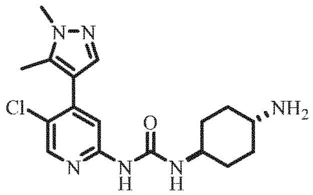 |
| 43 | 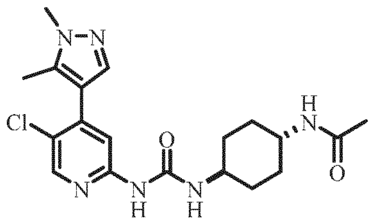 | 44 | 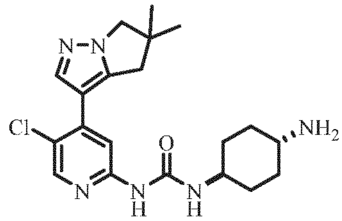 |
| 45 | 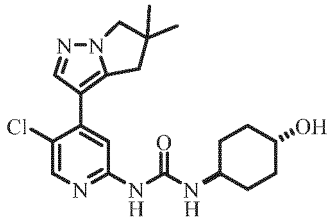 | 46 | 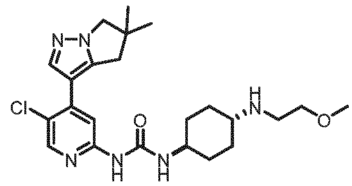 |
| 47 | 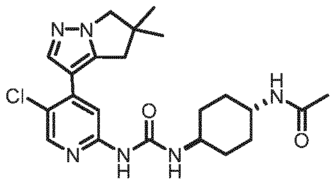 | 48 | 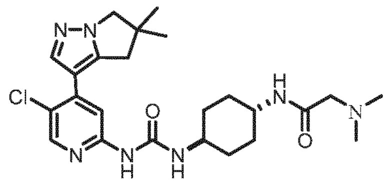 |
| 49 | 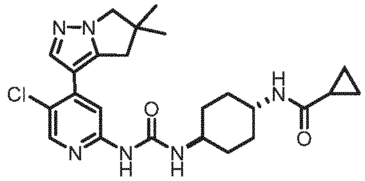 | 50 | 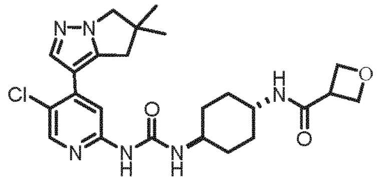 |

(continued)

| No. | Structure | No. | Structure |
| --- | --- | --- | --- |
| 51 | 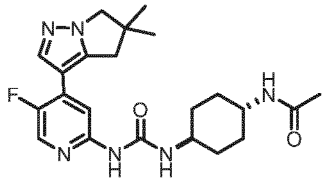 | 52 | 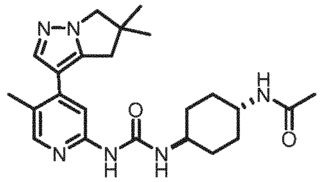 |
| 53 | 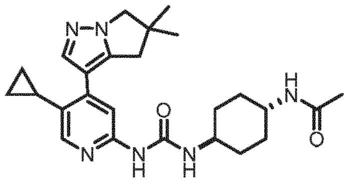 | 54 | 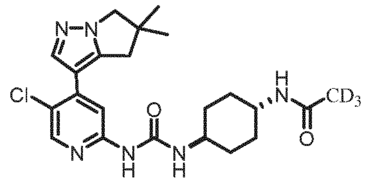 |
| 55 | 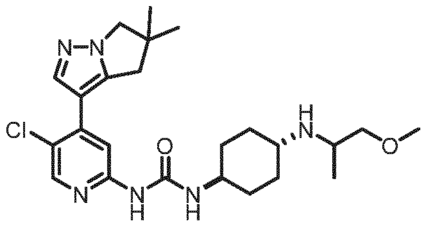 | 56 | 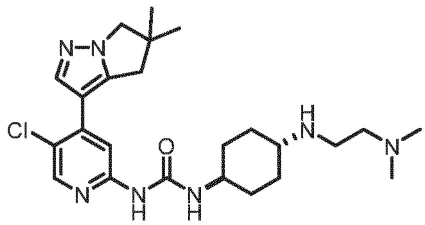 |
| 57 | 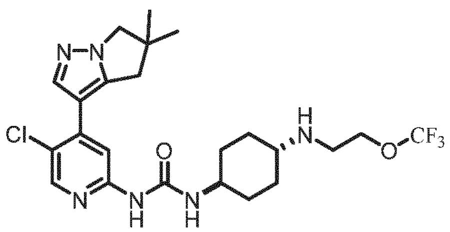 | 58 | 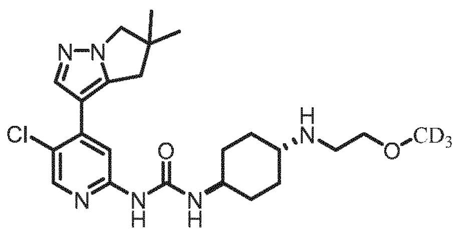 |
| 59 | 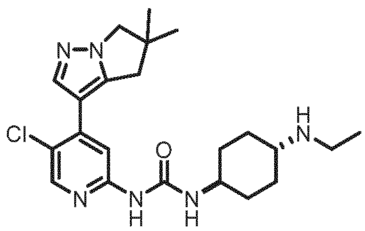 | 60 | 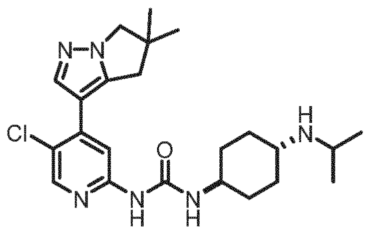 |
| 61 | 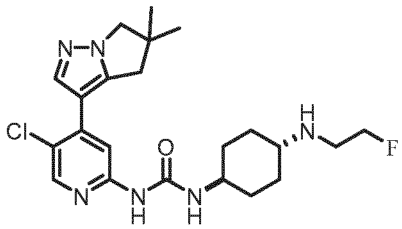 | 62 | 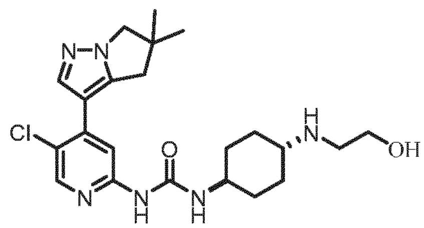 |
| 63 | 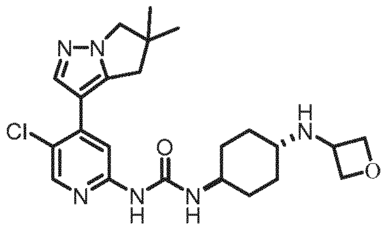 | 64 | 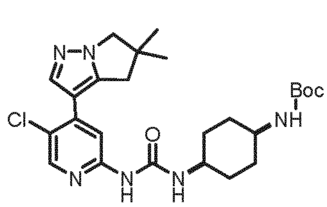 |

(continued)

| No. | Structure | No. | Structure |
|---|---|---|---|
| 65 | 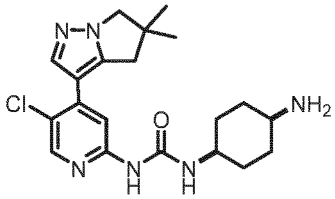 | 66 | 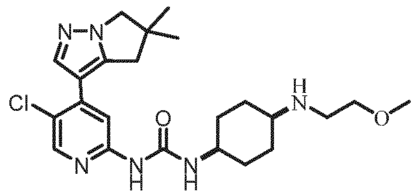 |
| 67 | 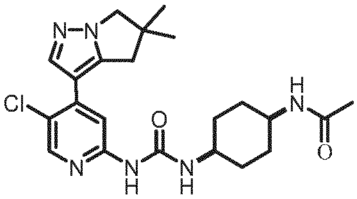 | 68 | 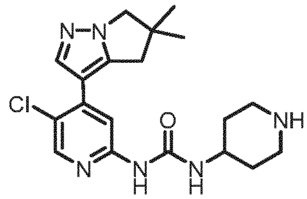 |
| 69 | 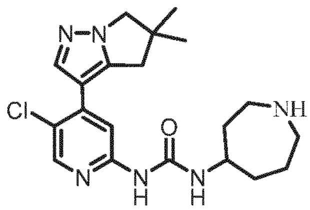 | 70 | 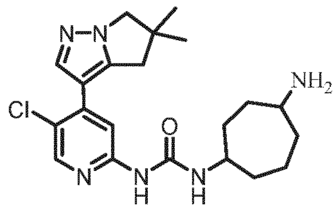 |
| 71 | 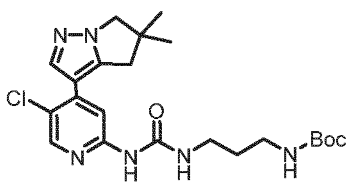 | 72 | 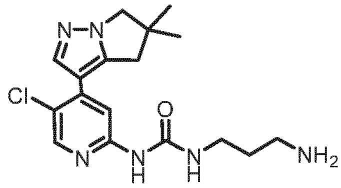 |
| 73 | 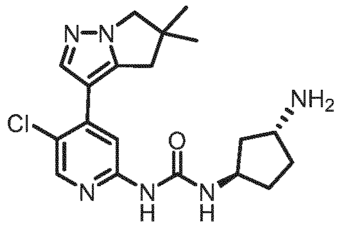 | 74 | 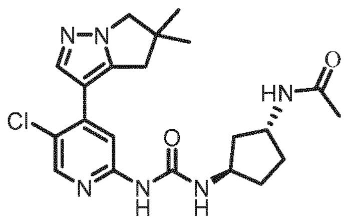 |
| 75 | 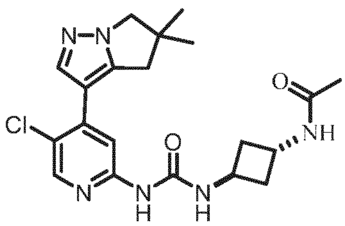 | 76 | 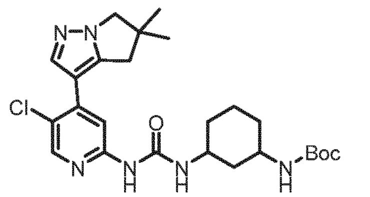 |
| 77 | 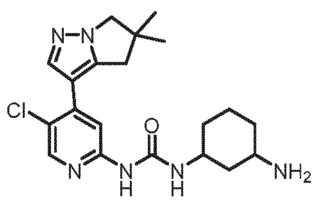 | 78 | 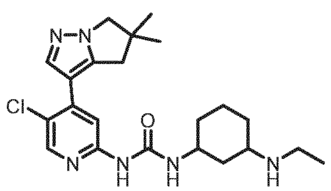 |

(continued)

| No. | Structure | No. | Structure |
|---|---|---|---|
| 79 | 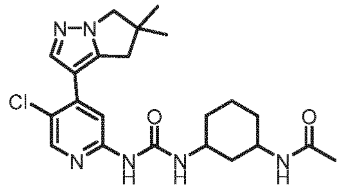 | 80 | 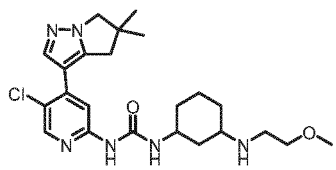 |
| 81 | 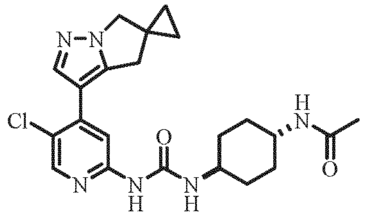 | 82 | 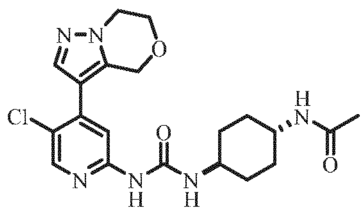 |
| 83 | 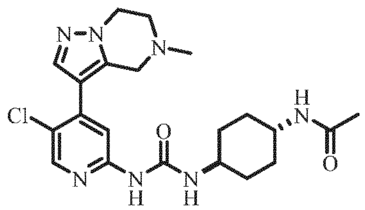 | 84 | 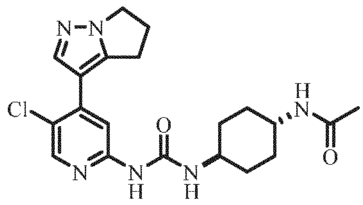 |
| 85 | 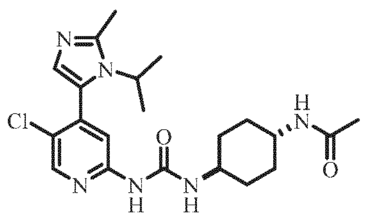 | 86 | 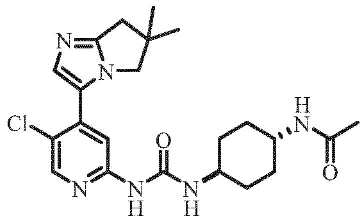 |
| 87 | 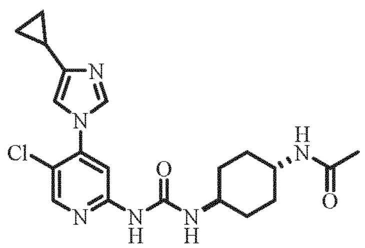 | 88 | 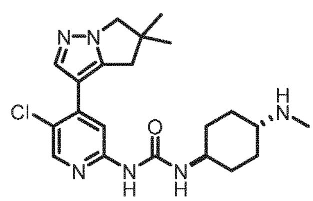 |
| 89 | 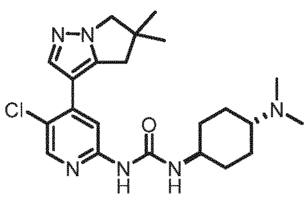 | 90 | 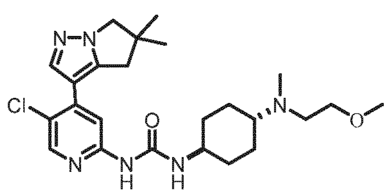 |
| 91 | 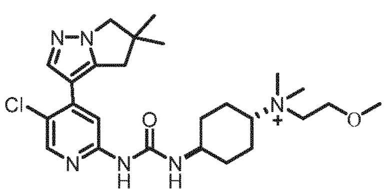 | 92 | 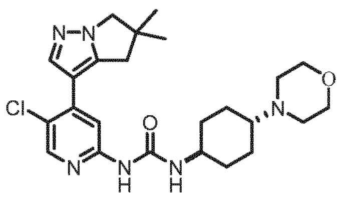 |

**5.** A method for preparing the compound of formula (I) or its deuterated compound or its pharmaceutically acceptable salt or prodrug as claimed in claim 1, which mainly includes the following steps:

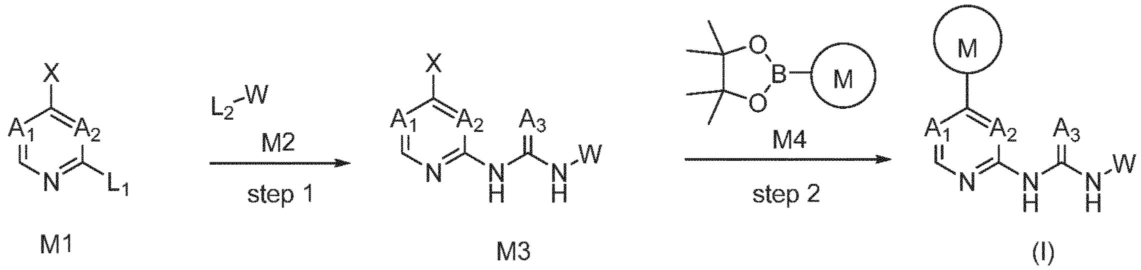


Wherein $A_1$, $A_2$, $A_3$, M and W are each independently defined as in claim 1; $L_1$ and $L_2$ are amino or carboxyl; X is bromine or chlorine;
Step 1: reacting compound M1 and compound M2 in a solvent to form urea/thiourea linked intermediate M3;
Step 2: conducting a coupling reaction between intermediate M3 and boric acid/boric acid pinacol ester M4 to obtain a compound of formula (I) in a solvent under the action of an alkali, a palladium catalyst and a ligand; the solvent is one or more selected from the group consisting of 1,4-dioxane, tetrahydrofuran, toluene, N,N-dimethylformamide, ethanol, ethylene glycol dimethyl ether and water; the palladium metal catalyst is any one of $Pd(PPh_3)_4$, $Pd_2(dba)_3$, $Pd(OAC)_2$ and $(dppf)PdCl_2$; the alkali is any one of $K_2CO_3$, $Cs_2CO_3$, KF, $K_2HPO_4$, $K_3PO_4$, $NaHCO_3$ and $Na_2CO_3$; the ligand is any one of X-Phos and $PCy_3$.

6. The method of claim 5, in step 1,

when $L_1$ and $L_2$ are amino groups, the synthesis method is: compound M1 or compound M2 first forms iso(thio)cyanate or chloroformate, and then reacts with the intermediate amine M2 or M1 to form (thio)urea-linked intermediate M3; or
when $L_1$ and $L_2$ are each amino and carboxyl, the urea-linked intermediate M3 is prepared using the following synthesis method: compound M1, compound M2, DPPA and alkali are reacted under microwave in anhydrous toluene to form intermediate M3.

7. A pharmaceutical composition, **characterized in that** it includes one or more of the compound of formula (I) according to any one of claims 1 to 4, their enantiomers, diastereomers, racemates or mixtures thereof, or pharmaceutically acceptable salts thereof, solvate, metabolite or prodrug, as well as pharmaceutically acceptable excipients.

8. Use of the compound of formula (I) according to any one of claims 1 to 4, their enantiomers, diastereomers, racemates or mixtures thereof, or pharmaceutically acceptable salts thereof, solvate, metabolite or prodrug, or the pharmaceutical composition according to claim 7 in the preparation of CDK inhibitors.

9. Use of the compound of formula (I) according to any one of claims 1 to 4, their enantiomers, diastereomers, racemates or mixtures thereof, or pharmaceutically acceptable salts thereof, solvate, metabolite or prodrug, or the pharmaceutical composition according to claim 7 in the preparation of the medicine for treating, preventing or releasing the diseases, disorders or conditions regulated by the activity of serine kinases, or diseases, disorders or conditions affected by them, or diseases, disorders or conditions involved with the activity of cyclin dependent kinase.

10. Use according to claim 9, wherein the disease, disorder or condition is selected from the group consisting of hyperproliferative diseases, virus-induced infectious diseases and cardiovascular diseases.
the hyperproliferative disease is preferably a tumor, the tumor is selected from acute myeloid leukemia, multiple myeloma, chronic lymphocytic leukemia, diffuse large B-cell lymphoma, Burkitt's lymphoma, follicular lymphoma, neuroblastoma, skin cancer, melanoma, lung cancer, gastric cancer, breast cancer, and intestinal cancer.

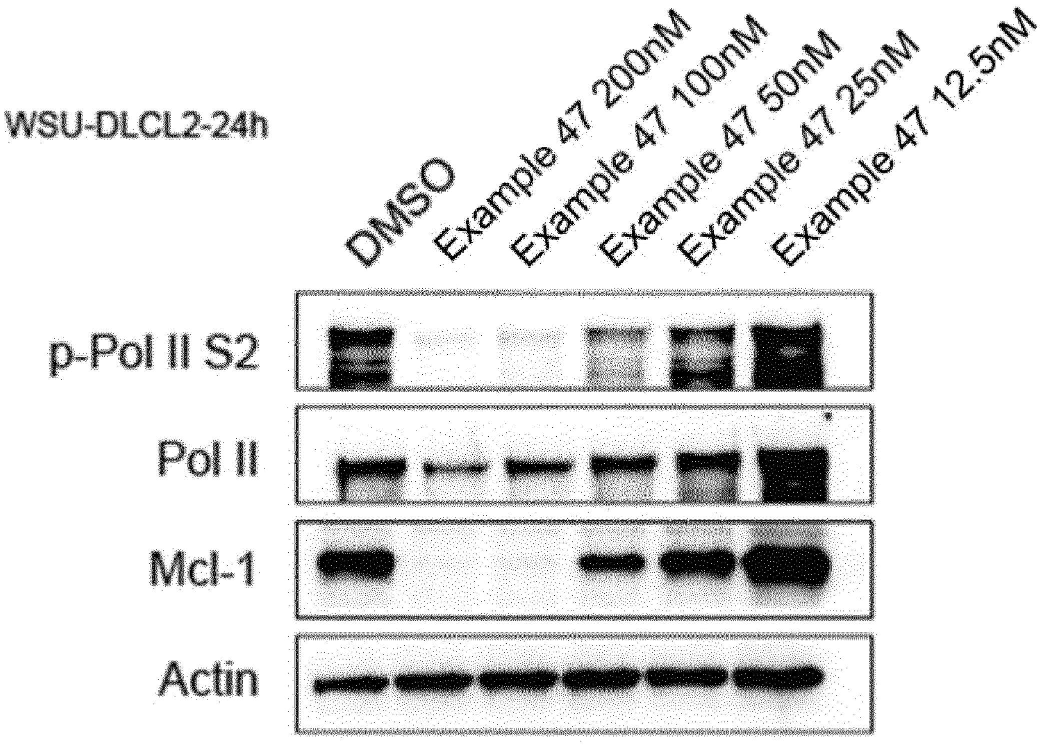
WSU-DLCL2-24h
DMSO
Example 47 200nM
Example 47 100nM
Example 47 50nM
Example 47 25nM
Example 47 12.5nM
p-Pol II S2
Pol II
Mcl-1
Actin

Fig.1

## INTERNATIONAL SEARCH REPORT

International application No.
**PCT/CN2022/107742**

**A. CLASSIFICATION OF SUBJECT MATTER**

C07D 213/75(2006.01)i; A61P 35/00(2006.01)i; A61K 31/506(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNTXT, WPABS, 万方, WANFANG, cnki, STN-REG, CAP, MARPAT: 中国科学院上海药物研究所, 胡有洪, 陈奕, 谢志铖, 丁健, 李欣, 方艳芬, 沈倩倩, cdk, 周期蛋白依赖性激酶, 癌症, 肿瘤, 脲, urea, cyclin dependent kinase, STN structure search

**C. DOCUMENTS CONSIDERED TO BE RELEVANT**

| Category* | Citation of document, with indication, where appropriate, of the relevant passages | Relevant to claim No. |
|---|---|---|
| X | JP 2002220338 A (BANYU PHARMA CO., LTD.) 09 August 2002 (2002-08-09) see abstract, and description, page 90, compound 267, pages 41-42, paragraph 230 | 1-10 |
| X | STNext Registry database. "CAS RN is 1279856-04-0" *American Chemical Society (ACS),* 14 April 2011 (2011-04-14), abstract | 1-3 |
| A | WO 2021155006 A1 (LES LABORATOIRES SERVIER SAS et al.) 05 August 2021 (2021-08-05) see entire document | 1-10 |
| A | CN 103923066 A (NANJING HUAWE MEDICINE TECHNOLOGY DEVELOPMENT CO., LTD.) 16 July 2014 (2014-07-16) see entire document | 1-10 |
| A | US 2007027147 A1 (HAYAMA TAKASHI et al.) 01 February 2007 (2007-02-01) see entire document | 1-10 |
| A | HONMA Teruki et al. "Structure-Based Generation of a New Class of Potent Cdk4 Inhibitors: New de Novo Design Strategy and Library Design" *Journal of Medicinal Chemistry,* Vol. 44, 13 December 2001 (2001-12-13), pp. 4615-4627 | 1-10 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 October 2022** | **21 October 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/107742**

| Patent document cited in search report | | | Publication date (day/month/year) | Patent family member(s) | | | Publication date (day/month/year) |
|---|---|---|---|---|---|---|---|
| JP | 2002220338 | A | 09 August 2002 | None | | | |
| WO | 2021155006 | A1 | 05 August 2021 | None | | | |
| CN | 103923066 | A | 16 July 2014 | None | | | |
| US | 2007027147 | A1 | 01 February 2007 | WO | 0107411 | A1 | 01 February 2001 |
| | | | | ES | 2251395 | T3 | 01 May 2006 |
| | | | | EP | 1199306 | A1 | 24 April 2002 |
| | | | | EP | 1557168 | A2 | 27 July 2005 |
| | | | | JP | 2011148811 | A | 04 August 2011 |
| | | | | AU | 6314900 | A | 13 February 2001 |
| | | | | DE | 60024631 | D1 | 12 January 2006 |
| | | | | US | 6958333 | B1 | 25 October 2005 |
| | | | | CA | 2380389 | A1 | 01 February 2001 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LIM S ; KALDIS P.** *Development,* 2013, vol. 140, 3079-3093
- **CHOU J et al.** *Cancer Discov.,* 2020, vol. 10 (3), 351-370
- **CHEN R et al.** *Blood,* 2009, vol. 113, 4637-4645
- **MACCALLUM DE et al.** *Cancer Res.,* 2005, vol. 65, 5399-5407
- **GREGORY GP et al.** *Leukemia,* 2015, vol. 29, 1437-1441
- **KRYSTOF V et al.** *Cell Cycle,* 2016, vol. 15, 519-527
- **SHUDONG WANG ; PETER M. FISCHER.** *Trends Pharmacol. Sci.,* 2008, vol. 29, 302-313
- *Chinese Nomenclature of Organic Compounds,* 2017, 274